(19) **Europäisches Patentamt**
**European Patent Office**
**Office européen des brevets**

(11) **EP 2 027 154 B1**

(12) **EUROPEAN PATENT SPECIFICATION**

(45) Date of publication and mention
of the grant of the patent:
**18.04.2012 Bulletin 2012/16**

(51) Int Cl.:
*C07K 16/00* (2006.01)  *A61P 35/00* (2006.01)
*A61P 37/00* (2006.01)

(21) Application number: **07755812.0**

(22) Date of filing: **20.04.2007**

(86) International application number:
**PCT/US2007/009693**

(87) International publication number:
**WO 2007/124077 (01.11.2007 Gazette 2007/44)**

(54) **IMMUNOGLOBULIN CONSTANT REGION DOMAINS WITH ENHANCED STABILITY**

DOMÄNEN DER KONSTANTEN REGION VON IMMUNOGLOBULINEN MIT VERBESSERTER STABILITÄT

DOMAINES DE REGION CONSTANTE D'IMMUNOGLOBULINE AYANT UNE STABILITE AMELIOREE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.04.2006 US 794202 P**
**21.11.2006 US 860774 P**

(43) Date of publication of application:
**25.02.2009 Bulletin 2009/09**

(73) Proprietor: **AMGEN INC.**
**Thousand Oaks, CA 91320-1799 (US)**

(72) Inventors:
• **MATSUMURA, Masazumi**
**Thousand Oaks, CA 91362 (US)**
• **JACOB, Jaby**
**Seattle, WA 98107 (US)**

(74) Representative: **Müller-Boré & Partner**
**Patentanwälte**
**Grafinger Straße 2**
**81671 München (DE)**

(56) References cited:
**WO-A-2004/074491     US-A1- 2002 098 193**

• **SCHULTZ DAVID A ET AL: "The crystal structure of the cis-proline to glycine variant (P114G) of ribonuclease A" PROTEIN SCIENCE, CAMBRIDGE UNIVERSITY PRESS, CAMBRIDGE, GB, vol. 14, no. 11, November 2005 (2005-11), pages 2862-2870, XP009097918 ISSN: 0961-8368**

• **DEMAREST S J ET AL: "Optimization of the Antibody CH3 Domain by Residue Frequency Analysis of IgG Sequences" JOURNAL OF MOLECULAR BIOLOGY, LONDON, GB, vol. 335, no. 1, 2 January 2004 (2004-01-02), pages 41-48, XP004476444 ISSN: 0022-2836**

• **THIES M J W ET AL: "Folding and association of the antibody domain CH3: prolyl isomerization preceeds dimerization" JOURNAL OF MOLECULAR BIOLOGY, LONDON, GB, vol. 293, no. 1, 15 October 1999 (1999-10-15), pages 67-79, XP004457326 ISSN: 0022-2836**

• **MAAULEY ARNOLD ET AL: "Contributions of a disulfide bond to the structure, stability, and dimerization of human IgG1 antibody C(H)3 domain" PROTEIN SCIENCE, CAMBRIDGE UNIVERSITY PRESS, CAMBRIDGE, GB, vol. 17, no. 1, January 2008 (2008-01), pages 95-106, XP009097931 ISSN: 0961-8368**

• **THIES M J W ET AL: "Folding and Oxidation of the Antibody Domain CH3" JOURNAL OF MOLECULAR BIOLOGY, LONDON, GB, vol. 319, no. 5, 21 June 2002 (2002-06-21), pages 1267-1277, XP004449701 ISSN: 0022-2836**

• **DALL'ACQUA WILLIAM ET AL: "Contribution of domain interface residues to the stability of antibody CH3 domain homodimers" BIOCHEMISTRY, AMERICAN CHEMICAL SOCIETY. EASTON, PA, US, vol. 37, no. 26, 30 June 1998 (1998-06-30), pages 9266-9273, XP002245748 ISSN: 0006-2960**

**Description**

This invention relates generally to medicines for the treatment of diseases and, more specifically to binding polypeptide pharmaceuticals having enhanced stability.

With the advent of recombinant DNA technology, protein-based therapeutics have become continually and increasingly commonplace in the repertoire of drugs available to medical practitioners for the treatment of a wide range of diseases from cancer to autoimmune diseases. Along with the scientific and technical advances that have occurred in the production of recombinant proteins, another reason for the success of protein therapeutics is their high specificity towards target molecules. The ability to employ biological molecules as pharmaceuticals in the treatment of diseases has significantly advanced medical care and quality of life over the past quarter of a century. As of the year 2005, there were more than one hundred and fifty approved protein-based pharmaceuticals on the market and this number is expected to increase in the coming years.

Proteins known to exhibit various pharmacological actions *in vivo* are now capable of being produced in large amounts for various pharmaceutical applications. Stability and homogeneity of a therapeutic protein preparation is a particularly beneficial criterion for safe, consistent and efficacious treatments. Loss of functionality of the therapeutic within a preparation will decrease its effective concentration for a given administration. Similarly, undesired conformational species of a therapeutic protein can lead to loss of efficacy and risk of adverse side effects.

Proteins are complex molecules with defined primary, secondary, tertiary and in some cases quaternary structures, all of which play a role in imparting specific biological function. Structural complexity of biological pharmaceuticals such as proteins makes them susceptible to various processes that result in structural and functional instability as well as loss of safety. For example, recombinant production of antibody therapeutics require each chain within the multimeric complex to be accurately synthesized, correctly folded and self assemble into dimeric or tetrameric complexes.

The third constant region domain of antibodies ($C_H3$) plays a structural role in the stability and the association of heavy chains of antibody molecules. Apart from the disulfide bonds that hold together the two heavy chains at the hinge region, strong non-covalent interactions between the two $C_H3$ domain monomers stabilize the constant region (Fc) of antibody molecules. The $C_H3$ domain possesses a buried intra-domain disulfide bond that stabilizes two β-sheets, a common feature observed in members of the immunoglobulin superfamily of polypeptides. However, several therapeutic antibodies (both IgG1 and IgG2) exhibit reduced disulfide bonds in as much as 15% of the $C_H3$ domains. It has been reported that the reduced $C_H3$ domain is much less stable than the oxidized molecule against heat, acidic pH and chemical denaturants such as guanidinium hydrochloride. In addition, in the reduced and unfolded state, where the cysteines are exposed to solvent, these residues can be prone to form inter-molecular disulfide bonds leading to irreversible covalent aggregates. Protein aggregation is of particular interest in biopharmaceutical production because it often results in diminished bioactivity that affects drug potency, and also can elicit undesirable immunological or antigenic reactions in patients.

In this context, Demarest, S. J. *et al.* (Demarest, S. J. et al., J. Mol. Biol. 335(1), January 02, 2004, pp. 41-48) describes the optimization of the folding/refolding characteristics of the antibody $C_H3$-domain by comparing $F_c$ sequences of 19 different mammalian species, identifying specific sites for optimization, and mutating said sites.

However, despite the advances made in the utilization of proteins in therapeutic treatments and their methods of production, there is still a need to develop antibody-based biopharmaceuticals with greater conformational homogeneity and enhanced stability. Manufacture of a therapeutic antibody preparation approaching homogeneity for a correctly folded, native conformation that possess correct disulfide bonds would increase the efficacy and safety of the therapeutic as well as lower the production and treatment costs. Numerous therapeutic antibodies and antibody-based binding proteins could benefit from such enhancements.

Thus, there exists a need for biopharmaceutical preparations that exhibit increased structural homogeneity and stability when produced under a variety of different manufacturing conditions. The present invention satisfies this need and provides related advantages as well.

The invention provides an isolated polypeptide including an immunoglobulin $C_H3$ region domain polypeptide, or functional fragment thereof, having at least one Pro to Gly substitution and exhibiting enhanced stability, including an enhanced folding rate, compared to an unsubstituted, wild-type (WT) $C_H3$ domain, wherein the at least one Pro to Gly substitution comprises $C_H3$ residue Pro38 or a $C_H3$ domain equivalent thereof. Multimeric complexes of an immunoglobulin $C_H3$ region domain polypeptide, or functional fragment thereof, having at least one Pro to Gly substitution with a second immunoglobulin $C_H3$ region domain polypeptide also are provided by the invention. The multimeric complexes can include antibodies, antibody functional fragments such as Fd, Fv, Fab, F(ab'), F(ab')$_2$, F(ab')$_2$, single chain Fv (scFv), chimeric antibodies, diabodies, triabodies, tetrabodies or minibody and other binding polypeptides having an immunoglobulin $C_H3$ region domain polypeptide such as a peptibody or an Fc fusion polypeptide. An isolated population of polypeptides including a plurality of immunoglobulin $C_H3$ region domain polypeptides, or functional fragments thereof, is further provided. Each $C_H3$ region domain polypeptide within the populations includes at least one Pro to Gly substitution and exhibits enhanced stability, including an enhanced folding rate, compared to an unsubstituted $C_H3$ domain, wherein

the at least one Pro to Gly substitution comprises $C_H3$ residue Pro38 or a $C_H3$ domain equivalent thereof. The invention further provides a method of producing a multimeric binding polypeptide and a binding polypeptide for the treatment of a pathological condition characterized by aberrant cell growth.

Figure 1 shows the amino acid sequence of a representative wild type (WT) $C_H3$ domain (SEQ ID NO:1) corresponding to an $IgG_1$ $C_H3$ domain.

Figure 2 shows an amino acid sequence alignment of the constant region domains for IgG subclasses $IgG_1$, $IgG_2$, $IgG_3$ and $IgG_4$ (SEQ ID NOS:2-5, respectively). A consensus amino acid sequence for the constant region domain of these IgG subclasses is shown below the alignment (SEQ ID NO:6). Pro38 of the $C_H3$ domain is located at residue 257 in each constant region domain for this alignment, which corresponds to residue 155 in the human Fc region sequence described below (SEQ ID NO:13).

Figure 3 shows an amino acid sequence alignment of the constant region domains for Ig classes IgG (represented by $IgG_1$), $IgA_1$, $IgA_2$, IgD, IgE and IgM (SEQ ID NOS:2 and 7-11, respectively). A consensus amino acid sequence for the constant region domain of these Ig classes is shown below the alignment (SEQ ID NO:12). Pro38 of the $C_H3$ domain is located at residue 381 in each constant region domain for this alignment, which corresponds to residue 155 in the human Fc region sequence described below (SEQ ID NO: 13).

Figure 4 shows an exemplary list of cancerous pathological conditions.

Figure 5 shows the spectroscopic properties of wild type (WT) and P38G $C_H3$ domain polypeptides for (a) Near-UV and (b) far-UV circular dichroism (CD) data of WT (solid line) and P38G (dashed line). Figure 5 (c) shows the fluorescence emission spectra of folded (solid line) and unfolded (dashed line) WT polypeptide.

Figure 6 shows guanidinium hydrochloride (GdmCl) induced denaturation results of reduced forms of WT ($\square$) and P38G ($\Delta$) $C_H3$ domain polypeptides.

Figure 7 shows exemplary unfolding kinetics for (a) WT and (b) P38G polypeptides.

Figure 8 shows exemplary results for dependency of unfolding rates on GdmCl concentration.

Figure 9 shows exemplary folding kinetics for (a) WT and (b) P38G polypeptides.

Figure 10 shows exemplary results for dependency of the folding rates and amplitudes (inset) on denaturant for WT and P38G polypeptides.

Figure 11 shows the results for double jump refolding kinetics of (a) WT and (b) P38G polypeptides.

Figure 12 shows revered-phase chromatograms of wild-type $C_H3$ polypeptide having various disulfide states.

Figure 13 shows an oxidative refolding analysis of (a) WT and (b) P38G $C_H3$ polypeptides analyzed by reversed-phase HPLC

This invention is directed to the discovery that substitution of one or more proline residues in an immunoglobulin domain can increase the rate of domain folding and extent of correctly folded domains, thus enhancing its stability. One particularly useful immunoglobulin domain proline residue that can be altered to increase folding rate and extent is the proline at position 38 (Pro38) of an immunoglobulin $C_H3$ region domain. This residue is located in close proximity to a β turn and favors a *cis* conformation. However, the folding of an immunoglobulin domain such as a $C_H3$ domain is coupled with the trans to cis isomerization of Pro38 that causes a slow folding. Therefore, substitution of Pro38 with an energetically favorable amino acid residue can increase the overall folding rate by reducing or eliminating this slow the *trans* to *cis* isomerization process during polypeptide folding, leading to more correctly folded domains in a shorter period of time. The Pro38 substituted immunoglobulin $C_H3$ region domains of the invention can be beneficially used in the production of more homogeneous preparations of therapeutic antibodies and other therapeutic binding polypeptides that utilize an immunoglobulin constant region domain fused to a ligand binding domain.

In one specific embodiment, the invention is directed to an immunoglobulin $C_H3$ region domain having Pro38 substituted with glycine (P38G). The P38G $C_H3$ domains of the invention are incorporated into immunoglobulins having a desired binding specificity to augment stability due to an increase in non-covalent interactions imparted by the greater number of correctly folded molecules. Such P38G $C_H3$ domain containing immunoglobulin preparations also exhibit greater conformational homogeneity, leading to more efficacious therapeutic preparations and enhanced safety.

As used herein, the term "isolated" when used in reference to a polypeptide is intended to mean that the referenced polypeptide is free from at least one component associated with the polypeptide as is found in nature. Therefore, an isolated polypeptide includes a substantially pure polypeptide as well as a heterogeneous mixture containing the isolated polypeptide. An example of a substantially pure polypeptide is a substantially homogeneous preparation that is suitable for administration to a human. A specific example of a heterogeneous mixture includes a cell extract containing the isolated polypeptide.

As used herein, the term "immunoglobulin" (Ig) is intended to mean an antibody, which is a polypeptide complex made up of heavy and light chains generally linked by disulfide bonds and non-covalent interactions and exhibits specific binding affinity for a molecular target or antigen. Immunoglobulins have a common core structure of two light and heavy chains held together by disulfide bonds. Both the light chains and the heavy chains contain a series of repeating, homologous units, each generally about 100-110 amino acid residues in length, which fold independently in a common globular motif, called an immunoglobulin domain. In each chain, one domain corresponds to a variable amino acid sequence (variable domain, variable region domain or V) depending on the antibody specificity of the molecule. The other domains have a constant sequence (constant domains, constant region domain or C) common among molecules of the same isotype. Heavy chains are designated by the letter of the Greek alphabet corresponding to the overall isotype of the antibody: IgA1 contains α1 heavy chains; IgA2, α2; IgD, δ; IgE, ε; IgG1, γ1, IgG2, γ2; IgG3, γ3; IgG4, γ4; and IgM, μ. Each heavy chain includes four domains; an amino terminal variable, or $V_H$ domain which displays the greatest sequence variation among heavy chains and three domains which form the constant region ($C_H1$, $C_H2$ and $C_H3$) in order from the amino to the carboxy terminus of the heavy chain. In γ, α and δ heavy chains, there is a non-globular region of amino acid sequence, known as the hinge, located between the first and second constant region domains ($C_H1$ and $C_H2$) permitting motion between these two domains. The $C_H3$ domain of heavy chains can homo-dimerize, facilitating the stability of a tetrameric antibody complex. Light chains contain a similar structure but are smaller compared to heavy chains. Light chains are designated as κ and λ and contain variable and constant regions. The constant region of light chains generally contains a single domain ($C_L$).

As used herein, the term "immunoglobulin domain" is intended to mean a polypeptide domain containing a region of homology within immunoglobulins recognized in the art as being about 80-150 amino acids and characterized as a β-sandwich. The β sandwich is organized into two layers of antiparallel β-sheets in which the flat hydrophobic faces of the β-sheets pack against each other. An immunoglobulin domain is used as a criterion for classifying polypeptides into the immunoglobulin superfamily of polypeptides. Immunoglobulin domains are contained in the heavy and light chain variable and constant region domains of immunoglobulins. Specific examples of immunoglobulin domains found in antibodies are $V_H$, $V_L$, $C_H1$, $C_H2$, $C_H3$, and $C_L$. Therefore, the term "immunoglobulin $C_H3$ region domain" or "immunoglobulin $C_H3$ domain" refers to the third constant region domain found in immunoglobulin heavy chains.

Immunoglobulin domains also are contained in diverse range of other polypeptides and are used for classifying polypeptides into superfamilies other than the immunoglobulin superfamily including, for example, the fibronectin type III, cadherin and other superfamilies. Ig domain structural similarity is maintained between members of different superfamilies irrespective of significant sequence identity. A description of the different Ig domain containing superfamilies can be found, for example, in Clarke et al., Structure Fold. Des. 7:1145-53 (1999) and within structural databases such as at the URL pdb.weizmann.ac.il/scop/data/scop.b.c.b.html. Examples of Ig domain containing superfamily members include, for example, the various individual members within the immunoglobulin domain containing superfamilies described above. Such individual members include, for example, T cell receptor, CD8, CD4, CD2, class I MHC, class II MHC, CD1, cytokine receptor, GCSF receptor, GMCSF receptor, growth hormone receptor, erythropoietin receptor, interferon receptor, interferon gamma receptor, prolactin receptor, NCAM, VCAM, ICAM, N-caderin, E-caderin, fibronectin, tenascin, and I-set containing domain polypeptides, or a functional fragment thereof. Exemplary descriptions of these and other Ig domain containing superfamily members can be found in, for example, Isacke and Horton, The Adhesion Molecule FactsBook, Second Ed., Academic Press, San Diego (2000); Fitzgerald et al., The Cytokine FactsBook, Second Ed., Academic Press, San Diego (2001), and Marsh et al., The HLA FactsBook, Second Ed., Academic Press, San Diego (1999).

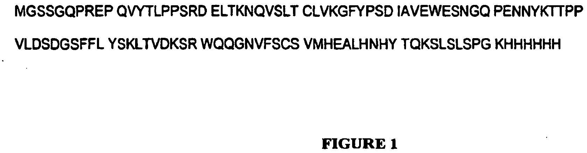

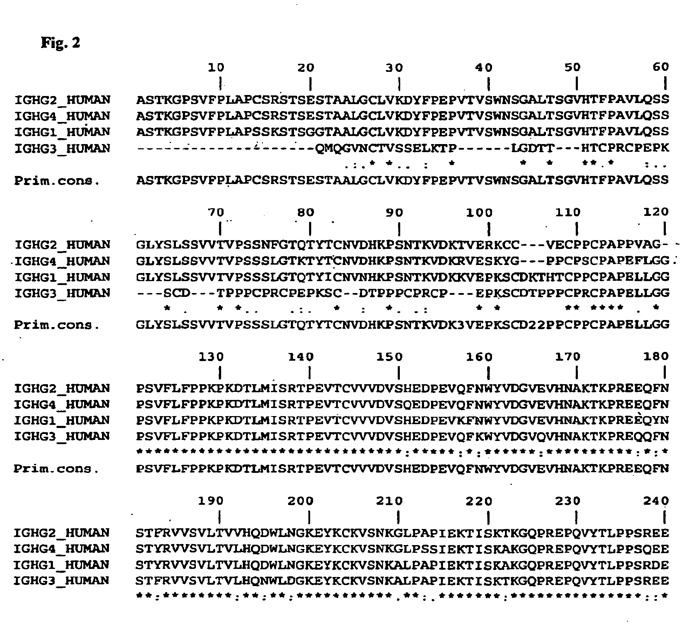

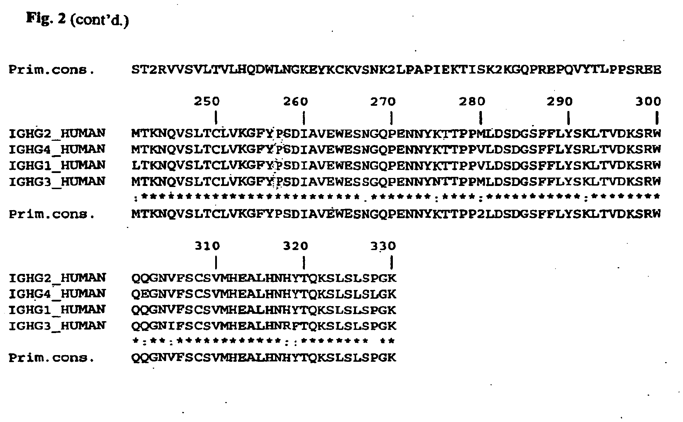

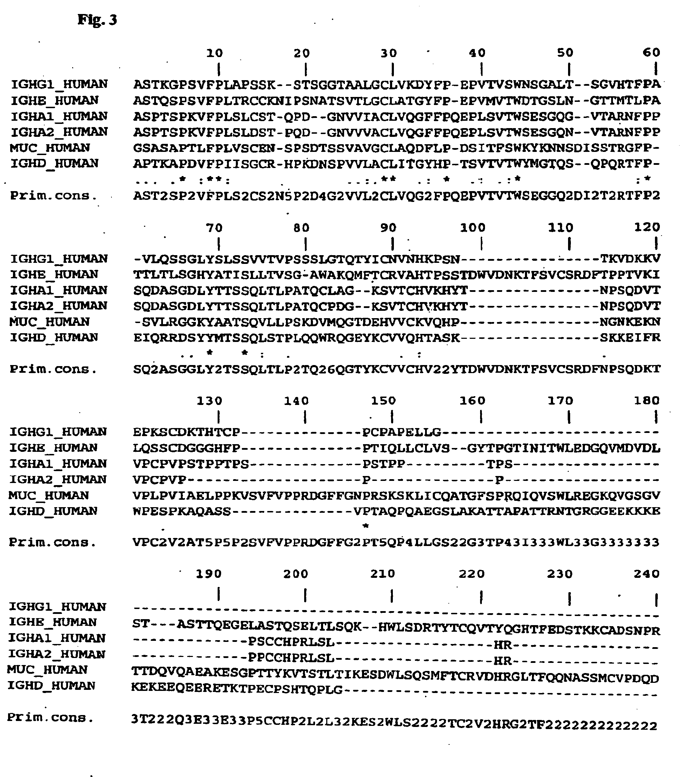

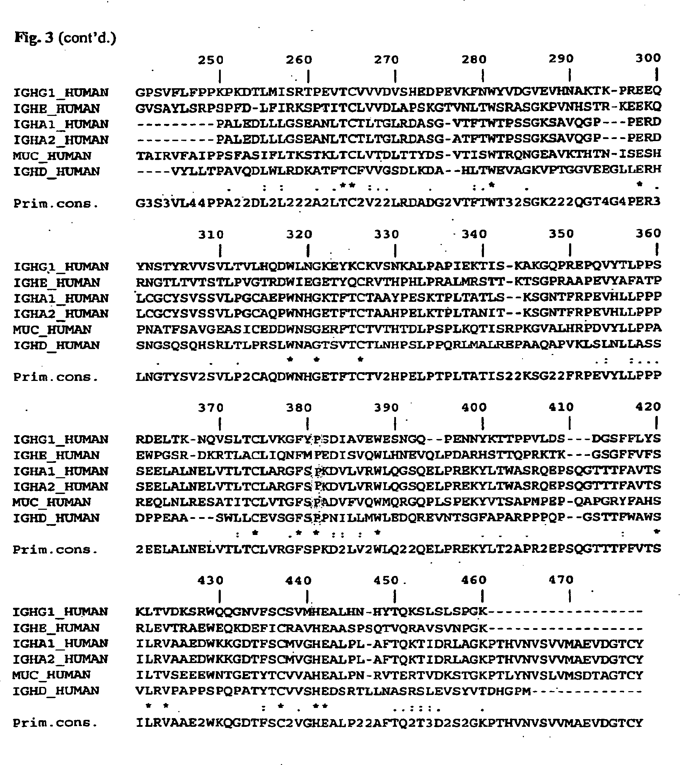

As used herein, the term "$C_H3$ residue Pro38" is intended to mean the proline (Pro or P) residue located proximal to a beta turn at amino acid position 38 in an immunoglobulin $C_H3$ region domain. A representative $C_H3$ domain sequence is shown in Figure 1, which corresponds to a recombinant IgG$_1$ $C_H3$ domain expressed in *E. coli*. Pro38 corresponds to the proline at amino acid position 38 in this exemplary $C_H3$ domain sequence. Pro38 is a relatively conserved amino acid residue among immunoglobulin domains, including immunoglobulin constant region domains, and particularly among immunoglobulin $C_H3$ domains. An amino acid sequence alignment of the constant region domains for the IgG subclasses IgG$_1$, IgG$_2$, IgG$_3$ and IgG$_4$ is shown in Figure 2. Although the actual amino acid position varies between different IgG subclasses because of alignment gaps, $C_H3$ domain Pro38 is located at residue 257 in all of these constant region domains. Pro38 located at residue 257 in these alignments corresponds to residue 155 in the human Fc region sequence described below as SEQ ID NO:13. An amino acid sequence alignment of the constant region domains for the Ig classes IgG (represented by $IgG_1$), $IgA_1$, $IgA_2$, IgD, IgE and IgM is shown in Figure 3. As with the IgG subclasses, the actual amino acid position of $C_H3$ Pro38 varies between the different Ig classes because of alignment gaps, but the Pro38 residue is shown to be conserved at $C_H$ residue 381. Pro38 located at residue 381 in these alignments corresponds to residue 155 in the human Fc region sequence described below as SEQ ID NO:13. Therefore, the terms "$C_H3$ domain residue Pro38," "$C_H3$ residue Pro38," "$C_H3$ Pro38," "Pro38" or grammatical equivalents refer to the conserved Pro residue located at position 38 in the representative WT $C_H3$ domain sequence shown in Figure 1, the Pro257 in IgG subclass constant regions, the Pro381 in Ig class constant regions and the Pro 155 shown in the human Fc region sequence of SEQ ID NO:13.

The term "$C_H3$ domain equivalent" when referring to a Pro38 residue is intended to refer to the set of immunoglobulin domain Pro38 residues as defined above that align with Pro38 within a $C_H3$ domain but have the conserved Pro residue located at an actual position other than domain position 38. For example, $IgA_1$ and $IgA_2$ contain a Pro38 $C_H3$ domain equivalent at position 39 since these $C_H3$ domains contain an extra amino acid compared to, for example, $IgG_1$ (corresponding to residue N367 in Figure 3). Therefore, a Pro38 $C_H3$ domain equivalent refers to the Pro residue located proximal to a beta turn in an immunoglobulin domain and conserved by sequence alignment to Pro38 in an immunoglobulin $C_H3$ domain.

As used herein, the term "functional fragment" when used in reference to an immunoglobulin $C_H3$ region domain polypeptide of the invention is intended to mean a portion of an immunoglobulin $C_H3$ domain polypeptide which retains at least about the same $C_H3$ homo-dimerization activity compared to an intact or full-length $C_H3$ domain polypeptide. Such functional fragments can include, for example, truncated, deleted or substituted amino acid residues of an immunoglobulin $C_H3$ domain or an immunoglobulin $C_H3$ domain containing polypeptide so long as it retains about the same ability to dimerize with a second immunoglobulin $C_H3$ domain polypeptide or a second immunoglobulin $C_H3$ domain containing polypeptide. Homo-dimerization activity can be retained, for example, where the three dimensional structure of the $C_H3$ domain is substantially retained.

With respect to antibodies and various forms thereof containing an immunoglobulin $C_H3$ region domain polypeptide of the invention, as described further below, any antibody including monoclonal antibodies and various antibody-like binding proteins known in the art, can be produced using methods well known in the art to contain a $C_H3$ region domain of the invention. For example, recombinant engineering methods are well known in the art which can be used to fuse an immunoglobulin variable region domain or domains to one or more constant region domains to produce hybrid binding polypeptides characteristic of antibodies and/or characteristic of immunoglobulin superfamily of proteins.

A monoclonal antibody refers to an antibody that is the product of a single cell clone or hybridoma. Monoclonal antibody also refers to an antibody produced by recombinant methods from heavy and light chain encoding immunoglobulin genes to produce a single molecular immunoglobulin species. Amino acid sequences for antibodies within a monoclonal antibody preparation are substantially homogeneous and the binding activity of antibodies within such a preparation exhibit substantially the same antigen binding activity when compared in the same or similar binding assay. As described further below, antibody and monoclonal antibody characteristics are well known in the art.

Monoclonal antibodies can be prepared using a wide variety of methods known in the art including the use of hybridoma, recombinant, myeloma cell-line expressed, phage display and combinatorial antibody library methodologies, or a combination thereof. For example, monoclonal antibodies can be produced using hybridoma techniques including those known in the art and taught, for example, in Harlow and Lane., Antibodies: A Laboratory Manual, Cold Spring Harbor Laboratory Press (1989); Hammerling, et al., in: Monoclonal Antibodies and T-Cell Hybridomas 563-681, Elsevier, N.Y. (1981); Harlow et al., Using Antibodies: A Laboratory Manual, Cold Spring Harbor Laboratory Press (1999), and Antibody Engineering: A Practical Guide, C.A.K. Borrebaeck, Ed., Second Ed., Oxford University Press, New York, (1995). Examples of known methods for producing monoclonal antibodies by recombinant, phage display and combinatorial antibody library methods, including libraries derived from immunized and naive animals can be found described in Antibody Engineering: A Practical Guide, C.A.K. Borrebaeck, Ed., supra. A monoclonal antibody for use as a biopharmaceutical is not limited to antibodies produced through hybridoma technology. Rather, as described previously, a monoclonal antibody refers to an antibody that is derived from a single clone, including any eukaryotic, prokaryotic, or phage clone, and not the method by which it is produced.

An antibody functional fragment refers to a portion of an antibody which retains some or all of its target-specific binding activity. These antibody functional fragments retaining target-specific binding activity can be produced to contain an immunoglobulin $C_H3$ region domain polypeptide of the invention. Such antibody functional fragments can include, for example, antibody functional fragments such as Fd, Fv, Fab, F(ab'), $F(ab)_2$, $F(ab')_2$, single chain Fv (scFv), chimeric antibodies, diabodies, triabodies, tetrabodies and minibodies. Other functional fragments can include, for example, heavy (H) or light (L) chain polypeptides, variable heavy ($V_H$) and variable light ($V_L$) chain region polypeptides, complementarity determining region (CDR) polypeptides, single domain antibodies, and polypeptides that contain at least a portion of an immunoglobulin that is sufficient to retain target-specific binding activity. Such antibody binding fragments can be found described in, for example, Harlow and Lane, supra; Molec. Biology and Biotechnology: A Comprehensive Desk Reference (Myers, R.A. (ed.), New York: VCH Publisher, Inc.); Huston et al., Cell Biophysics, 22:189-224 (1993); Plückthun and Skerra, Meth. Enzymol., 178:497-515 (1989) and in Day, E.D., Advanced Immunochemistry, Second Ed., Wiley-Liss, Inc., New York, NY (1990).

With respect to antibodies and functional fragments thereof that exhibit beneficial binding characteristics to a target molecule, various forms, alterations and modifications are well known in the art. Target-specific monoclonal antibodies containing an immunoglobulin $C_H3$ region domain of the invention can include any of such various monoclonal antibody forms, alterations and modifications. Examples of such various forms and terms as they are known in the art are set forth below.

A Fab fragment refers to a monovalent fragment consisting of the $V_L$, $V_H$, $C_L$ and $C_H1$ domains; a $F(ab')_2$ fragment is a bivalent fragment comprising two Fab fragments linked by a disulfide bridge at the hinge region but lacking the Fc; a Fd fragment consists of the $V_H$ and $C_H1$ domains; an Fv fragment consists of the $V_L$ and $V_H$ domains of a single arm of an antibody; and a dAb fragment (Ward et al., Nature 341:544-546, (1989)) consists of a $V_H$ domain.

An antibody or an antibody functional fragment can have one or more binding sites. If there is more than one binding site, the binding sites may be identical to one another or may be different. For example, a naturally occurring immunoglobulin has two identical binding sites, a single-chain antibody or Fab fragment has one binding site, while a "bispecific" or "bifunctional" antibody has two different binding sites. Therefore, the binding activity of antibodies or antibody functional fragments can be, for example, monospecific, bispecific or multi-specific.

A single-chain antibody (scFv) refers to an antibody in which a $V_L$ and a $V_H$ region are joined via a linker (e.g., a synthetic sequence of amino acid residues) to form a continuous polypeptide chain wherein the linker is long enough to allow the protein chain to fold back on itself and form a monovalent antigen binding site (see, e.g., Bird et al., Science 242:423-26 (1988) and Huston et al., Proc. Natl. Acad Sci. USA 85:5879-83 (1988)). Diabodies refer to bivalent antibodies comprising two polypeptide chains, wherein each polypeptide chain comprises $V_H$ and $V_L$ domains joined by a linker that is too short to allow for pairing between two domains on the same chain, thus allowing each domain to pair with a complementary domain on another polypeptide chain (see, e.g., Holliger et al., Proc. Natl. Acad Sci. USA 90:6444-48 (1993), and Poljak et al., Structure 2:1121-23 (1994)). If the two polypeptide chains of a diabody are identical, then a diabody resulting from their pairing will have two identical antigen binding sites. Polypeptide chains having different sequences can be used to make a diabody with two different antigen binding sites. Similarly, tribodies and tetrabodies are antibodies comprising three and four polypeptide chains, respectively, and forming three and four antigen binding sites, respectively, which can be the same or different.

A CDR refers to a region containing one of three hypervariable loops (H1, H2 or H3) within the non-framework region of the immunoglobulin (Ig or antibody) $V_H$ β-sheet framework, or a region containing one of three hypervariable loops (L1, L2 or L3) within the non-framework region of the antibody $V_L$ β-sheet framework. Accordingly, CDRs are variable region sequences interspersed within the framework region sequences. CDR regions are well known to those skilled in the art and have been defined by, for example, Kabat as the regions of most hypervariability within the antibody variable (V) domains (Kabat et al., J. Biol. Chem. 252:6609-6616 (1977); Kabat, Adv. Prot. Chem. 32:1-75 (1978)). CDR region sequences also have been defined structurally by Chothia as those residues that are not part of the conserved β-sheet framework, and thus are able to adapt different conformations (Chothia and Lesk, J. Mol. Biol. 196:901-917 (1987)). Both terminologies are well recognized in the art. The positions of CDRs within a canonical antibody variable domain have been determined by comparison of numerous structures (Al-Lazikani et al., J. Mol. Biol. 273:927-948 (1997); Morea et al., Methods 20:267-279 (2000)). Because the number of residues within a loop varies in different antibodies, additional loop residues relative to the canonical positions are conventionally numbered with a, b, c and so forth next to the residue number in the canonical variable domain numbering scheme (Al-Lazikani et al., supra (1997)). Such nomenclature is similarly well known to those skilled in the art.

For example, CDRs defined according to either the Kabat (hypervariable) or Chothia (structural) designations, are set forth in the table below.

**Table 1: CDR Definitions**

|  | Kabat[1] | Chothia[2] | Loop Location |
|---|---|---|---|
| $V_H$ CDR1 | 31-35 | 26-32 | linking B and C strands |
| $V_H$ CDR2 | 50-65 | 53-55 | linking C' and C" strands |
| $V_H$ CDR3 | 95-102 | 96-101 | linking F and G strands |
| $V_L$ CDR1 | 24-34 | 26-32 | linking B and C strands |
| $V_L$ CDR2 | 50-56 | 50-52 | linking C' and C" strands |

| | Kabat[1] | Chothia[2] | Loop Location |
|---|---|---|---|
| V$_L$ CDR3 | 89-97 | 91-96 | linking F and G strands |

(continued)

[1] Residue numbering follows the nomenclature of Kabat et al., *supra*

[2] Residue numbering follows the nomenclature of Chothia et al., *supra*

A chimeric antibody refers to an antibody that contains one or more regions from one antibody and one or more regions from one or more other antibodies. In one specific example, one or more of the CDRs are derived from a non-human donor antibody having specific activity to a target molecule and the variable region framework is derived from a human recipient antibody. In another specific example, all of the CDRs can be derived from a non-human donor antibody having specific activity to a target molecule and the variable region framework is derived from a human recipient antibody. In yet another specific example, the CDRs from more than one non-human target-specific antibodies are mixed and matched in a chimeric antibody. For instance, a chimeric antibody can include a CDR1 from the light chain of a first non-human target-specific antibody, a CDR2 and a CDR3 from the light chain of a second non-human target-specific antibody and the CDRs from the heavy chain from a third target-specific antibody. Further, the framework regions can be derived from one of the same or from one or more different human antibodies or from a humanized antibody. Chimeric antibodies can be produced where both the donor and recipient antibodies are human.

A humanized antibody or grafted antibody has a sequence that differs from a non-human species antibody sequence by one or more amino acid substitutions, deletions, and/or additions, such that the humanized antibody is less likely to induce an immune response, and/or induces a less severe immune response, as compared to the non-human species antibody, when it is administered to a human subject. In one specific example, certain amino acids in the framework and constant domains of the heavy and/or light chains of the non-human species antibody are changed to produce the humanized antibody. In another specific example, the constant domain(s) from a human antibody are fused to the variable domain(s) of a non-human species. Examples of how to make humanized antibodies may be found in U.S. Pat. Nos. 6,054,297, 5,886,152 and 5,877,293. Humanized antibodies also include antibodies produced using antibody resurfacing methods and the like.

A human antibody refers to antibodies that have one or more variable and constant regions derived from human immunoglobulin sequences. For example, a fully human antibody includes an antibody where all of the variable and constant domains are derived from human immunoglobulin sequences. Human antibodies can be prepared using a variety of methods known in the art.

One or more CDRs also can be incorporated into a molecule either covalently or noncovalently to make it an immunoadhesin. An immunoadhesin can incorporate the CDR(s) as part of a larger polypeptide chain, can covalently link the CDR(s) to another polypeptide chain, or can incorporate the CDR(s) noncovalently. The CDRs permit the immunoadhesin to specifically bind to a particular antigen of interest.

A neutralizing antibody or an inhibitory antibody refers to a target-specific monoclonal antibody that inhibits the binding of the target molecule to its binding partner when an excess of the target-specific monoclonal antibody reduces the amount of binding partner bound to the target. Binding inhibition can occur by at least 10%, particularly by at least about 20%. In various specific examples, the monoclonal antibody can reduce the amount of binding partner bound to the target by, for example, at least 30%, 40%, 50%, 60%, 70%, 75%, 80%, 85%, 90%, 95%, 97%, 99%, and 99.9%. The binding reduction can be measured by any means known to one of ordinary skill in the art, for example, as measured in an *in vitro* competitive binding assay.

An antagonistic antibody refers to an antibody that inhibits the activity of a target molecule when added to a cell, tissue or organism expressing the target molecule. Diminution in activity can be by at least about 5%, particularly by at least about 10%, more particularly, by at least about 15% or more, compared to the level of target molecule activity in the presence of binding partner alone. In various specific examples, the target-specific monoclonal antibodies for use as a biopharmaceutical of the invention can inhibit the target molecule activity by at least about 20%, 30%, 40%, 50%, 60%, 70%, 80%, 90% or 100%.

An agonist antibody refers to an antibody that activates a target molecule by at least about 5%, particularly by at least about 10%, more particularly, by at least about 15% when added to a cell, tissue or organism expressing the target molecule, where 100% activation is the level of activation achieved under physiological conditions by the same molar amount of binding partner. In various specific examples, the target-specific monoclonal antibodies for use as a biopharmaceutical of the invention can activate target molecule activity by at least about 20%, 30%, 40%, 50%, 60%, 70%, 80%, 90%, 100%, 125%, 150%, 175%, 200%, 250%, 300%, 350%, 400%, 450%, 500%, 750% or 1000%.

With respect to binding polypeptides containing a C$_H$3 region or other immunoglobulin domain, a C$_H$3 region domain polypeptide of the invention having a P38G substitution also can be incorporated into any of these such binding polypeptides to enhance their stability. Exemplary forms of such binding polypeptides include peptibodies and Fc fusion polypeptides.

A peptibody refers to a binding polypeptide consisting of an immunoglobulin constant region domain or the Fc portion of a constant region (Fc) linked to at least one binding peptide through either the carboxyl- or amino- termini of the Fc domain. Peptibodies can be, for example, monovalent (one attached binding peptide), divalent (two attached binding peptides) or multivalent (more than two attached peptides). The binding peptides linked to divalent peptibodies can exhibit the same binding specificity (monospecific) or exhibit different binding specificity (dispecific). Peptibodies are well known in the art and can be found described in, for example, U.S. Patent No. 6,660,843 and U.S. Patent Application US2003/0176352.

A Fc fusion polypeptide, or Fc fusion binding polypeptide, refers to a binding polypeptide consisting of an immunoglobulin constant region domain or the Fc portion of a constant region (Fc) linked to at least one binding polypeptide. The binding polypeptide generally includes, for example, an intact protein or intact binding domain thereof. Fc fusion polypeptides are well known in the art. Specific examples of Fc fusion polypeptides include, for example, an Fc region fused to a leptin or a leptin derivative such as those described in patent applications WO 96/40912, WO 96/05309, WO 97/06816, WO 97/18833 and US20070020284.

As used herein, the term "plurality" when used in reference to a population of immunoglobulin $C_H3$ region domain polypeptides is intended to mean two or more $C_H3$ region domain polypeptides. Therefore, the term is intended to include populations of immunoglobulin $C_H3$ region domain polypeptides from two polypeptides to large libraries of polypeptides including, for example, $10^6$, $10^7$, $10^8$, $10^9$, $10^{10}$ or more. Other pluralities include populations having 2, 3, 4, 5, 6, 7, 8, 9, 10, 11, 12, 13, 14, 15, 16, 17, 18, 19 or 20 or more immunoglobulin $C_H3$ region domain polypeptides. Yet other pluralities include populations having 25, 30, 35, 40, 45, 50, 55, 60, 65, 70, 75, 80, 85, 90, 95 or 100 or more immunoglobulin $C_H3$ region domain polypeptides. Yet other pluralities include populations having 125, 150, 175, 200, 250, 300, 350, 400, 450, 500, 550, 600, 650, 700, 750, 800, 850, 900, 950, $10^3$, $10^4$ or $10^5$ or more immunoglobulin $C_H3$ region domain polypeptides. All population sizes above and below these exemplary numbers also is included within the meaning of the term "plurality" as it is used herein.

As used herein, the term "selective" when used in reference to the target binding activity of an immunoglobulin $C_H3$ region domain containing polypeptide of the invention is intended to mean that the target molecule binding domain exhibits preferential binding affinity or avidity toward the target antigen. Target molecule binding domain or target binding domain refers to an antigen binding domain of an immunoglobulin or a ligand binding domain of a binding polypeptide. An immunoglobulin $C_H3$ region domain containing polypeptide of the invention that exhibits selective binding affinity also does not substantially cross-react with non-target molecules. Selective binding includes specific binding activity such as when an immunoglobulin $C_H3$ region domain containing polypeptide of the invention does not measurably cross-react with non-target molecules.

As used herein, the term "target molecule" is intended to mean a molecule which can be bound by the antigen or ligand binding domain of an immunoglobulin $C_H3$ region domain containing polypeptide of the invention and form a covalent or non-covalent complex. In the specific case of antibodies, a noncovalent complex is formed in the antigen combining site of a variable region of the antibody. Target molecules include, for example, cellular antigens, which refer to antigens that are endogenous to a cell of a mammalian organism. Cellular antigens originate or derive from the mammalian organism or are encoded by the genome of the mammalian organism. Cellular antigens consist of all types of macromolecules and include, for example, proteins, glycoproteins, carbohydrate and lipid. Target molecues also include, for example, heterologous antigens, which refer to antigens that are originally foreign to a mammalian organism. Heterologous antigens include, for example, macromolecules originating, derived from or encoded by the genome of pathogens such as viruses, bacteria and parasites. Cellular antigens and heterologous antigens are similarly intended to included inappropriate or aberrantly expressed antigens derived from or encoded by each of their respective mammalian cell or pathogen origins.

As used herein, the term "pathological condition" when used in reference to aberrant cell growth, is intended to mean a disease or abnormal condition, including an injury, of a mammalian cell or tissue. Therefore, the term "aberrant cell growth" is intended to refer to those diseases or abnormal conditions that result in unwanted or abnormal cell growth, viability or proliferation. Pathological conditions characterized by unwanted or abnormal cell growth include, for example, cancer and other neoplastic conditions, infectious diseases and autoimmune diseases. For example, cancer cells proliferate in an unregulated manner and consequently result in tissue destruction. Similarly, the proliferation of cells mediating autoimmune diseases are aberrantly regulated which results in, for example, the continued, proliferation and/or activation of immune mechanisms with destruction of the host's cells and tissue. The growth of cells infected by a pathogen are unwanted and abnormal due to the intrusion of the foreign organism, for example. Specific examples of cancer include prostate, breast, lung, ovary, uterus, brain and skin cancer. Specific examples of infectious diseases include DNA or RNA viral diseases, bacterial diseases, paracytic diseases whereas autoimmune diseases include, for example, diabetes, rheumatoid arthritis and multiple sclerosis.

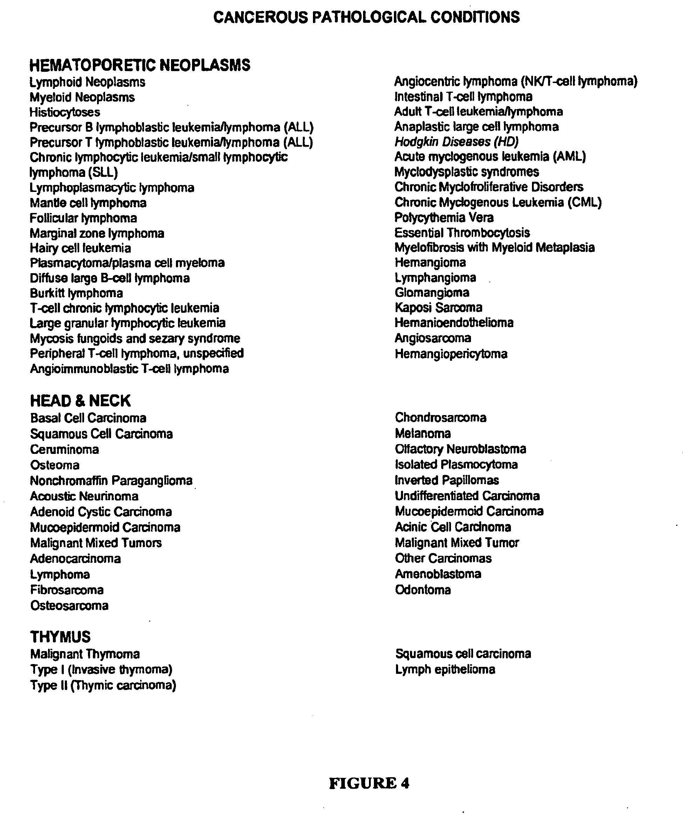

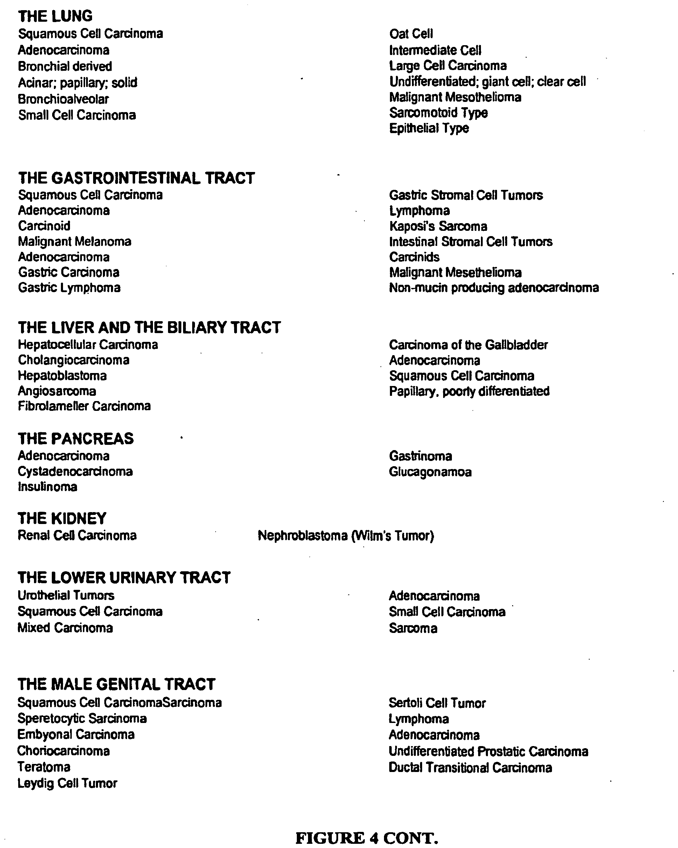

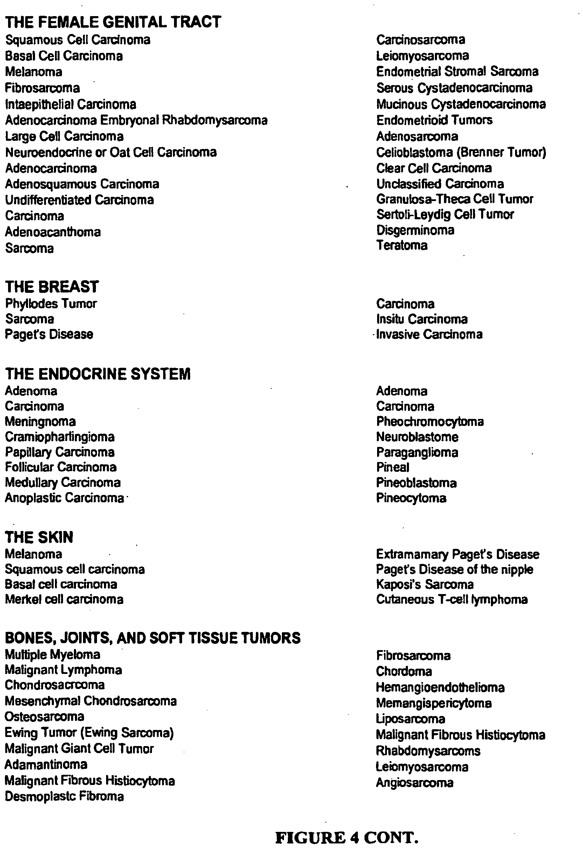

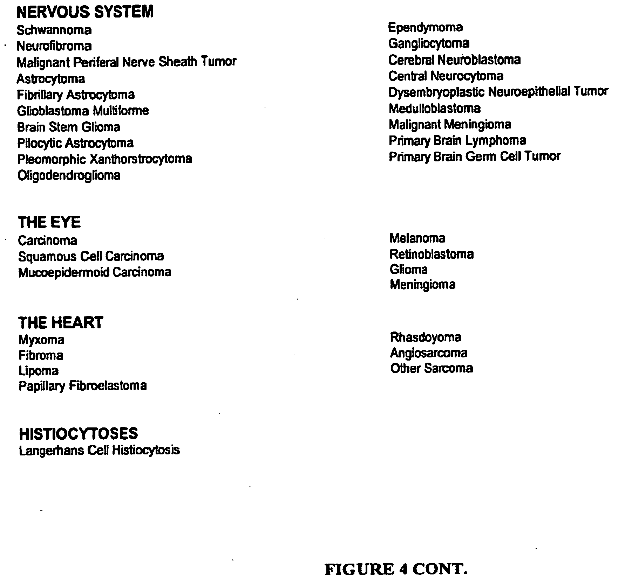

By specific mention of the above categories of pathological conditions, those skilled in the art will understand that such terms include all classes and types of these pathological conditions. For example, the term cancer is intended to include all known cancers, whether characterized as malignant, benign, soft tissue or sold tumor. By exemplification, a list of known cancers is provided in Figure 4. Similarly, and by analogy to the classes and types of cancers shown in Figure 4, the terms infectious diseases and autoimmune diseases are intended to include all classes and types of these pathological conditions. Those skilled in the art will known the various classes and types of infectious and autoimmune diseases.

As used herein, the term "treating" is intended to mean reduction in severity or prevention of a pathological condition characterized by aberrant cell growth. Reduction in severity includes, for example, an arrest or a decrease in clinical symptoms, physiological indicators, biochemical markers or metabolic indicators. Prevention of the disease includes, for example, precluding the occurrence of the disease or restoring a diseased individual to their pre-diseased state of health.

As used herein, the term "effective amount" is intended to mean an amount of an immunoglobulin $C_H3$ region domain containing polypeptide of the invention required to effect a decrease in the extent, amount or rate of spread of a pathological condition when administered to an individual. Therefore, an effective amount when used in reference to a binding polypeptide containing an immunoglobulin $C_H3$ region domain polypeptide having a Pro to Gly substitution at Pro38 (P38G) is intended to mean an amount of the binding polypeptide sufficient to ameliorate at least one symptom associated with a targeted disease or pathological condition characterized by aberrant cell growth.

The dosage of a binding polypeptide of the invention required to be therapeutically effective will depend, for example, on the pathological condition to be treated, the affinity and avidity of the targeting molecules, the level of abundance and density of the cognate antigens as well as the weight and condition of the individual, and previous or concurrent therapies. The appropriate amount considered to be an effective dose for a particular application of the method can be determined by those skilled in the art, using the guidance provided herein. For example, the amount can be extrapolated from *in vitro* or *in vivo* assays as described below. One skilled in the art will recognize that the condition of the patient needs to be monitored throughout the course of therapy and that the amount of the composition that is administered can be adjusted according to the response of the therapy.

The invention provides an isolated polypeptide including an immunoglobulin $C_H3$ region domain polypeptide, or functional fragment thereof, having at least one Pro to Gly substitution and exhibiting enhanced stability compared to an unsubstituted $C_H3$ domain, wherein the at least one Pro to Gly substitution comprises $C_H3$ residue Pro38 or a $C_H3$ domain equivalent thereof, wherein said polypeptide further comprises an enhanced folding rate compared to an un-substituted $C_H3$ domain.

The $C_H3$ domain of an immunoglobulin plays a role in the stability of the molecule because it associates with other $C_H3$ domains to form a $C_H3$ domain homodimer. For example, the homodimer is held together by strong non-covalent interactions between the $C_H3$ domain. The intra-molecular disulfide bond within the $C_H3$ domain stabilizes the domain itself. In manufacturing and purification processes of therapeutic antibodies about 15% or more of the molecules form without $C_H3$ domain intra-molecular disulfide bonding. The lack of this intra-molecular disulfide bond reduces stability of the $C_H3$ domain by about 4 kcal/mol and also can cause aggregate formation due to the free thiol groups forming disulfide bonds with other molecules. Conversion of $C_H3$ Pro38 to an amino acid residue that reduces or eliminates the Pro *trans* to *cis* isomerization during folding enhances $C_H3$ domain folding kinetics and correct intra-molecular disulfide bond formation. A particularly useful amino acid for substitution with Pro38 is glycine (Gly) because it contains only a hydrogen atom at the a carbon side chain position, which minimizes steric hindrance and inhibitory conformational transitions compared to more bulky side chains.

In one specific embodiment, substitution of Pro38 to Gly in an immunoglobulin $C_H3$ domain polypeptide results in P38G domain refolding to native states significantly faster under reducing conditions compared to wild type Pro38 domains when diluted to low denaturant concentrations. In another specific embodiment, under oxidizing conditions, the formation of the intra-molecular disulfide bond proceeds faster in a P38G $C_H3$ domain polypeptide compared to a wild type Pro38 $C_H3$ domain polypeptide. Immunoglobulins and other immunoglobulin domain containing binding polypeptides containing a P38G $C_H3$ domain polypeptide can exhibit less susceptibility to aggregation formation due to faster folding. Similarly, such P38G $C_H3$ domain containing polypeptides also can exhibit greater long-term stability due to faster and more complete intra-molecular disulfide bond formation.

The isolated polypeptides of the invention include an immunoglobulin $C_H3$ region domain having at least one Pro to Gly substitutions at $C_H3$ residue position 38. Accordingly, in specific embodiments of the invention, an isolated immunoglobulin $C_H3$ region domain polypeptide of the invention includes at least a P38G substitution or a $C_H3$ domain equivalent thereof.

As described above, substitution of Gly for Pro beneficially promotes the folding of $CH_3$ domains since the *trans* to *cis* isomerization of Pro38 is a cause of the slow folding step. While not wishing to be bound by theory, Gly is thought to be energetically favorable for increasing the folding rate when located proximal to a β strand because it is the least bulky amino acid. However, in certain embodiments, amino acids other than Gly can be substituted at $C_H3$ residue position 38. Given the teachings and guidance provided herein, those skilled in the art will understand that selection of amino acids having side chains more bulky than Gly also can facilitate an increase the folding rates compared to a Pro residue, albeit not to the same extent as a Gly residue. Other amino acids containing less bulky or non-polar amino acid side chains include, for example, Ala and Val.

Immunoglobulin $C_H3$ domains contain a conserved Pro38 residue as set forth previously. The stability, including kinetic stability, of immunoglobulins containing a $C_H3$ domain can be enhanced by substitution of Pro38 with a Gly residue, for example. Similarly, other Pro residues within an immunoglobulin $C_H3$ domain also can be substituted with a Gly residue, for example, to further augment the rate and extent of correct domain folding as well as the stability of the $C_H3$ polypeptide domain. For example, $IgG_1$ contains nine Pro residues. Substitution of one or more Pro residues in addition to Pro38 can proportionately augment the folding rate and extend, leading to a greater proportion of correctly folded domains. The additional Pro residues in, for example, $IgG_1$ which can be substituted include Pro7, Pro 10, Pro16, Pro17, Pro51, Pro59, Pro60 and Pro109. Other immunoglobulin $C_H3$ region domains contain some or all of these Pro residues and can similarly be substituted in addition to Pro38 or a $C_H3$ domain equivalent thereof. Therefore, an isolated $C_H3$ domain polypeptide can be provided having in addition to a P38G substitution, one or more $C_H3$ Pro residues substituted with Gly, for example. The number of additional Pro substitutions incorporated into a $C_H3$ domain polypeptide can include, for example, 1, 2, 3, 4, 5, 6, 7, 8, 9 or up to all Pro residues contained in an immunoglobulin $C_H3$ domain.

Similarly, in the embodiments of the invention where an immunoglobulin $C_H3$ region domain contains a $C_H3$ domain equivalent to Pro38 and/or contains a $C_H3$ domain equivalent to other Pro residues within this domain, substitution of one or more Pro residues with Gly, for example, in addition to Pro38 or its $C_H3$ domain equivalent also can be included in an isolated polypeptide of the invention. Such multiply substituted immunoglobulin $C_H3$ domain polypeptides can exhibit further enhancement in their folding rate and/or extent of correctly folded polypeptides, also leading to increased stability compared to unsubstituted or wild-type $C_H3$ domains.

Exemplified in Example I below, are immunoglobulin $C_H3$ region domain polypeptides of the invention that exhibit increases in folding rate of up to 18-fold or more. Substitution of Pro38 with Gly in an immunoglobulin $C_H3$ region domain can result in increased folding rate, for example, at least as much as about 2-fold and higher than about 30-fold or more. Accordingly, the immunoglobulin $C_H3$ region domain polypeptides of the invention can exhibit increased folding rates of about 3, 4, 5, 6, 7, 8, 9, 10, 11, 12, 13, 14, 15, 16, 17, 18, 19, 20 or more fold compared to a WT immunoglobulin $C_H3$ domain containing Pro at position 38. Additional substitution of Gly at Pro positions other than Pro38 can, for example, increase these exemplary increases in folding rates proportionally. Accordingly, immunoglobulin $C_H3$ region domain polypeptides of the invention can exhibit folding rates by as much as 25, 30, 35 or 40-fold or more compared to Pro residues at the substituted position.

The immunoglobulin $C_H3$ region domain polypeptides having a P38G substitution also can exhibit greater stability imparted by, for example, the greater rate of folding and/or extent of folding into native conformations. Greater stability and/or extent of the number of molecules within a population of immunoglobulin $C_H3$ region domain polypeptides containing a P38G substitution is a particularly useful characteristic when producing a population of $C_H3$ region domain polypeptides because it results in a more homogeneous population. The stability of a $C_H3$ region domain can be enhanced by, for example, 2, 3, 4, 5, 6, 7, 8, 9 or 10-fold or greater by including a P38G substitution. Inclusion of one or more additional Pro substitutions can further enhance the stability of an immunoglobulin $C_H3$ region domain polypeptide of the invention.

Immunoglobulin domain containing polypeptides similarly can contain a conserved $C_H3$ domain equivalent corresponding to Pro38 in an immunoglobulin $C_H3$ domain. Examples of immunoglobulin domain containing polypeptides that contain such conserved $C_H3$ domain equivalents corresponding to Pro38 include, for example, HLA1, $F_c$ gamma receptor and $\beta_2$ microglobulin. Although the exact amino acid sequence of these immunoglobulin superfamily proteins and the Pro position within their respective immunoglobulin domains will vary, conservation of a $C_H3$ domain equivalent corresponding to Pro38 can be shown by amino acid sequence alignment using methods and algorithms well known in the art. Numerous immunoglobulin domain containing polypeptides other than HLA1, $F_c$ gamma receptor and $\beta_2$ microglobulin contain a conserved Pro residue corresponding to Pro38 in an immunoglobulin $C_H3$ domain are well know in the art. Polypeptides containing these or other known immunoglobulin domains having a conserved Pro residue corresponding to Pro38 can be stabilized by substitution of the Pro38 $C_H3$ domain equivalent with a Gly residue, for example.

Immunoglobulin $C_H3$ domains are capable of forming homo-dimers. Similarly, the P38G $C_H3$ domain polypeptides and functional fragments thereof also maintain their ability to form homo-dimers with other immunoglobulin $C_H3$ domains. Homo-dimer activity with respect to a P38G $C_H3$ domain polypeptide includes homo-dimerization with other P38G polypeptides as well as homo-dimerization with WT immunoglobulin $C_H3$ domains. The enhanced stability of P38G $C_H3$ domain polypeptides can be beneficially used to augment the stability of all classes and subclasses of immunoglobulins, dimeric polypeptides within the immunoglobulin superfamily as well as other binding polypeptides designed to form complexes that include non-covalent association using immunoglobulin $C_H3$ domains.

For example, P38G $C_H3$ domain polypeptides of the invention can be incorporated into antibodies, and functional fragments thereof, to enhance stability of these multimeric complexes. For example, a $C_H3$ region domain polypeptide

of the invention can be fused to heavy and light chain variable region binding domains of immunoglobulins to produce antibody functional fragments such as a Fab or other mono- or bi-specific immunoglobulin functional fragments. A $C_H3$ region domain polypeptide of the invention also can be fused to binding molecules such as an affinity peptide or polypeptide to produce immunoglobulin domain containing binding polypeptides such as peptibodies and/or Fc fusion polypeptides. Similarly, the P38G $C_H3$ domain polypeptides can be incorporated into any antibody in lieu of a WT $C_H3$ domain or incorporated into any antibody functional fragment or binding polypeptide as a single constant region domain. Alternatively, a P38G $C_H3$ domain polypeptide can be used in combination with one or more other constant region domains to generate more stable antibodies, antibody functional fragments, peptibodies or Fc fusion polypeptides and the like, including all combinations and permutations of WT constant region domains and one or more P38G $C_H3$ domain polypeptide.

Specific examples of antibodies for incorporation of a P38G $C_H3$ polypeptide of the invention include those described below in Table 2. Exemplary antibodies listed therein specific to EGFR and applicable for the treatment of a variety of cancers include, for example, panitumumab (Vecibix™, Amgen, Inc.); cetuximab (Erbitux™; Imclone Systems, New York City); IMC-11F8 (Imclone Systems); Humax-EGFR (Genmab, Copenhagen, Denmark); matuzumab (EMD-7200; Merck KGaA, Darmstadt, Germany), and nimotuzumab (TheraCIM hR3; YM Biosciences, Mississauga, Ontario, Canada). All of the above antibodies as well as those listed in Table 2 are well known in the art and equally applicable for use with a P38G $C_H3$ polypeptide of the invention. For example, panitumumab is commercially available from Amgen and is the subject matter of the human anti-EGFR antibody described in U.S. Patent No. 6,235,883. IMC-11F8 is the subject matter of U.S. Patent No. 7,060,808 and Humax-EGFR is the subject matter of U.S. Patent Publications 20030091561 and 20030194403.

Other specific examples for the use of a P38G $C_H3$ domain to enhance stability of an antibody functional fragment include incorporation into diabodies, minibodies and miniantibodies. A diabody is a bivalent or bispecific recombinant antibody functional fragment which is a non-covalent dimer of two single-chain Fv molecules having relatively short linkers such that the $V_L$ and $V_H$ domains of one chain pair with the complementary $V_H$ and $V_L$ domain of the second chain to form two antigen binding sites. The stability of diabodies can be enhanced by incorporation of a P38G $C_H3$ domain of the invention between each $V_H$ and $V_L$ domain, for example. Diabodies are well known in the art and can be found described in, for example, Holliger et al., Proc. Natl. Acad. Sci. USA 90:6444-6448 (1993)and Carter and Merchant, Curr. Opin. Biotech. 8:449-454 (1997).

Other exemplary antibody functional fragments known in the art which can be used in conjunction with a P38G $C_H3$ domain polypeptide of the invention include minibodies and miniantibodies. A minibody is a bivalent or bispecific recombinant antibody functional fragment produced as a covalent or non-covalent dimer of two polypeptides which each contain a single chain Fv molecule fused to an antibody domain corresponding to the third constant region of an antibody heavy chain. Incorporation of a P38G $C_H3$ domain of the invention in lieu of or in addition to a WT $C_H3$ domain can further enhance stability of such minibodies. Minibodies can be found described in, for example, Holliger and Winter, Cur. Op. in Biotechnology 4:446-449 (1993) and Carter and Merchant, *supra.*

A miniantibody is a bivalent or bispecific antibody fragment that also is a dimer of two single chain Fv fragments. However, each of the single chain Fv fragments are linked via a hinge region to a dimerization domain such as a leucine zipper or helix-turn-helix motif. Incorporation of a P38G $C_H3$ domain of the invention in lieu of or in addition to the dimerization domain can further enhance stability of miniantibodies. Miniantibodies can be found described in, for example, Pack et al., Biotechnol. 11:1271 (1993) and Holliger and Winter, *supra.*

Further specific examples for the use of a P38G $C_H3$ domain to enhance stability of an a immunoglobulin domain containing binding polypeptide include incorporation into a peptibody or Fc fusion polypeptide. As described previously, peptibody refers to a molecule having an immunoglobulin constant region domain or Fc portion thereof attached to at least one peptide. An Fc fusion polypeptide contains a polypeptide attached to an immunoglobulin constant region domain or Fc portion thereof. The production of peptibodies is well known in the art and is generally described in, for example, patent publications WO 00/24782; US20030236193; US20030236193, US20070072801 and in the patent publications described previously. The production of Fc fusion polypeptides is similarly well known in the art and generally described in, for example, U.S. Patent Nos. 6,936,439; 6,294,170 and U.S. Patent application 20070020284, and in the patent publications described previously. Specific examples of peptibodies include those directed to human angiopoietin-2 described in the aforementioned publications and also is listed below in Table 3. Specific examples of Fc fusion polypeptides include Fc region polypeptides fused to, for example, leptin or leptin derivatives as described previously or, for example, Fc fusions to an interleukin (IL) receptor antagonist such as IL1 receptor antagonist (IL1RA), and are listed below in Table 4. These and other peptibodies or Fc fusion polypeptides are well known in the art and are equally applicable for use with a P38G $C_H3$ polypeptide of the invention.

An exemplary Fc region polypeptide applicable for construction of a peptibody or Fc fusion polypeptide is shown below in as SEQ ID NO:13. This human Fc region amino acid sequence aligns with the exemplary $IgG_1$ sequence shown in Figure 2 (SEQ ID NO:4) beginning at residue 104 of SEQ ID NO:4 and includes an initiator methionine (M). As described previously, the corresponding P38G residue in this Fc polypeptide is located at residue 155 (bolded and underlined). Following the teachings and guidance provided herein, an exemplary conversion of this Fc region polypeptide into a P38G $C_H3$ domain containing polypeptide of the invention is shown below as SEQ ID NO:14 where Pro155 is substituted with a glycine (G) to yield G155 (bolded and underlined).

Fc Sequence (SEQ ID NO:13):

MDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFY**P**SDIAVEWESNGQPE
NNYKTTPPVLDSDGSFFLYSKLTVDKSRWQQGNVFSCSVMHEALHNHYTQKSLSLS
PGK

Fc Sequence Containing a P38G $C_H3$_Domain Polypeptide (SEQ ID NO:14):

MDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFY**G**SDIAVEWESNGQPE
NNYKTTPPVLDSDGSFFLYSKLTVDKSRWQQGNVFSCSVMHEALHNHYTQKSLSLS
PGK

Amino acid sequences for the exemplary peptibody and Fc fusion polypeptides described above that contain a P38G $C_H3$ domain polypeptide of the invention also are show below. The P38G position is show as a bolded and underlined glycine residue.

Sequence for Human Angiopoietin-2 Peptibody Containing a P38G Domain Polypeptide (SEQ ID NO:15):

MDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFY**G**SDIAVEWESNGQPE
NNYKTTPPVLDSDGSFFLYSKLTVDKSRWQQGNVFSCSVMHEALHNHYTQKSLSLS
PGKGGGGGAQQEECEWDPWTCEHMGSGSATGGSGSTASSGSGSATHQEECEWDPW
TCEHMLE

Sequence for a Fc Fusion to a Leptin Polypeptide Containing a P38G Domain Polypeptide (SEQ ID NO:16):

MEPKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFY**G**SDIAVEWESN

GQPENNYKTPPVLDSDGSFFLYSKLTVDKSRWQQGNVFSCSVMHEALHNHYTQKSL
SLSPGKVPIQKVQDDTKTLIKTIVTRINDISHTQSVSSKQKVTGLDFIPGLHPILTLSKM
DQTLAVYQQILTSMPSRNVIQISNDLENLRDLLHVLAFSKSCHLPWASGLETLDSLGG
VLEASGYSTEVVALSRLQGSLQDMLWQLDLSPGC

Sequence for a Fc Fusion to an IL1RA Polypeptide Containing a P38G Domain Polypeptide (SEQ ID NO:17):

MDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPA
PIEKTISKAKGQPREPQVYTLPPSRDELTKNQVSLTCLVKGFY$\underline{G}$SDIAVEWESNGQPE
NNYKTTPPVLDSDGSFFLYSKLTVDKSRWQQGNVFSCSVMHEALHNHYTQKSLSLS
PGKRPSGRKSSKMQAFRIWDVNQKTFYLRNNQLVAGYLQGPNVNLEEKIDVVPIEPH
ALFLGIHGGKMCLSCVKSGDETRLQLEAVNITDLSENRKQDKRFAFIRSDSGPTTSFE
SAACPGWFLCTAMEADQPVSLTNMPDEGVMVTKFYFQEDE

Given the teachings and guidance provided herein, those skilled in the art will understand that a P38G $C_H3$ polypeptide of the invention is equally applicable to all types of immunoglobulin family and immunoglobulin domain-containing binding polypeptides containing because of their inclusion of an immunoglobulin domain. Given the teachings and guidance provided herein, those skilled in the art also will understand that the selection of, for example, an antigen binding domain (Fv), polypeptide or other affinity binding domain and fusion with a P38G $C_H3$ polypeptide of the invention can be made based characteristics well known in the art for immunoglobulin polypeptides, including antibodies, functional fragments thereof, peptibodies and/or Fc fusion polypeptides. Accordingly, the P38G $C_H3$ polypeptides of the invention are exemplified herein and further below with reference to antibodies and antibody functional fragments. However, the range of applicability, considerations and methodology applied to antibodies and functional fragments thereof are similarly applicable to immunoglobulin superfamily members other than antibody and functional fragments. Moreover, given the teachings and guidance provided herein, those skilled in the art will understand that essentially any binding peptide or polypeptide can be incorporated into an antibody constant region domain such as an Fc fusion binding polypeptide and/or peptibody, for example, for production of a stable binding polypeptide exhibiting specific binding to its cognate binding partner. A P38G $C_H3$ polypeptide can be utilized to further enhance the stability of such peptibodies compared to a $C_H3$ domain having a wild-type Pro38 residue.

Exemplary types of binding polypeptides that can be produced in the form of a Fc fusion polypeptide, for example, and applicable for use with a P38G $C_H3$ polypeptide of the invention, include all types of therapeutic polypeptides including, for example, the immunoglobulin superfamily of polypeptides as described above, growth factors, cytokines, cell signaling molecules and hormones. Specific examples of such binding polypeptides applicable for use as a fusion with a P38G $C_H3$ polypeptide of the invention include interleukins, G-CSF, GM-CSF, TNF and TNFR ligands including Fhm, cyclins, erythropoietin, nerve growth factors (NGF), developmentally regulated nerve growth factor VGF, neurotrophic factors, neurotrophic factor NNT-1, Eph receptor ligands, Eph-like receptor ligands, inhibitors of apoptosis proteins (IAP), Thy-1 specific protein, Hek ligand (hek-L), Elk receptor ligands, STATs, collagenase inhibitor, osteoprotegerin (OPG), APRIL/G70, AGP-3/BLYS, BCMA, TACI, Her-2/neu, Apolipoprotein polyeptides, integrins, tissue inhibitor of metalloproteinases, C3b/C4b complement receptor, SHC binding protein, DKR polypeptides, extracellular matrix polypeptides, antibodies to the above therapeutic polypeptides and antibody functional fragments thereof, antibodies to receptors for the above therapeutic polypeptides and antibody functional fragments thereof, functional polypeptide fragments thereof, fusion polypeptides, chimeric polypeptides and the like.

Specific examples of commercially available therapeutic binding polypeptides that can be reconstructed as an Fc fusion polypeptide, for example, include Epoetin alfa (a mammalian cell expressed glycoprotein (Amgen, Inc.)); Interferon alfacon-1 (an *E. Coli* expressed recombinant protein (Amgen, Inc.)); anakinra (an *E.coli* expressed recombinant, nonglycosylated form of the human interleukin-1 receptor antagonist (IL-1Ra) (Amgen, Inc.)); darbepoetin alfa (a CHO expressed recombinant human erythropoiesis stimulating protein (Amgen, Inc.)); pegfilgrastim (covalent conjugate of recombinant methionyl human G-CSF and 20kD PEG (Amgen, Inc.)); Filgrastim (an *E. coli* expressed human granulocyte colony-stimulating factor (G-CSF) (Amgen, Inc.)), and Ancestim (stem cell factor; an *E.Coli* expressed recombinant human protein (Amgen, Inc.)). A specific example of a commercially available Fc fusion polypeptide is Etanercept (Amgen), which is a CHO expressed dimeric Fc fusion polypeptide linked to soluble tumor necrosis factor receptor. Soluble fusion polypeptides of TNF receptors and their methods of use are described in, for example, U.S. Patent Nos. 5,605,690. These and all other commercially available biopharmaceuticals can be, for example, fused to a P38G $C_H3$ polypeptide of the invention and produced as a peptibody having enhanced stability compared to a peptibody having a Pro38 containing $C_H3$ domain.

Incorporation can include use of recombination methods well know in the art to change one or more Pro residues, including Pro38, to a Gly residue, for example. For example, the Pro38 codon can be altered by site-directed mutagenesis and the P38G encoding nucleic acid expressed to produce a P38G $C_H3$ domain polypeptide of the invention. Alternatively, incorporation can be performed by, for example, substitution of entire $C_H3$ domain encoding nucleic acids that contain at least a P38G substitution. Encoding nucleic acids of such fusion polypeptides can similarly be expressed to produce the encoded P38G gene product. Other methods well known in the art also can be employed. Such methods are well known in the art and can be found described in, for example, Sambrook et al., Molecular Cloning: A Laboratory Manual, Third Ed., Cold Spring Harbor Laboratory, New York (2001) and Ansubel et al., Current Protocols in Molecular Biology, John Wiley and Sons, Baltimore, MD (1999).

P38G $C_H3$ domain polypeptides of the invention also can be incorporated into binding polypeptides other than antibodies which are designed to form dimeric complexes through non-covalent binding interactions. For example, the P38G $C_H3$ domain polypeptides of the invention can be used for the generation of monospecific, bispecific or multispecific binding polypeptides that associate subunits through an immunoglobulin domain. As with the antibody and antibody functional fragments exemplified above, a $C_H3$ region domain polypeptide of the invention similarly can be fused to, for example, respective binding domains of dimeric binding polypeptdes to produce immunoglobulin-like mono- or bispecific binding polypeptides having enhanced stability. For example, a P38G $C_H3$ domain polypeptide can be fused to the binding domains for MHC molecules and used to associate a and β chain binding domains into a functional binding polypeptide.

There are a wide variety of binding polypeptides based on association of binding domain containing subunits within the immunoglobulin superfamily of polypeptides and within immunoglobulin domain containing polypeptides. Specific examples of such binding polypeptides can be found described in, for example, Antibody Engineering: A Practical Guide, C.A.K. Borrebaeck, Ed., *supra;* Antibody Engineering: Methods and Protocols, in Methods in Molecular Biology, B.K.C. Lo, Ed., Humana Press, Totowa, NJ (2004), and Molecular Biology and Biotechnology: A Comprehensive Desk Reference, Robert A. Myers, Ed. VCH Publishers, Inc., New York, (1995). Given the teachings and guidance provided herein, those skilled in the art will understand that the P38G $C_H3$ domain polypeptides can be incorporated into any of such dimeric and/or multimeric binding polypeptides to stably associate or increase the stability of binding domain subunits that together form a target molecule binding domain.

Therefore, the invention provides an isolated immunoglobulin including a P38G immunoglobulin $C_H3$ region domain polypeptide of the invention, or functional fragment thereof. Also provided is a dimeric or multimeric binding polypeptide complexes associated through dimerization of a P38G $C_H3$ domain polypeptide of the invention with a second immunoglobulin $C_H3$ region domain polypeptide. The second immunoglobulin $C_H3$ region binding domain polypeptide also can include a P38G $C_H3$ domain polypeptide of the invention. The dimeric or multimeric binding polypeptides can be produced from fusion of target binding domain subunits with a P38G $C_H3$ domain polypeptide of the invention.

The invention additionally provides an isolated population of polypeptides including a plurality of immunoglobulin $C_H3$ region domain polypeptides, or functional fragments thereof, each $C_H3$ region domain polypeptide having at least one Pro to Gly substitution and exhibiting an enhanced folding rate compared to an unsubstituted $C_H3$ domain, wherein the at least one Pro to Gly substitution comprises $C_H3$ residue Pro38 or a $C_H3$ domain equivalent thereof.

Any of the immunoglobulin $C_H3$ region domain polypeptides containing at least a P38G substitution described previously can be produced as a population of P38G $C_H3$ region domain containing polypeptides. For example, an immunoglobulin, or functional fragment thereof, containing a P38G $C_H3$ domain polypeptide can be produced as population of monoclonal antibodies. Such a P38G monoclonal antibody population will contain a plurality of molecules that exhibit substantially the same binding specificity. The monoclonal population can be isolated from, for example, cells expressing P38G $C_H3$ domain polypeptides by any of a variety of methods well known in the art including, for example, specific binding activity using the target molecule binding domain. Isolated populations can be employed in a variety of diagnostic and therapeutic procedures.

Any of the immunoglobulin $C_H3$ region domain polypeptides containing at least a P38G substitution described previously also can be produced as a diverse population of different P38G $C_H3$ region domain containing polypeptides. For example, a library of immunoglobulins, or functional fragments thereof, containing a P38G $C_H3$ domain polypeptide can be produced as plurality of different antibodies species. Such a P38G antibody population will contain a plurality of molecules that exhibit a range of different binding specificity. The generation and screening of antibody libraries have a wide range of diversity is well known in the art and can be found described in, for example, Antibody Engineering: A Practical Guide, C.A.K. Borrebaeck, Ed., *supra;* Antibody Engineering: Methods and Protocols, in Methods in Molecular Biology, B.K.C. Lo, *supra,* and Molecular Biology and Biotechnology: A Comprehensive Desk Reference, R.A. Myers, *supra.* The population can be isolated from, for example, a phage or cell library expressing P38G $C_H3$ domain polypeptides exhibiting diverse binding activities conferred by fusion with different target binding domains by any of a variety of methods well known in the art including, for example, isolation of phage or cells expressing a plurality of $C_H3$ domain polypeptides of the invention or by isolation of supernatants containing such polypeptides expressed from phage or cells. Pluralities of P38G $C_H3$ domain containing polypeptides also can be screened for identification and isolation of P38G $C_H3$ domain containing antibodies, antibody functional fragments or other binding polypeptides by methods well known in the art including, for example, phage screening and panning using surface display libraries. Isolated populations of P38G $C_H3$ domain polypeptide libraries of the invention are particularly useful for identification and/or optimization of therapeutic leads.

As with the isolated P38G $C_H3$ domain polypeptides of the invention described previously, pluralities of immunoglobulin $C_H3$ region domain polypeptides containing a P38G substitution can include substitution of one or more Pro residues within the $C_H3$ domain in addition to Pro38. As with the P38G substitution, incorporation of Gly is particularly useful at these additional Pro positions because Gly contains the smallest side chain out of all 20 naturally occurring amino acids. Other amino acids containing less bulky or non-polar amino acid side chains include, for example, Ala and Val. Accordingly, a plurality of immunoglobulin $C_H3$ region domain polypeptides can be substituted at, for example, residues Pro7, Pro10, Pro16, Pro17, Pyro51, Pro59, Pro60 and Pro109, or a $C_H3$ domain equivalent thereof.

A plurality of immunoglobulin $C_H3$ region domain polypeptides contain at least a Pro38 substitution with Gly, for example, will exhibit enhanced folding rates compared to WT immunoglobulin $C_H3$ region domain polypeptides. Specific examples of enhanced folding rates are described further below in Example I where populations corresponding to single P38G species exhibited increases in folding rates of about 12-fold or 18-fold or more compared to a WT population of immunoglobulin $C_H3$ region domain polypeptides. As with the immunoglobulin $C_H3$ region domain containing polypeptides describe previously, populations P38G immunoglobulin $C_H3$ region domain polypeptides including, for example, a plurality of polypeptides of substantially the same amino acid sequence and/or a plurality of polypeptides of diverse amino acid sequences, can exhibit increased folding rates of at least as much as about 2-fold and higher than 30-fold or more. All values above, below and in between the exemplary fold increases described above and previously also can be achieved by substitution of Pro38 and/or one or more additional $C_H3$ Pro residues or a $C_H3$ domain equivalent thereof with, for example, Gly.

The fraction of correctly folded and the stability of immunoglobulin $C_H3$ region domain polypeptides also can be augmented by inclusion of a P38G substitution or by inclusion of a P38G substitution and one or more $C_H3$ Pro residues or a $C_H3$ domain equivalent thereof. Stability of a P38G population, for example, can be enhanced by, for example, 2, 3, 4, 5, 6, 7, 8, 9 or 10-fold or greater as described previously. Inclusion of one or more additional Pro substitutions can further enhance the stability of an immunoglobulin $C_H3$ region domain polypeptide of the invention.

Similarly, the fraction of immunoglobulin $C_H3$ region domain polypeptides containing a P38G substitution or $C_H3$ domain equivalent thereof can result in populations having greater than 90% correctly folded $C_H3$ domain polypeptides. Populations resulting in, for example, about 91%, 92%, 93%, 94%, 95%, 96%, 97%, 98% or 99% or higher percentages of correctly folded immunoglobulin $C_H3$ domain polypeptides also can be achieved with a P38G $C_H3$ region domain polypeptide of the invention. Inclusion of 1, 2, 3, 4, 5, 6, 7, 8, 9 or 10 or more $C_H3$ domain Pro residue substitutions additional to P38G with, for example, a Gly residue can result in proportional increases in the extent of correctly folded $C_H3$ region domain polypeptides with a population of the invention. Therefore, the P38G $C_H3$ region domain polypeptides of the invention are particularly useful for producing homogeneous and/or substantially homogeneous populations of P38G immunoglobulin $C_H3$ domain containing polypeptides. Such homogeneous and substantially homogeneous populations exhibit greater stability compared to WT Pro containing domains and can be particularly useful in a wide variety of therapeutic applications.

As described previously, the P38G $C_H3$ region domain polypeptides can be used to increase the stability of immunoglobulin populations, including antibody functional fragments, through homo-dimerization of P38G immunoglobulin $C_H3$ domain contain polypeptides of the invention and through hetero-dimerization of a P38G immunoglobulin $C_H3$ domain containing polypeptide with other immunoglobulin $C_H3$ domain containing polypeptides including, for exmple, WT $C_H3$ domain contain polypeptides. P38G immunoglobulin $C_H3$ region domain polypeptides also can be used to increase the stability of a wide variety of other binding polypeptides within the immunoglobulin superfamily and within other superfamilies of immunoglobulin domain containing binding polypeptides through enhanced homo dimerization due to greater percentages of correctly folded molecules. Thus, the stability of target binding domain subunits such as $V_H$ and $V_L$, for example, within a population can be enhanced by inclusion of one or more P38G $C_H3$ region domain polypeptides fused to these or other binding domain subunits.

Therefore, the invention provides an isolated population of P38G immunoglobulin $C_H3$ region domain polypeptides, or functional fragments thereof, that include a dimeric complex associated through non-covalent interactions between a P38G immunoglobulin $C_H3$ region domain polypeptide and a second immunoglobulin $C_H3$ region domain polypeptide. The second immunoglobulin $C_H3$ region domain polypeptide can include an immunoglobulin $C_H3$ region

domain polypeptide, or functional fragment thereof, having at least a P38G substitution and exhibiting an enhanced folding rate compared to an unsubstituted $C_H3$ domain. The second immunoglobulin $C_H3$ region domain polypeptide can include one or more additional Pro substitutions or include a WT $C_H3$ domain polypeptide. The isolated population can be a population of immunoglobulins or a population of fusion polypeptides having a P38G $C_H3$ region domain polypeptide fused to a target binding domain.

The invention also provides a method of producing a dimeric binding polypeptide. The method includes combining a first polypeptide comprising an immunoglobulin $C_H3$ region domain polypeptide, or functional fragment thereof, having a Pro to Gly substitution at $C_H3$ domain position 38 (P38G) and a target binding domain with a second polypeptide comprising an immunoglobulin $C_H3$ region domain polypeptide, or functional fragment thereof, and a target binding domain, wherein the immunoglobulin $C_H3$ region domain having a P38G substitution exhibits an enhanced folding rate compared to an unsubstituted $C_H3$ domain.

Dimeric polypeptides and populations of dimeric polypeptides of the invention can be produced by self assembly of a P38G immunoglobulin $C_H3$ domain polypeptide with a second immunoglobulin $C_H3$ domain polypeptide. The second immunoglobulin $C_H3$ domain polypeptide can be, for example, a P38G immunoglobulin $C_H3$ domain polypeptide, a WT $C_H3$ domain polypeptide of any of a variety of immunoglobulin classes or subclasses. The polypeptides be produced as a fusion with a target binding domain as described previously to produce dimeric binding polypeptides stabilized by non-covalent interactions between a P38G immunoglobulin $C_H3$ domain and a second immunoglobulin $C_H3$ domain.

A variety of approaches and avenues of construction can be employed to generate a dimeric binding polypeptide of the invention. The end result of any approach is the joinder of heterologous binding domain sequences with a P38G immunoglobulin $C_H3$ domain sequence for two subunits and association of the fusion subunits that includes dimerization of $C_H3$ domains. The association or linkage of heterologous sequences can be generated by, for example, the insertion of an amino acid substitution at least at Pro38 in an immunoglobulin or, for example, the fusion of a heterologous binding domain at a terminus of a P38G immunoglobulin $C_H3$ domain. Additional immunoglobulin constant region domains such as $C_H1$ and/or $C_H2$ also can be included in the dimeric binding polypeptides of the invention. The sequences for either of the components of a dimeric binding polypeptide of the invention can be derived, for example, wholly or partially from other polypeptides or domains, generated *de novo* or a combination thereof.

Association of first and second polypeptides can be accomplished using any of a variety of methods well known in the art. For example, the binding polypeptide subunits can be produced separately and combined into a single mixture to allow the subunits to self assemble into dimeric species. Alternatively, the binding polypeptide subunits can be co-expressed using any of a variety of well known recombinant expression systems. Co-expression of encoding nucleic acids will result in concomitant self-assembly of the subunit polypeptides. Vectors, methods and host cells for the co-expression of subunit binding polypeptides, including immunoglobulins and functional fragments thereof are well known in the art and can be found described in, for example, Antibody Engineering: A Practical Guide, C.A.K. Borrebaeck, Ed., *supra;* Antibody Engineering: Methods and Protocols, in Methods in Molecular Biology, B.K.C. Lo, *supra,* and Molecular Biology and Biotechnology: A Comprehensive Desk Reference, R.A. Myers, *supra.* Exemplary methods and systems also are described further below.

With respect to methods for constructing dimeric binding polypeptides, approaches can include insertion, substitution or directed changes of amino acid sequences. Approaches for inclusion of secondary functional characteristics can include, for example, the fusion or attachment of functional domains, polypeptides or other moieties. Methods to implement such approaches can include, for example, recombinant construction and *in vitro* or *in vivo* synthesis, chemical synthesis, conjugation, linkers as well as the use of domains corresponding to affinity binding partners. Various other approaches and methods well known in the art can similarly be utilized to design and construct a dimeric binding polypeptide of the invention.

Methods for substitution of amino acid residues can be performed at the level of the encoding nucleic acid or directly at the polypeptide level. In the former method, a nucleic acid encoding the desired polypeptide is generated and then the polypeptide is produced by, for example, *in vitro* translation or *in vivo* biosyntesis in a host cell or organism, both of which are well known in the art. In the latter method, the amino acid sequence corresponding to a dimeric binding polypeptide of interest can be synthesized directly.

Method for constructing an encoding nucleic acid also are well known in the art. For example, encoding nucleic acids can be produced by any method of nucleic acid synthesis known to those skilled in the art. Such methods include, for example, chemical synthesis, recombinant synthesis, enzymatic polymerization and combinations thereof. These and other synthesis methods are well known to those skilled in the art.

For example, methods for synthesizing polynucleotides can be found described in, for example, Oligonucleotide Synthesis: A Practical Approach, Gate, ed., IRL Press, Oxford (1984); Weiler et al., Anal. Biochem. 243:218 (1996); Maskos et al., Nucleic Acids Res. 20:1679 (1992); Atkinson et al., Solid-Phase Synthesis of Oligodeoxyribonucleotides by the Phosphitetriester Methods, in Oligonucleotide Synthesis 35 (M.J. Gait ed., 1984); Blackburn and Gait (eds.), Nucleic Acids in Chemistry and Biology, Second Edition, New York: Oxford University Press (1996), and in Ansubel et al., Current Protocols in Molecular Biology, John Wiley and Sons, Baltimore, MD (1999).

Recombinant and enzymatic synthesis, including polymerase chain reaction and other amplification methodologies can be found described in, for example, Sambrook et al., Molecular Cloning: A Laboratory Manual, Third Ed., Cold Spring Harbor Laboratory, New York (2001) and in Ansubel et al., (1999), *supra.*

Solid-phase synthesis methods for generating numerous different polynucleotides and other polymer sequences can be found described in, for example, Pirrung et al., U.S. Pat. No. 5,143,854 (see also PCT Application No. WO 90/15070), Fodor et al., PCT Application No. WO 92/10092; Fodor et al., Science (1991) 251:767-777, and Winkler et al., U.S. Pat No. 6,136,269; Southern et al. PCT Application No. WO 89/10977, and Blanchard PCT Application No. WO 98/41531. Such methods include synthesis and printing of arrays using micropins, photolithography and ink jet synthesis of polynucleotide arrays. Methods for efficient synthesis of nucleic acid polymers by sequential annealing of polynucleotides can be found described in, for example, in U.S. Patent No. 6,521,437, to Evans.

Similarly, chemical synthesis of polypeptides also is well known to those skilled in the art. Accordingly, a dimeric binding polypeptide of the invention can be synthesized directly using any of a variety of methods well known in the art. Such methods include, for example, those previously set forth and described in Roberts and Vellaccio (Academic Press, Inc.), *supra*; Wilson and Czamik (John Wiley & Sons Inc.), *supra*; Manfred E. Wolff (John Wiley & Sons Inc.), *supra*; U.S. Patent Nos. 5,420,109; 5,849,690; 5,686,567; 5,990,273; in PCT publication WO 01/00656, *supra,* and in M. Bodanzsky (Springer-Verlag), *supra,* and Stewart and Young (Pierce Chemical Co.), *supra.*

Alternatively, binding activity and/or $C_H3$ domain Pro residue substitutions can be generated *de novo* by altering one or more regions within a target binding domain polypeptide or by selected or random alterations of $C_H3$ domain Pro residues. The altered polypeptides can then be screened for a desired binding activity or for enhancement in the folding rate or enhancement in the disulfide formation or extent of the substituted $C_H3$ domains. Alteration of an amino acid sequence within a target binding domain or for substitution of $C_H3$ domain Pro residues can include, for example, from one to all twenty different naturally occurring amino acids at one or more positions. The alterations also can include, for example, incorporation of amino acid analogues and mimetics at any or all positions. The production of populations of dimeric binding polypeptides of the invention molecules having altered amino acid sequences and subsequent identification of binding activity is well known to those skilled in the art. Accordingly, using the teachings and guidance provided herein a dimeric binding polypeptide can be generated *de novo* by producing a library of altered P38G $C_H3$ domain fusion sequences and then screening the library population for an increase in P38G $C_H3$ domain stability or folding rate or for a desired binding activity.

The dimeric binding polypeptides generated and assembled as described above, for example, can be screened for the ability to bind a target molecule of interest, screened for enhanced P38G $C_H3$ domain stability, rate of folding or formation of the disulfide bond or screened for both binding and enhanced stability, rate of folding or formation of the disulfide bond.

For example, encoding nucleic acids can be cloned into an appropriate vector for propagation, manipulation and expression. Such vectors are known or can be constructed by those skilled in the art and should contain expression elements sufficient for the transcription, translation, regulation, and if desired, sorting and/or secretion of the dimeric binding polypeptides. The vectors also can be for use in either procaryotic or eukaryotic host systems so long as the expression and regulatory elements are of compatible origin. The expression vectors can additionally included regulatory elements for inducible or cell type-specific expression. One skilled in the art will know which host systems are compatible with a particular vector and which regulatory or functional elements are sufficient to achieve expression of the polypeptides in soluble, secreted or cell surface forms.

Appropriate host cells, include for example, bacteria and corresponding bacteriophage expression systems, yeast, avian, insect and mammalian cells. Methods for recombinant expression, screening and purification of dimeric binding polypeptides in various host systems are well known in the art and are described, for example, in Sambrook et al., *supra,* and in Ansubel et al., *supra.* The choice of a particular vector and host system for expression and screening or for large-scale production of dimeric binding polypeptides of the invention are known by those skilled in the art and will depend on the preference of the user.

Screening for enhanced stability, rate of folding or formation of the disulfide bond can be accomplished using methods such as those exemplified in Example I. Screening for target molecule binding specificity can be accomplished using various methods well known in the art for determining the binding affinity of a polypeptide or compound. Additionally, methods based on determining the relative affinity of binding molecules to their WT counterpart by comparing the amount of binding between a P38G immunoglobulin $C_H3$ domain containing polypeptide and and an unaltered $C_H3$ domain, for example, can similarly be used for the identification of a predetermined binding species. All of such methods can be performed, for example, in solution or in solid phase. Moreover, various formats of binding assays are well known in the art and include, for example, immobilization to filters such as nylon or nitrocellulose; two-dimensional arrays, enzyme linked immunosorbant assay (ELISA), radioimmuno assay (RIA), panning and plasma resonance. Such methods can be found described in, for example, Sambrook et al., *supra,* and Ansubel et al. Methods for measuring the affinity, including association and disassociation rates using surface plasma resonance are well known in the art and can be found described in, for example, Jönsson and Malmquist, Advances in Biosnsors, 2:291-336 (1992) and Wu et al. Proc.

Natl. Acad. Sci. USA, 95:6037-6042 (1998). Moreover, one apparatus well known in the art for measuring binding interactions is a BIAcore 2000 instrument which is commercially available through Pharmacia Biosensor, (Uppsala, Sweden).

Using any of the above described screening methods, as well as others well known in the art, P38G $C_H3$ domain containing polypeptide be identified by detecting the binding of a dimeric binding polypeptide of the invention within a population to a predetermined target molecule. Those skilled in the art will know, or can determine using the teachings and guidance provided herein, which methods can be used to construct or facilitate the construction of any of the various dimeric binding polypeptides of the invention.

The invention also provides an effective amount of a binding polypeptide comprising an immunoglobulin $C_H3$ region domain polypeptide, or functional fragment thereof, for the treatment of a pathological condition characterized by aberrant cell growth, said immunoglobulin $C_H3$ region domain polypeptide, or functional fragment thereof having a Pro to Gly substitution at $C_H3$ domain position 38 fused to a target binding domain selective for a target molecule expressed on the surface of cells mediating the pathological condition, wherein the immunoglobulin $C_H3$ region domain having a P38G substitution exhibits an enhanced folding rate compared to an unsubstituted $C_H3$ domain.

The effective amount of a binding polypeptide of the invention for treating conditions characterized by aberrant cell growth is applicable to a wide variety of pathologies because a P38G immunoglobulin $C_H3$ domain polypeptide can be used to complex and/or stabilize, for example, the binding domains of antibodies, antibody functional fragments and a variety of other polypeptide binding domains. One or more of these binding domain polypeptides having selective binding activity to a target molecule or target cell mediating a pathological conditions can be selected and fused to one or more P38G immunoglobulin $C_H3$ domain polypeptides of the invention as described previously. Once constructed, the P38G $C_H3$ binding polypeptides will exhibit, for example, enhanced folding rate and stability due to the inclusion of a P38G $C_H3$ domain polypeptide. Such P38G binding polypeptides of the invention can be used to treat the same or similar pathological condition as treated with the donor of the binding domain polypeptides.

By exemplification with reference to antibody biopharmaceuticals currently approved or under review for administering to humans, a P38G immunoglobulin $C_H3$ domain polypeptide of the invention can be substituted for one or both of the WT $C_H3$ domains contained in any of these antibody biopharmaceuticals to enhance the stability and homogeneity of the preparation. Alternatively, a P38G immunoglobulin $C_H3$ domain polypeptide of the invention can be newly included into one or more heavy or light chain binding domain fragments to augment the stability and homogeneity of the antibody functional fragment. Set forth below in Table 2 are antibody and antibody functional fragments currently approved or under review. Set forth below in Tables 3 and 4 are exemplary peptibody and Fc fusion polypeptides, respectively, currently approved or under review. As shown in the below tables, this exemplary sample of biopharmaceutical binding polypeptides are applicable for treatment of pathological conditions ranging from cancer and autoimmune diseases to obesity. The stability of any of these biopharmaceuticals can be improved by inclusion of a P38G immunoglobulin $C_H3$ domain polypeptide of the invention into one, some or all chains of these multimeric binding polypeptides. These improved biopharmaceuticals can be used

for the treatment of the same pathological condition as was treated with the parent or non-P38G $C_H3$ containing binding polypeptide.

**Table 2. Antibody Biopharmaceuticals**

| Name | Generic Name | Manufacturer | Brief Description | Indication |
|---|---|---|---|---|
| HERCEPTIN | Trastuzumab | GlaxoSmithKline | CHO expressed humanized recombinant IgG1 monoclonal antibody | Treatment of patients with metastatic breast cancer whose tumors overexpress the HER2 protein. |
| BEXXAR | Tositumomab | Genentech | Murine IgG2a lambda monoclonal Ab | Treatment of patients with CD20 antigen-expressing relapsed or refractory, low-grade, follicular, or transformed non-Hodgkin's lymphoma. |

(continued)

| Name | Generic Name | Manufacturer | Brief Description | Indication |
|---|---|---|---|---|
| HUMIRA | Adalimumab | Abbott | Mammalian cell expression of recombinant human IgG1 monoclonal antibody | Treatment of patients with moderate to severe rheumatoid arthritis (RA) or with psoriatic arthritis (PsA). RA is an inflammatory disease of the joints. PsA is an inflammatory disease of the joints and skin. |
| RITUXAN | Rituximab | Biogen Idec and Genentech | CHO expressed humanized IgG1 monoclonal antibody | Treatment of patients with relapsed or refractory, low-grade or follicular, CD20-positive, B-cell, non-Hodgkin's lymphoma, diffuse large B-cell (DLBCL), CD20-positive, non-Hodgkin's lymphoma in combination with CHOP (cyclophosphamide, doxorubicin, vincristine and prednisone) or other anthracycline-based chemotherapy regimens |
| ORTHOCLONE OKT | Muromonab-CD3 | Ortho Biotech | Murine monoclonal antibody | Treatment of acute renal allograft rejection, and acute cardiac and hepatic allograft rejection |
| REMICADE® | Infliximab | Centocor | Recombinant chimeric IgG I [kgr] monoclonal antibody | Treatment of patients with moderate to severe active rheumatoid arthritis. |
| REOPRO | Abciximab | Eli Lilly | Mammalian cell culture expressed Fab fragment of a chimeric human-murine monoclonal antibody | Treatment of acute ischemic stroke; as an adjunct to percutaneous coronary intervention (PCI) for the prevention of cardiac ischemic |

(continued)

| Name | Generic Name | Manufacturer | Brief Description | Indication |
|---|---|---|---|---|
| SIMULECT | Basiliximab | Novartis | CHO expressed glycoprotein, chimeric monoclonal antibody IgG1 | Prophylatic treatment of acute organ rejection in patients receiving renal transplantation |
| SYNAGIS® | Palivizumab | MedImmune | Humanized recombinant monoclonal antibody IgG1 | Prevention of lower respiratory tract disease caused by respiratory syncytial virus (RSV) |
| ZENAPAX® | Daclizumab | Roche Laboratories-Genentech | Recombinant humanized IgG1 monoclonal antibody | Prophylatic treatment of acute organ rejection in patients receiving renal transplants. |
| Vecibix™ | Panitumumab | Amgen, Inc | Recombinant human monoclonal antibody | Treatment of metastatic colorectal cancer |
| Erbitux™ | Cetuximab | Imclone Systems | Recombinant humanized monoclonal antibody | Treatment ofmetastatic colorectal cancer |
| IMC-11F8 | | Imclone Systems | Recombinant human monoclonal antibody | Treatment of metastatic colorectal cancer |
| Humax-EGFR | | Genmab A/S | Recombinant human monoclonal antibody | Treatment of head and neck cancer |
| EMD-7200 | Matuzumab | Merck KGaA | Recombinant humanized monoclonal antibody | Treatment of gastric and colorectal cancer |
| TheraCIM hR3 | Nimotuzumab | YM Biosciences | Recombinant humanized Monoclonal antibody | Treatment of head and neck cancer |

**Table 3. Peptibody Biopharmaceuticals**

| Name | Generic Name | Manufacturer | Brief Description | Indication |
|---|---|---|---|---|
| Angiopoietin-2 Peptibody | | Amgen, Inc. | Recombinant Fc-peptide fusion protein | Targets angiopoietins for treatment of angiogenesis-mediated diseases such as cancer |

**Table 4. Fc Fusion Biopharmaceutical**

| Name | Generic Name | Manufacturer | Brief Description | Indication |
|---|---|---|---|---|
| Fc-leptin | | Amylin Pharmaceuticals | Fe fusion to leptin polypeptide | Treatment of obesity, diabetes, high blood lipid level, arterial sclerosis, arterial plaque, gall stone formation, insufficient lean tissue mass, insufficient sensitivity to insulin, and stroke |
| FcIL1RA | | Amgen, Inc. | Fe fusion to IL1 receptor $\alpha$ | Treatment of inflammatory and other disorders |

In further embodiments, application of the effective amount of a binding polypeptide of the invention recognize that there are two broad categories of target cells or tissues that can be targeted for elimination in treating or reducing the severity of a pathological condition. One category consists of pathological conditions derived from, or associated with, cells or tissues that are non-essential for survival or maintenance of the quality of life of an individual. Exemplary of such tissues include, for example, the prostate and breast. In the other category are pathological conditions derived from or associated with cells or tissues that perform essential functions for the survival or maintenance of the quality of life of an individual. Cells and tissues performing essential functions include, for example, the brain, liver, lung and hematopoietic system. The effective amount of a binding polypeptide of the invention for treating a pathological condition are employed whether the target cells associated with the pathological condition are within essential or non-essential tissue.

In this regard, an effective amount of a P38G $C_H3$ domain containing binding polypeptide selective for a target molecule expressed on the surface of cells mediating the pathological condition is administered to the patient to inhibit aberrant cell growth. Target cells include, for example, those cells mediating the pathological condition and/or those cells exhibiting deleterious pathological effects. Such cells are characterized by aberrant regulation or cell growth, for example. Contacting these cells, by administration to the individual, one or more P38G immunoglobulin $C_H3$ domain containing binding polypeptides of the invention selective for a cell surface molecule will result in selective inhibition of these cells through a variety of mechanisms well known in the art with concomitant treatment or reduction in the severity of the disease. Exemplary mechanisms for inhibition of aberrant cell growth include, for example, inhibition of signal transduction pathways through receptor binding, induction of apoptosis through receptor binding, induction of antibody mediated cell cytotoxicity, T-cell mediated cytotoxicity and/or the delivery of cytotoxic and/or cytostatic agents to the target cell or tissue.

An effective amount of a binding polypeptide having an immunoglobulin $C_H3$ region domain polypeptide, or functional fragment thereof, containing a P38G substitution is an amount of binding polypeptide sufficient to effect a decrease in the amount of target cell number or rate of proliferation or a decrease in at least one symptom of the pathological condition when administered to an individual. The dosage of binding polypeptide required to be therapeutically effective will depend, for example, on the pathological condition characterized by aberrant cell growth to be treated, the route and form of administration, the weight and condition of the individual, and previous or concurrent therapies. The appropriate amount considered to be an effective dose for a particular application of the method can be determined by those skilled in the art, using the guidance provided herein. For example, the amount can be extrapolated from *in vitro* or *in vivo* assays as described previously. One skilled in the art will recognize that the condition of the patient can be monitored throughout the course of therapy and that the amount of the P38G $C_H3$ domain containing binding polypeptide that is administered can be adjusted accordingly.

A binding polypeptide of the invention will, in general, be prepared according to pharmaceutical standards and using pharmaceutical grade reagents. Similarly, a binding polypeptide of the invention will, in general, be prepared using sterile reagents in a sterile manufacturing environment or sterilized following preparation. Sterile injectable solutions can be prepared using well known procedures in the art including, for example, by incorporating one or more binding polypeptides in the required amount in a delivery buffer and/or excipient followed by sterilization microfiltration. In the specific embodiment of sterile powders for the preparation of sterile injectable solutions, particularly useful methods of preparation include, for example, vacuum drying and freeze-drying (lyophilization). Such drying methods will yield a powder of the one or more binding polypeptides together with any additional desired components from a previously sterile-filtered solution thereof.

Administration and dosage regimens can be adjusted to provide an effective amount for an optimum therapeutic response. For example, a single bolus can be administered, several divided doses can be administered over time or the dose can be proportionally reduced or increased as indicated by the exigencies of the therapeutic situation. It can be particularly useful to formulate a binding polypeptide of the invention for intravenous, parenteral or subcutaneous injection in a unit dosage form for ease of administration and uniformity of dosage in administering an effective amount of one or more binding polypeptides. Unit dosing refers to a physically discrete amount of pharmaceutical suited as unitary dosages for the subjects to be treated; each unit contains a predetermined quantity of active biopharmaceutical calculated to produce a desired therapeutic effect.

For further exemplification, an effective amount of a binding polypeptide of the invention such as a therapeutic antibody, or functional fragment thereof, containing a P38G immunoglobulin $C_H3$ domain polypeptide can be administered, for example, more than once, at scheduled intervals over a period of time. In certain embodiments, a therapeutic antibody is administered over a period of at least a month or more including, for example, one, two, or three months or longer. For treating chronic conditions, long-term, sustained treatment is generally most effective. Shorter periods of administration can be sufficient when treating acute conditions including, for example, from one to six weeks. In general, a therapeutic antibody or other biopharmaceutical is administered until the patient manifests a medically relevant degree of improvement over baseline for the chosen indicator or indicators.

Depending on the selected binding polypeptide and indication to be treated, a therapeutically effective amount is sufficient to cause a reduction in at least one symptom of the targeted pathological condition by at least about 5%, 10%, 15%, 20%, 25%, 30%, 35%, 40%, 45%, 50%, 55% or 60% or more, relative to untreated subjects. The ability of a binding polypeptide to reduce or inhibit a symptom can be evaluated, for example, in an animal model system predictive of efficacy for the targeted condition in human. Alternatively, the ability of a biopharmaceutical formulation to reduce or inhibit a symptom can be evaluated, for example, by examining an *in vitro* function or activity of the biopharmaceutical formulation indicative of *in vivo* therapeutic activity.

Actual dosage levels of one or more binding polypeptides of the invention can be varied so as to obtain an amount of the active biopharmaceutical which is effective to achieve the desired therapeutic response for a particular patient, formulation, and mode of administration, without being toxic to the patient. One skilled in the art would be able to determine administered amounts based on factors such as the subject's size, the severity of the subject's symptoms, and the selected biopharmaceutical and/or route of administration. The selected dosage level can depend, for example, upon a variety of pharmacokinetic factors including the activity of the biopharmaceutical employed, the route of administration, the time of administration, the rate of excretion, the duration of the treatment, other drugs, compounds and/or materials used in combination with the particular compositions employed, the age, sex, weight, condition, general health and prior medical history of the patient being treated, and like factors well known in the medical arts. Particular embodiments of the present invention involve administering a therapeutic P38G $C_H3$ domain containing polypeptide such as an antibody, or functional fragment thereof, in a pharmaceutically acceptable formulation at a dosage of from about 1 ng of antibody per kg of subject's weight per day (1 ng/kg/day) to about 10 mg/kg/day, more particularly from about 500 ng/kg/day to about 5 mg/kg/day, and even more particularly from about 5 μg/kg/day to about 2 mg/kg/day, to a subject.

A physician or veterinarian having skill in the art can readily determine and prescribe the effective amount of the required binding polypeptide as exemplified above in, for example, Table 2 with reference to current binding polypeptide pharmaceuticals. For example, the physician or veterinarian can initiate doses of a binding polypeptide of the invention at levels lower than that required in order to achieve the desired therapeutic effect and gradually increase the dosage until the desired effect is achieved. In general, a suitable daily dose of a binding polypeptide of the invention will be that amount of the biopharmaceutical which is the lowest dose effective to produce a therapeutic effect. Such an effective amount will generally depend upon the factors described previously. It is particularly useful that administration be intravenous, intramuscular, intraperitoneal, or subcutaneous. If desired, the effective daily dose to achieve an effective amount of a biopharmaceutical formulation can be administered as two, three, four, five, six or more sub-doses administered separately at appropriate intervals throughout the day, optionally, in unit dosing amounts.

A binding polypeptide of the invention can be administered, for example, with medical devices known in the art. For example, in a particularly useful embodiment, a biopharmaceutical formulation of the invention can be administered with a needleless hypodermic injection device, such as the devices described in U.S. Pat. Nos. 5,399,163; 5,383,851; 5,312,335; 5,064,413; 4,941,880; 4,790,824; or 4,596,556. Examples of well-known implants and modules useful in the present invention include: U.S. Pat. No. 4,487,603, which describes an implantable micro-infusion pump for dispensing medication at a controlled rate; U.S. Pat. No. 4,486,194, which describes a therapeutic device for administering medicants through the skin; U.S. Pat. No. 4,447,233, which describes a medication infusion pump for delivering medication at a precise infusion rate; U.S. Pat. No. 4,447,224, which describes a variable flow implantable infusion apparatus for continuous drug delivery; U.S. Pat. No. 4,439,196, which describes an osmotic drug delivery system having multi-chamber compartments, and U.S. Pat. No. 4,475,196, which describes an osmotic drug delivery system. Many other such implants, delivery systems, and modules are known to those skilled in the art.

In certain specific embodiments, a binding polypeptide of the invention can additionally be formulated to facilitate selective distribution *in vivo*. For example, the blood-brain barrier (BBB) excludes many highly hydrophilic compounds. To facilitate crossing of the BBB if desired, a biopharmaceutical formulation can additionally include, for example, liposomes for encapsulation of one or more biopharmaceuticals. For methods of manufacturing liposomes, see, for example, U.S. Pat. Nos. 4,522,811; 5,374,548; and 5,399,331. The liposomes can further contain one or more moieties which are selectively transported into specific cells or organs, thus enhancing targeted delivery of a selected biopharmaceutical (see, e.g., V. V. Ranade (1989) J. Clin. Pharmacol. 29:685). Exemplary targeting moieties include folate or biotin (see, e.g., U.S. Pat. No. 5,416,016 to Low et al.); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180) or surfactant protein A receptor (Briscoe et al. (1995) Am. J. Physiol. 1233:134).

Therefore, the invention provides an effective amount of a binding polypeptide for treating a wide range of pathological conditions characterized by aberrant cell growth. An immunoglobulin or other multimeric binding polypeptide containing a P38G $C_H3$ domain polypeptide can be used to treat, for example, cancer, including prostate, breast, lung and skin cancer. Similarly, an immunoglobulin or other multimeric binding polypeptide containing a P38G $C_H3$ domain polypeptide also can be used to treat any of a variety of infectious diseases or autoimmune diseases.

It is understood that modifications which do not substantially affect the activity of the various embodiments of this invention are also included within the definition of the invention provided herein. Accordingly, the following examples are intended to illustrate but not limit the present invention.

### EXAMPLE I

### Destabilization of the Folding Transition State of an Immunoglobulin $C_H3$ Domain

This Example shows that destabilization of an immunoglobulin $C_H3$ folding transition state through substitution of proline (Pro) enhances the folding rate for correctly folded domains.

The $C_H3$ domain of antibodies plays an important role in the stability and the association of heavy chains of the antibody molecules. In the native or folded state, a $C_H3$ domain has the immunoglobulin fold (Ig-fold) characterized by two β-sheets stabilized by a conserved intra-chain disulfide bond. It has been shown that in several therapeutic antibodies including, for example, IgG1 and IgG2, as much as 15% of the $C_H3$ domains can have reduced disulfide bonds. This Example shows the folding kinetics of the reduced form of wild type (WT) and an altered form of the human IgG1 $C_H3$ domain.

Described below are studies characterizing the equilibrium stability and folding kinetics of oxidized and reduced forms of a $C_H3$ domain compared to an altered form having Pro at position 38 substituted with a Gly (P38G). An immunoglobulin $C_H3$ domain generally contains about 100-120 residues. An IgG1 $C_H3$ domain containing 114 residues was used in these studies, which is characteristic of immunoglobulin $C_H3$ domains (Figure 1; SEQ ID NO:1). This sequence also contains an additional four amino acids at the amino terminus (MGSS) and six amino acids at the carboxy terminus (HHHHHH) for expression in *Escherichia coli* and purification, respectively. The glycine at position 5 correspond to the amino acid position 224 of the human immunoglobulin gamma-1 chain (Swiss-Plot entry name IGHG1_HUMAN; accession number P01857). Both the wild type (P38) and the P38G altered forms were expressed in *E. coli* and purified using standard procedures. Briefly, recombinant polypeptides were expressed in *E.coli* and purified using Ni-NTA resin by Abgent, Inc. (San Diego, California). The purified polypeptides (approximately 1 mg/ml) were stored in 10mM acetate buffer at pH 5.0 at -80°C.

As described further below, the reduced forms of the polypeptides were prepared by unfolding the polypeptide in 3M guanidinium hydrochloride (GdmCl) and adding 2mM dithiotreitol (DTT). Briefly, unfolding was initiated by manual mixing of the native polypeptide (1.2 mg/ml polypeptide, 10mM acetate, 2mM DTT, pH 5.0) with the same buffer containing different GdmCl concentrations at 10°C. The polypeptide was refolded by dialyzing extensively against 10mM acetate, pH-5.0 buffer containing 2mM DTT. A reverse phase chromatography method was used to characterize the reduced and oxidized species and to confirm complete reduction as described below.

Fluorescence measurements, except equilibrium GdmCl denaturation, were carried out using a PTI (Birmingham, NJ) fluorometer equipped with two detection channels. The excitation wavelength was set to 280nm with bandwidth 1 nm and the emission was monitored at 357nm with bandwidth set to 20nm and 10nm on channels A and B respectively. Kinetic data obtained from both channels yielded identical rates. All measurements were carried at 21°C. For the manual mixing experiments, the dead-time (time interval between mixing and acquisition of the first data point) of measurement was between 9-11 seconds and was measured in each study. The samples were continuously stirred during the course of data acquisition to minimize photobleaching. Equilibrium denaturation data were obtained on an AVIV spectropolarimeter (Lakewood, NJ) with instrument setting similar to that used on the PTI fluorometer. The CD spectra were measured using a JASCO-J810 spectropolarimeter in a 1cm pathlength cell for the near-UV data and 0.1cm pathlength for the far-UV data. The polypeptide concentration was 0.23 mg/ml and the buffer was 2mM acetate at pH - 5.0.

Equilibrium denaturation studies were performed to assess the stability of wild type and P38G $C_H3$ domains. In the native state, the oxidized form of wild type (WT) $C_H3$ domains has been shown to be dimeric at concentrations as low as 0.1nM (Isenman et al., Biochemistry 18:3327-36 (1979). Previous studies by the inventors have shown that even the reduced form of the wild type $C_H3$ domain exists as a folded dimer under native conditions, albeit with somewhat different secondary and tertiary structure compared to the oxidized form. Similarly, both the oxidized and reduced forms of P38G were found to be dimeric under native conditions.

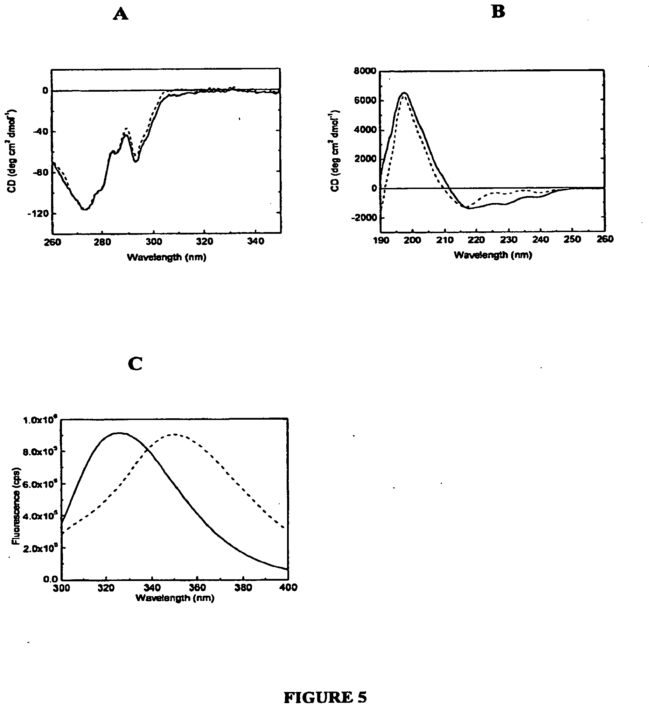

Figure 5 shows a comparison of the spectroscopic properties to assess secondary and tertiary structures of the reduced forms of WT and P38G obtained from near-UV (Figure 5a) and far-UV (Figure 5b) circular dichroism (CD) measurements. The results show that the near-UV CD spectra were identical for WT and P38G indicating that they possess similar tertiary structure. The results also show the far-UV data to appear qualitatively similar between the two molecules. However, the magnitude of the signal was weaker between 220nm and 240nm for P38G. These CD measurements indicate that the Tyr37-Pro38 peptide bond in the *cis* conformation in the WT molecule can change to Tyr37-Gly38 *trans* peptide bond in P38G since non-prolyl *cis* peptide bonds are unfavorable energetically (Ramachandran, G. N. & Mitra, A. K., J. Mol. Biol. 107:85-92 (1976); Odefey et al., J. Mol. Biol. 245, 69-78 (1995)). Since the P38G mutation is located in a turn region between two β-strands, a *cis* to *trans* backbone conformational change at this position in P38G can be accompanied by local structural changes to maintain similar tertiary structure as the WT molecule.

Because the magnitude of the far-UV CD signal was small in the above study for the native state (-1400 deg cm$^2$ dmol$^{-1}$ at 218nm for WT), fluorescence emission spectroscopy also was used to distinguish between the folded and unfolded forms of WT and the altered P38G $C_H3$ domains. Fluorescent measurements were determined as described above. The buffer conditions were 10mM acetate, pH-5.0, 2mM DTT and 0M Gdm and 4.5M Gdm for the folded and unfolded states respectively. Emission wavelength was 280nm and sample temperature was 21°C. The results for these fluorescent emission spectra of folded (solid line) and unfolded (dashed line) WT domain are shown in Figure 5c and indicate that in the folded state the emission maximum occurred at a wavelength of 325 nm which was shifted to 350 nm in the unfolded state for both WT (Figure 5c) and P38G (data not shown).

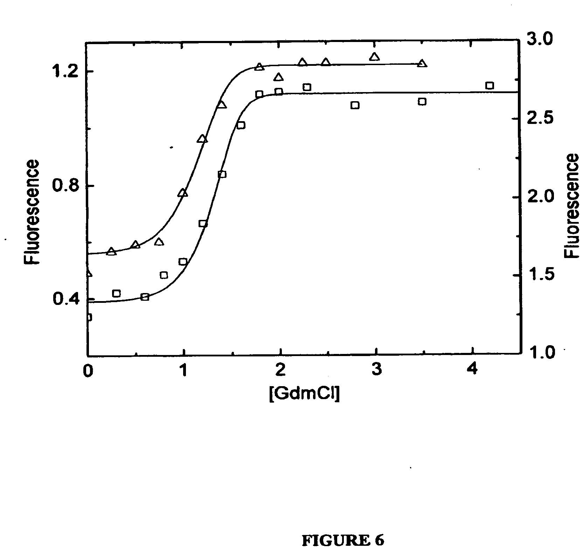

GdmCl-induced denaturation was used to determine the equilibrium stability of the WT and P38G altered form. The buffer conditions were 10mM Acetate, pH-5.0 and 2mM DTT. Polypeptide concentration was 50ug/ml in both cases. Samples were incubated at the appropriate GdmCl concentrations for ~24 hours at room temperature prior to fluorescence measurements. The excitation wavelength was set to 280nm and the emission was monitored at 357nm. The results for reduced forms of WT (□) and P38G (Δ) are shown in Figure 6 where the solid lines indicate fits to the data assuming a two-state model ($F_2$ <==> 2U).

Though the fluorescence data shown in Figure 6 indicate a simple two-state transition between folded dimer and unfolded monomer for both WT and P38G, there is some evidence from equilibrium analytical ultracentrifugation data that a folded monomer can be populated at intermediate GdmCl concentrations for the WT molecule (data not shown). Since the two tryptophan residues in the molecule are not located in the vicinity of the dimer interface, fluorescence measurements may not be able to distinguish between a folded monomer and folded dimer. From the data shown in Figure 6, estimated $C_m$ (midpoint of the transition) values were determined t be 1.3 and 1.1M GdmCl for WT and P38G respectively. Though a global analysis of the data using a three-state model involving a stable folded monomer is required to obtain the dissociation and unfolding free energies, analysis of the fluorescence data using a two-state model (folded dimer ← → unfolded monomer) yielded ΔG values of 14.4 ± 0.2 and 12.9 ± 0.6 kcal/mol for WT and P38G, respectively, which is consistent with those obtained for dimeric polypeptides (Neet, K. E. & Timm, D. E., Protein Science 3:2167-74 (1994).

Previous studies on the reduced and alkylated form of human IgG1 $C_H3$ domain (peptic fragment) indicated that this molecule does not significantly fold under native conditions of 0.5M GdmCl, which is in contrast to the results described above (Isonman et al., *supra*). Alkylation of cysteine residues buried in the hydrophobic core of the $C_H3$ domain can introduce steric hindrance that prevents folding of this molecule similar to what has been reported for a $C_L$ fragment from an antibody molecule (Ashikari et al., Biochem (Tokyo) 97:517-28 (1985). Studies on the murine $C_H3$ domain indicated similar tertiary structures for the reduced and oxidized form of the polypeptide but somewhat altered secondary structures (Thies et al., J. Mol. Biol. 319, 1267-77 (2002). The results described above indicate that while the secondary structure was somewhat affected by both reduction of the disulfide bond and the proline to glycine mutation, the tertiary structure was resistant to either of these changes. Equilibrium chemical denaturation data on the reduced form of murine $C_H3$ domain indicated that the molecule folds to the native state though with significantly reduced stability than the oxidized form (Thies et al., *supra*). The estimated equilibrium stability of the murine molecule was ~2 kcal/mol which is approximately 7-fold less than the stability obtained for the reduced WT human domain described above even though the reduced forms of the murine and human $C_H3$ domains are structurally quite similar (Saphire et al., J. Mol. Biol. 319: 9-18 (2002).

Unfolding kinetics under denaturing conditions can be described by the simple dimer two-state model:

$$F_2 \underset{k_f}{\overset{k_u}{\Leftrightarrow}} 2U \qquad \frac{d[U]}{dt} = k_u[F_2] \text{---------------- (1)}$$

and the data can be fit to the following exponential equation:

$$A = A_0 + A_1\left(1 - e^{-k_u t}\right) \text{------------------------ (2)}$$

where $[F_2]$ and $[U]$ are the concentrations of native dimer and denatured monomer, $k_u$ is the unfolding rate constant, $k_f$ is the folding rate constant ($k_u \gg k_f$ under strongly unfolding conditions), $A$ is the fluorescence signal at time $t$, $A_0$ is the fluorescence signal at $t = 0$ and $A_1$ corresponds to the fluorescence change during the unfolding reaction.

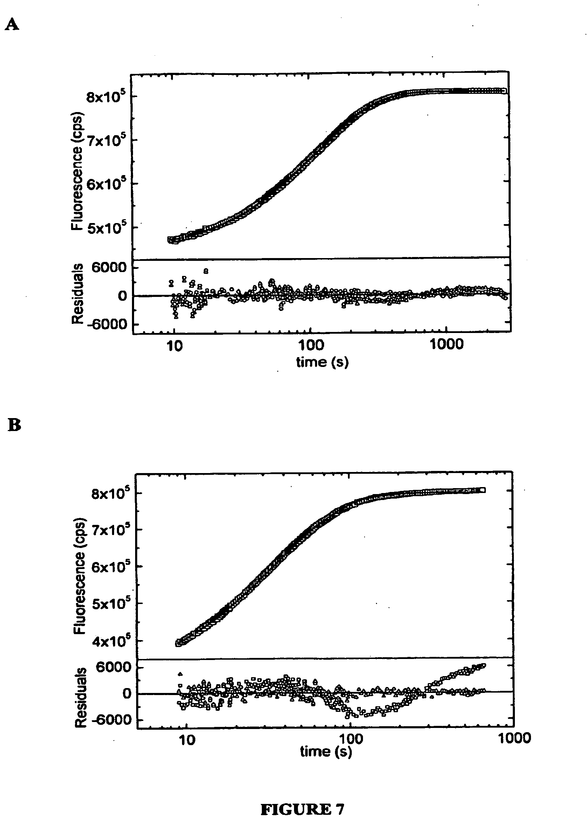

Representative unfolding kinetics data and fits to the above model are shown in Figure 7. Briefly, unfolding is initiated by mixing native polypeptide in buffer containing 10mM acetate, pH-5.0, and 2mM DTT with the same buffer containing different GdmCl concentrations at 21°C. The resulting final polypeptide concentration in most cases was 0.015 mg/ml. In order to check the concentration dependence of unfolding, a few measurements were carried out at a final polypeptide concentration of 144μg/ml. In this study, high denaturant buffer was mixed with native polypeptide to obtain final conditions of 3.5M GdmCl, 2mM DTT, and 0.015mg/ml polypeptide. The excitation wavelength was 280nm and emission was monitored at 357nm. The sample was stirred continuously during measurement at a temperature of 21 °C as set forth previously.

The lower insert in each panel of Figure 7 represents residuals obtained from fitting the data to Eq. (2) (open squares) and Eq. (3) (open triangles). The results indicate that for the WT molecule the unfolding kinetics data obtained at all GdmCl concentrations were fitted well using Eq. (2) (Figure 7a). However, in the case of P38G, fitting the data using Eq. (2) yielded slightly higher systematic residuals and fitting the data to the equation below, which includes a second kinetic phase improved the quality of the fit (Figure 7b).

$$A = A_0 + A_1(1 - e^{-k_{u1} t}) + A_2(1 - e^{-k_{u2} t}) \text{------------------ (3)}$$

As shown in Figure 7, the amplitude corresponding to the fast phase ($A_1$) remained constant between 90-95% at all GdmCl concentrations. Double exponential unfolding kinetics is not commonly observed and the source of the second kinetic phase for P38G was not further characterized. Due to the extremely long timescales involved (greater than 3 hours), unfolding to final GdmCl concentrations lower than 2.5M were not investigated.

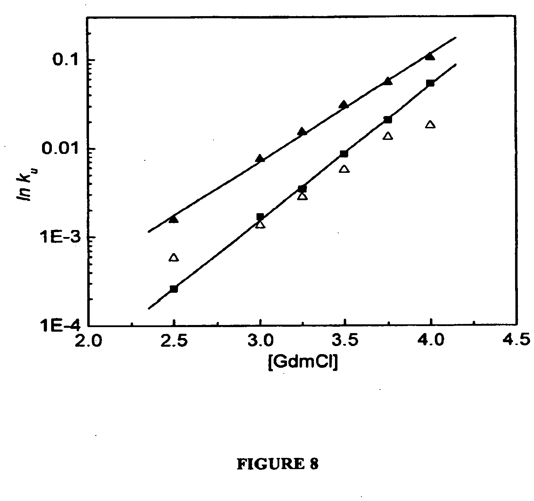

Dependence of unfolding rates on the GdmCl concentration also was characterized by assessing the natural logarithm of the unfolding rates for WT and P38G. The results are shown in in Figure 8 and indicate a linear dependence on GdmCl concentration. The rate of unfolding of WT is shown by the solid squares whereas the major unfolding phase and minor phase of P38G are shown by solid and open triangles, respectively. The amplitudes of the two unfolding phases were independent of GdmCl concentration with the major unfolding phase contributing to 90-95% of the observed fluorescence change. Linear extrapolation of the unfolding rates to zero denaturant concentration yielded unfolding rates of $4.2 \times 10^{-8}$ and $1.7 \times 10^{-6}$ s$^{-1}$ for WT and P38G respectively. The uncharacterized second unfolding phase for P38G is shown as the 5-10% amplitude. Since the equilibrium stability of the WT molecule is relatively similar to that of P38G, the faster unfolding rate observed for P38G indicates that the unfolding transition state is destabilized in the altered $C_H 3$ domain polypeptide.

Since the unfolding reaction modeled by Eq. (1) indicates a unimolecular reaction, the unfolding kinetics are independent of concentration. Unfolding rates for both WT and P38G also were measured at two different concentrations at a final GdmCl concentration of 3.5M. Briefly, native polypeptide in 10mM acetate, pH - 5.0, 2mM DTT, in the absence of GdmCl was unfolded by mixing with appropriate GdmCl buffers to obtain the final indicated conditions. The results of this analysis are summarized in Table 3 and corroborate that the unfolding kinetics were independent of concentration. Due to the relatively long timescales involved, the concentration dependence of unfolding at 2.5M GdmCl was studied only for the WT molecule.

Table 3. Amplitudes and rates of unfolding of WT and P38G at two polypeptide concentrations under strongly unfolding conditions.

|  | GdmCl (M) | Concentration (mg/ml) | $k_{u1}$ (s$^{-1}$) ($\times 10^3$) | $k_{u2}$ (s$^{-1}$) ($\times 10^3$) | A$_1$ (%) | A$_2$ (%) |
|---|---|---|---|---|---|---|
| WT | 3.5 | 0.015 | 8.6 | - | 1 | - |
|  |  | 0.144 | 8.6 | - | 1 | - |
| P38G | 3.5 | 0.015 | 30.5 | 5.7 | 94 | 6 |
|  |  | 0.144 | 30.3 | 4.2 | 95 | 5 |

(continued)

| | GdmCl (M) | Concentration (mg/ml) | $k_{u1}$ (s$^{-1}$) ($\times 10^3$) | $k_{u2}$ (s$^{-1}$) ($\times 10^3$) | $A_1$ (%) | $A_2$ (%) |
|---|---|---|---|---|---|---|
| WT | 2.5 | 0.015 | 0.26 | - | 1 | - |
| | | 0.144 | 0.28 | - | 1 | - |

In comparison, unfolding of the reduced murine $C_H3$ molecule appeared to be approximately one hundred fold faster than unfolding of the human WT $C_H3$ domain described above, which is consistent with the significantly lower equilibrium stability reported for the murine molecule. Since two unfolding kinetic phases (amplitudes of 64% and 36%) also have been observed in the unfolding of the reduced form of the murine $C_H3$ molecule (Thies et al., *supra*), these results indicate a sequential destruction of the dimer domain interface and subsequent unfolding of the monomer. The single exponential unfolding kinetics described above indicate that unfolding of the human $C_H3$ molecule occured in a single step where destruction of the domain interface and unfolding occurs simultaneously.

Comparison of the WT and P38G $C_H3$ domains also was assessed by characterization of each polypeptide's folding kinetics. Briefly, the polypeptide was completely unfolded by incubation at room temperature for ~24 hours in 10mM acetate, 4.5M GdmCl and 2mM DTT at pH-5.0. Polypeptides were refolded by mixing with buffer solutions containing low GdmCl concentrations such that the final GdmCl concentrations are between 0.06 - 0.5M.

Because the reduced form of human $C_H3$ domain is dimeric under strongly native conditions, folding of this polypeptide can be concentration-dependent. Under strongly folding conditions ($k_f \gg k_u$), the refolding reaction can be described by the following equation:

$$2U \underset{k_u}{\overset{k_f}{\Leftrightarrow}} F_2 \quad ; \quad \frac{d[F_2]}{dt} = k_f [U]^2 \dots\dots\dots (5)$$

Hence, the folding kinetics are second order in polypeptide concentration and thus are described by the hyperbolic equation set forth below (Milla, M. E. & Sauer, R. T., Biochemistry 33:1125-33 (1994); Gloss, L. M. & Matthews, C. R., Biochemistry 37:15990-15999(1998)).

$$F_2 = A_0 + A_1 \left(\frac{1}{1 + k_{app}t}\right); k_{app} = k_f.c \dots\dots\dots\dots(6)$$

In equation (6), $A_0$ is the fluorescence amplitude at time zero, $A_1$ is the fluorescence change during the refolding reaction, $k_{app}$ is the apparent rate constant, $k_f$ is the actual second-order rate constant and c is the total polypeptide concentration. In most cases fitting the data using Eq. (6) yielded large systematic residuals. This result indicates that folding of the $C_H3$ domain can be affected by the presence of the large number of proline residues which can result in multiple kinetics phases due to proline isomerization. Analysis of data using the following equation that incorporates an additional kinetic phase yielded residuals within 0.5%.

$$F_2 = A_0 + A_1 \left(\frac{1}{1 + k_{f1}t}\right) + A_2 \left(\frac{1}{1 + k_{f2}t}\right) \dots\dots\dots (7)$$

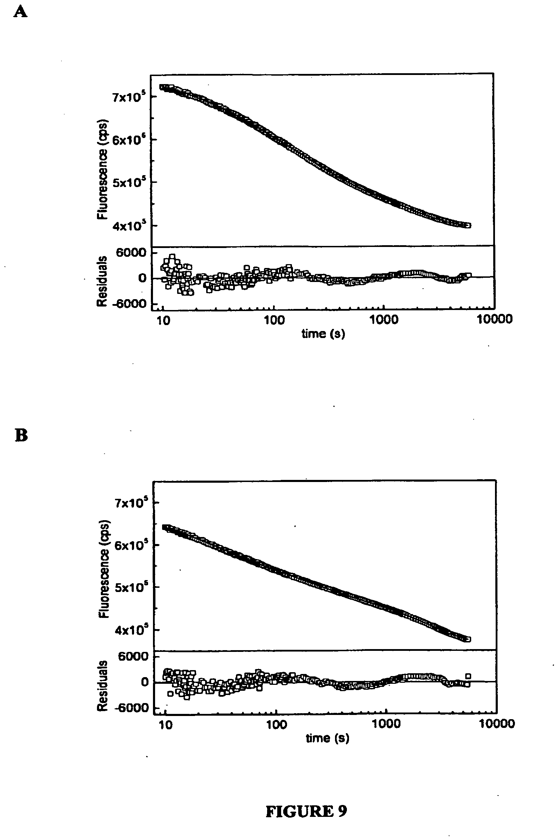

Exemplary folding kinetics that applied Eq. (7) are shown in Figure 9. Briefly, folding kinetics data obtained for (a) WT and (b) P38G at a final GdmCl concentration of 0.06M, 2mM DTT and 0.015mg/ml polypeptide concentration in 10mM acetate and pH - 5.0 at 21°C. Solid lines are fits to data using Eq. (7) and the lower insert in each panel indicate residuals obtained from the fits. Unfolded polypeptide in 10mM acetate, 2mM DTT, pH - 5.0, and 5M GdmCl was incubated at room temperature for ~24 hours was refolded by diluting to 0.06M GdmCl.

The folding kinetics data also were analyzed using conventional exponential kinetics. Three kinetic phases were required to obtain fits that were comparable to those obtained using Eq. (7). Moreover, since analysis of folding kinetics obtained at different polypeptide concentrations indicated that folding was concentration dependent (see, for example, Table 4), Eq. (7) was more appropriate to describe the folding kinetics of human $C_H3$ domain. This analysis showed that for the WT molecule at the lowest GdmCl concentrations (0.06 and 0.15M), the folding kinetics data were described well using Eq. (7) whereas at the two higher GdmCl concentrations (0.26 and 0.36M) the data were described well using a single kinetic phase (Eq. 6).

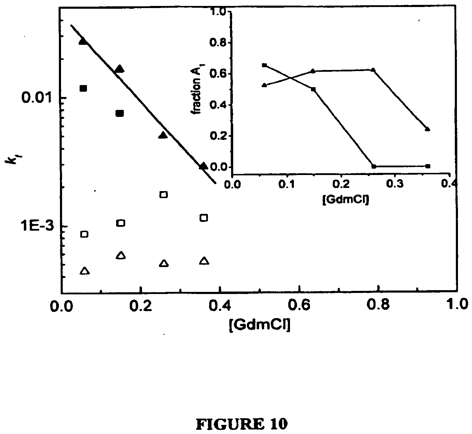

While the two kinetic phases were well-separated at the low denaturant concentrations, these rates can be similar at the higher GdmCl concentrations as shown in Figure 10, and it may not be possible to quantitatively obtain the two rates from the data. Figure 10 shows the denaturant dependence of the folding rates and amplitudes (inset) for WT and P38G $C_H3$ domain polypeptides. Solid squares and open squares denote the fast and slow rates observed for the WT molecule and solid and open triangles indicate the fast and slow rates observed for P38G. Solid squares in the inset show the amplitudes of the fast phase for WT and solid triangles represent the amplitudes of the fast phase for P38G.

This analysis indicates that in the case of P38G, the data at all GdmCl concentrations were fitted well using Eq. (7). The two rates were well-separated and the amplitude of the fast phase decreases from ~50-60% to about 25% at 0.36M GdmCl (Figure 10). For both WT and P38G the fast rate is linearly dependent on the GdmCl concentration whereas the slow rate appears to be independent of GdmCl concentration. The lack of denaturant concentration dependence of the slow phase indicates that this phase results from proline isomerization (Odefey et al., *supra*). The smaller rate for the P38G slow phase compared to WT was not further characterized. However, a comparison of the fast rates at low denaturant concentrations, indicated that the fast rate is about 2-fold faster for P38G than the WT molecule.

Folding kinetics data also were obtained under strongly folding conditions (0.06M Gdm) at two different polypeptide concentrations (0.015 and 0.14 mg/ml) for both WT and P38G. Table 4 summarizes these folding rates and amplitudes. Briefly, unfolded polypeptide in 10mM acetate, pH - 5.0, 2mM DTT, and 5.0M GdmCl was refolded as described previously by appropriate dilution of GdmCl concentration to obtain the final indicated conditions.

Table 4. Amplitudes and rates of folding of WT and P38G at two polypeptide concentrations.

|  | GdmCl (M) | Concentration (mg/ml) | $k_{f1}$ (s$^{-1}$) ($\times 10^3$) | $k_{f2}$ (s$^{-1}$) ($\times 10^3$) | $A_1$ (%) | $A_2$ (%) |
|---|---|---|---|---|---|---|
| WT | 0.06 | 0.015 | 11.8 | 0.86 | 65 | 35 |
|  |  | 0.144 | 44.5 | 0.52 | 64 | 36 |
| P38G | 0.06 | 0.015 | 27.3 | 0.44 | 52 | 48 |
|  |  | 0.144 | 73.8 | 0.40 | 44 | 56 |

The results shown in Table 4 indicate that folding of both the WT and the altered P38G are concentration dependent, which indicates a bimolecular reaction according to Eq. (5). The fast phase (amplitude ~ 65%) was approximately 3.5-fold faster at the higher polypeptide concentration for the WT molecule whereas it was approximately 2.7-fold faster (amplitude ~ 52%) for P38G. The fast phase also was approximately twice faster for P38G compared to the WT molecule (Table 4). The slow phase exhibited a weak dependence on polypeptide concentration for the WT molecule whereas for P38G the slow rates were identical at the two concentrations. The polypeptide concentration dependence observed indicates that folding and dimerization was occurring concurrently. A polypeptide concentration dependence of folding rates would not be expected if folding of monomer molecules were to occur prior to dimerization since no fluorescence change is expected due to dimerization. The slow phase that exhibits an absence of polypeptide concentration dependence can result from an alternate folding pathway where the folding rate canbe limited by proline isomerization and folding of monomers can proceed significantly before dimerization occurs.

Approximately 40% and 55% of the total fluorescence intensity change between the folded and unfolded states for WT and P38G respectively occured within the dead time (~12 -13 seconds) of the manual mixing experiments performed in this study. Despite this large fluorescence change observed within the dead-time (12-13 seconds) of the measurement, the remaining fluorescence change occured over a much longer time scale (> 1 hour). The large fluorescence change within the burst phase can result from formation of a specific folding intermediate.

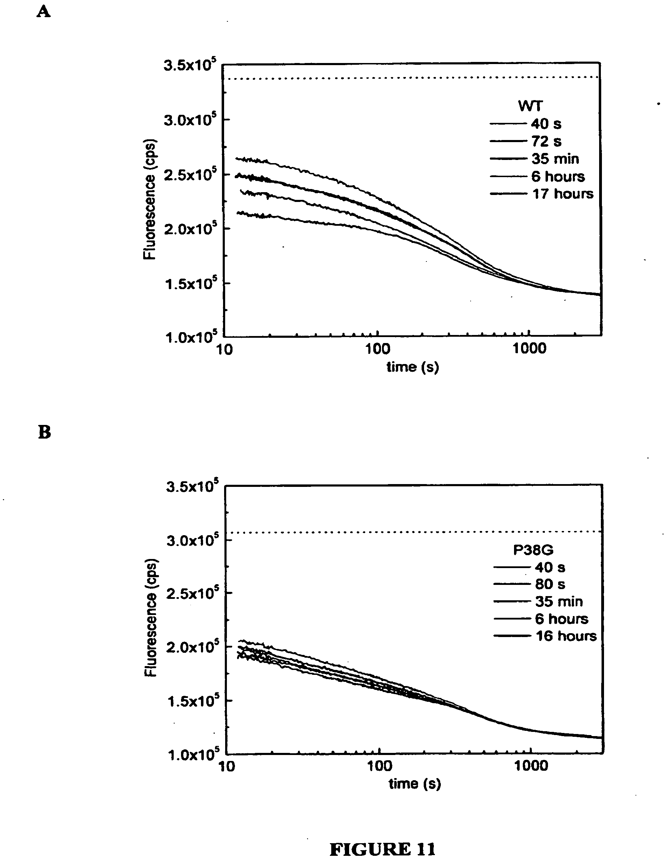

The human IgG 1 $C_H3$ domain contains nine prolines. Crystal structures of this domain from murine and human IgG molecules show that all prolines except for the one corresponding to proline at position 38 are in a *trans* conformation in the native state (Thies et al., J. Mol. Biol. 293:67-79 (1999); Saphire et al., *supra*). This proline position (corresponding to proline 38 in human IgG1 $C_H3$ domain) in the *cis* conformation in the native state is highly conserved among the immunoglobulin family of polypeptides. In order to investigate the effect of proline isomerization on folding kinetics, double jump refolding experiments were carried out where the native polypeptide was unfolded under high denaturant concentrations for varying time periods before refolding (Brandts et al., Biochemistry 14:4953-63 (1975)). Briefly, in the first step, unfolding was initiated by mixing the native polypeptide in 10mM acetate, pH - 5.0 with unfolding buffer as described previously to result in final conditions of 5M GdmCl, 2mM DTT, pH - 5.0 and a polypeptide concentration of 0.375 mg/ml. The polypeptide was allowed to unfold for varying intervals of time as noted in Figure 11 before initiating refolding by diluting the denaturant to a final GdmCl concentration of 0.2M in 2mM DTT and 0.015 mg/ml polypeptide concentration. Fluorescence intensity was monitored during the refolding reaction. At the unfolding condition used in these studies (5M GdmCl), the fluorescence intensity attained equilibrium value corresponding to the unfolded state within the dead-time (12-13 seconds) of the measurement. Fluorescence emission was monitored during refolding. Generally, since the final refolding conditions were the same at all delay times, the fluorescence intensity at the end of refolding should be the same for all samples. However, due to the relatively long refolding time (~ 60 minutes) and instrumental instability, the fluorescence intensities after refolding did not exactly match at different delay times.

Figure 11 shows the relative fluorescence intensities for both WT (panel a) and P38G (panel b) at different delay times. The dashed lines in Figure 11 also show the fluorescence intensities measured for the corresponding unfolded samples in 5M GdmCl (0.015 mg/ml unfolded polypeptide). The results indicate that for the WT molecule, the initial decrease in the fluorescence intensity was dependent on the delay-time whereas for P38G the initial change in fluorescence intensity did not depend on the delay time. Analysis of the data using Eq. (7) yielded higher systematic residuals than those obtained from analysis of the folding kinetics data at different GdmCl concentrations. Qualitatively, similar folding rates were obtained in the case of P38G at all delay times. However, for the WT molecule, the rates obtained showed some dependence on the delay time.

Comparison of the dependence of the 'burst-phase' amplitudes on the delay times between the WT and P38G molecules showed that isomerization of proline at position 38 plays a significant role in the folding of this molecule. Since the burst-phase amplitudes did not change with delay times for P38G, isomerization of all prolines other than P38 did not seem to affect folding. The burst phase amplitude for P38G remained constant at 55% at all delay times. On the other hand, the burst phase amplitude at long delay times was approximately 40% and it became closer to about 55% at short delay times (40 seconds) for the WT molecule. For the WT molecule at short unfolding delay times, proline at position 38 can still maintain the native *cis* conformation, though under the unfolding conditions employed here, the molecule unfolds within the first 10 seconds (data not shown). At longer delay times, proline at position 38 underwent isomerization to the *trans* conformation and re-isomerization to the *cis* conformation, leading to smaller changes in the burst phase amplitudes. The results indicate that the corresponding peptide bond in P38G adopts a *trans* conformation in the native state and this trans conformation resulted in constant burst phase amplitudes at all delay times. The observed burst phase amplitudes for both WT and P38G molecules were slightly higher than what can be observed in folding of polypeptides of this size. The observed burst phase amplitude can correspond to formation of a specific folding intermediate or a non-specific response of the unfolded molecule to the native like buffer conditions (Qi et al., Nature Struct. Biol. 5:882-84 (1998)). However, the dependence of burst phase amplitudes on delay times for the WT molecule was indicative of the formation of a specific folding intermediate.

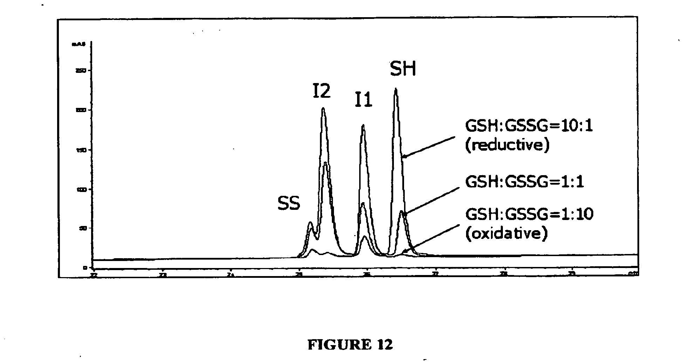

To investigate the effect of the P38G alteration on the oxidative folding of human $C_H3$, the folding of WT and P38G in the presence of reduced (GSH) and oxidized (GSSG) glutathione was monitored by revered-phase HPLC. Prior to studying oxidative folding, the effect of redox potentials on the oxidation of $C_H3$ polypeptide was analyzed. The redox potential was controlled by changing the molar ratio between GSH and GSSG in the redox buffer. Figure 12 shows the chromatograms of GdmCl unfolded WT polypeptide at various ratios of GSH and GSSG. Briefly, SH and SS in the figure represent the reduced (SH) and the oxidized (SS) forms, respectively. I1 and I2 represent the $C_H3$ polypeptide with one and two disulfide-linked glutathione molecules, respectively. The $C_H3$ polypeptides (11.5 µg/mL) were incubated in 10 mM Tris, 4 M GdmCl, pH 8.2 with different ratios as indicated in the figure of reduced glutathione (GSH) and oxidized glutathione (GSSG) at room temperature prior to chromatography.

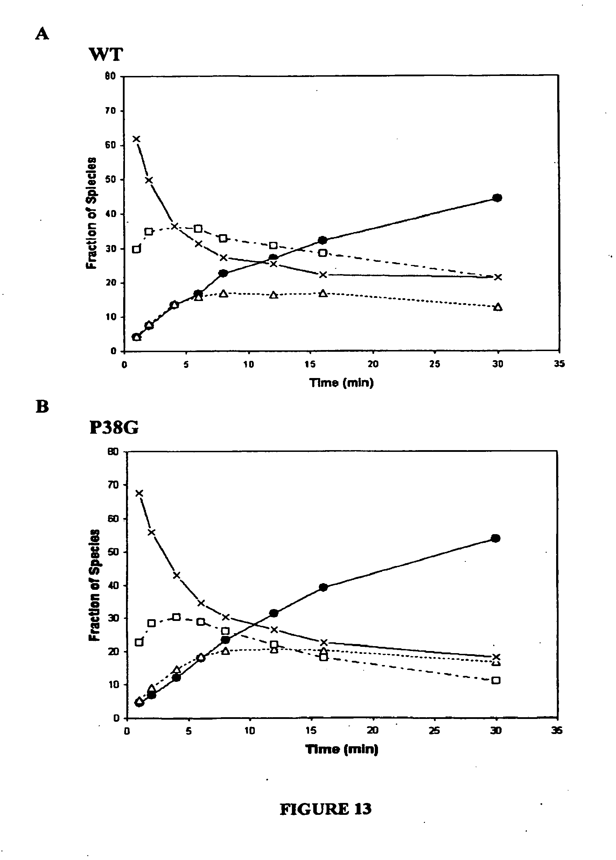

The results shown in Figure 12 reveal that in all cases, four peaks were observed. The peak eluting at 25.2 minutes and 26.5 minutes were identified as the oxidized and reduced forms respectively. However, peaks eluting between the oxidized and reduced forms (designated I1 and I2 in both Figures 12 and 13) also were observed in this study. To identify these oxidative intermediate species, their molecular masses were determined by mass spectroscopy. The measured molecular mass of I1 was larger than that of the SH form by 306 Da, indicating that I1 contained one glutathione molecule that was disulfide-linked with either one of the two free cysteine residues of the reduced $C_H3$ polypeptide. Similarly, the I2 species was identified as a species containing two disulfide-linked glutathione molecules.

Figure 12 also shows that these four species were populated at different levels depending on the redox potential. For example, when the GSH:GSSG molar ratio was 10:1, the redox buffer was reductive, whereas when the GSH:GSSG was 1:10, the buffer was oxidative. Under reducing conditions (chromatogram with GSH:GSSG = 10:1 in Figure 12), the reduced form was most dominant followed by I1 species. As the redox buffer became more oxidative (chromatogram with GSH:GSSG = 1:10), the fraction of reduced form decreased and 12 increased. In all cases, the oxidized form was a minor population among them, because a large excess of GSH (~1,000-fold than the CH3 polypeptide) promoted the inter-molecular disulfide linkage between the $C_H3$ polypeptide and GSH than the intra-molecular disulfide formation within the $C_H3$ polypeptide.

Following the above redox potential determinations, oxidative refolding of WT and the P38G altered form in the presence of GSH and GSSG at a molar ratio of 2:1 also was assessed. Briefly, both WT and P38G polypeptides at the concentration of 230 µg/mL (8.7 µM dimer) were unfolded in a buffer containing 10 mM acetate, 4 M GdmCl at pH 5. The total volume of the solution was 70 µL. The polypeptide solutions were then incubated for 40 minutes at room temperature to complete the denaturation. To initiate oxidative refolding, each denatured polypeptide was diluted into an ice-cold solution containing 100 mM Tris at pH 8.2, 5 mM reduced glutathione (GSH), and 2.5 mM oxidized glutathione (GSSG). The dilution factor was 20-fold and the total volume for refolding was 1.4 mL. The final concentrations of the polypeptide and GdmCl were 11.5 $\mu$g/mL and 0.2 M, respectively. The solution was kept in ice-water through refolding. An aliquot of 135 $\mu$L was withdrawn from the refolding solution after 1, 2, 4, 6, 8, 12, 16 and 30 minutes of refolding. The samples were immediately mixed with 15 $\mu$L of 10% trifluoroacetic acid (TAF) to stop the disulfide exchange reaction by decreasing the pH below 2. Reversed-phase high performance chromatography (HPLC) was performed on an Agilent 1100 system. Approximately 100 $\mu$L of sample was injected to an Agilent ZORBAX 300SB-C18 column (2.1x100 mm, 3.5 $\mu$m pore size, xxx A pore size), and the chromatogram was developed at 0.5 mL/min using a 16.2%-43.2% acetonitrile gradient in the presence of 0.1% TFA. Peak areas were analyzed using Agilent Chemstation software.

The oxidative refolding results are shown in Figure 13 where the key symbols correspond to: (x) SH, ($\square$) I1, ($\Delta$) I2 and ($\bullet$) SS. For both polypeptides, at the beginning of the oxidation, the fully reduced form (x) disappeared fast. After 8 minutes, the observed decrease of the reduced $C_H3$ continued very slowly. The intermediate I1 increased rapidly in the fist 8 minuets and then decreased slowly over time. The I2 species also increased rapidly in the first 8 minutes, but it did not change appreciably following that time point. However, the I2 peak is non-productive as both cysteine residues were disulfide-linked with glutathiones, preventing the intra-molecular disulfide formation between cysteine residues. The fully oxidized from increased steadily over time as the SH and I1 species decreased. Subsequently, the SS form became most abundant. After 30 minutes, WT represented ~45% of the SS form whereas P38G represented ~55% of the SS form. These observations indicate that P38G folds faster than WT under oxidative conditions as well. The I1 species, which is a direct precursor to the SS from, transiently accumulated more in WT (~35%) than in P38G (~30%). Nonetheless, the kinetics of conversion from I1 to SS was faster in P38G than in WT, again indicating that the folding kinetics of P38G is faster than WT.

In conclusion, the above studies show that the equilibrium stability of the reduced forms of both WT and P38G were comparable, witch the WT form being slightly more stable. However, he stabilities determined in the above studies were significantly higher than that of the reduced murine $C_H3$ molecule. P38G unfolded faster than the WT form and the unfolding kinetics of both molecules displayed simple two-state behavior. The folding kinetics of both molecules indicated that folding of the monomer can be coupled to dimerization. Proline at position 38 plays an important role in the folding of this polypeptide as shown by the double jump studies, though isomerization of this proline does not appear to be the main rate-limiting step in folding. Folding studies carried out under oxidative conditions also indicated that disulfide bond formation and folding is faster in the case of P38G compared to the WT molecule.

Although the invention has been described with reference to the disclosed embodiments, those skilled in the art will readily appreciate that the specific examples and studies detailed above are only illustrative of the invention.

Accordingly, the invention is limited only by the following claims.

**Claims**

1. An isolated polypeptide comprising an immunoglobulin $C_H3$ region domain polypeptide, or functional fragment thereof, having at least one Pro to Gly substitution and exhibiting enhanced stability compared to an unsubstituted $C_H3$ domain, wherein said at least one Pro to Gly substitution comprises $C_H3$ residue Pro38 wherein said isolated polypeptide further comprises an enhanced folding rate compared to an unsubstituted $C_H3$ domain, optionally wherein said enhanced folding rate comprises an increase of about 3-fold or more compared to a wild type $C_H3$ domain.

2. The isolated polypeptide of claim 1 wherein said enhanced folding rate comprises an increase selected from about 6-fold, about 12-fold and about 18-fold or more.

3. An isolated immunoglobulin comprising the immunoglobulin $C_H3$ region domain polypeptide, or functional fragment thereof, of claim 1.

4. An isolated binding polypeptide comprising the immunoglobulin $C_H3$ region domain polypeptide, or functional fragment thereof, of claim 1 fused to a target binding domain.

5. An isolated population of polypeptides, wherein said population comprises a plurality of polypeptides according to claim 1 or claim 2.

6. The isolated population of polypeptides of claim 5, wherein said population comprises a plurality of polypeptides according to claim 1, and wherein said plurality of immunoglobulin $C_H3$ region domain polypeptides, or functional fragments thereof, comprise greater than about 92% correctly folded $C_H3$ domain polypeptides, and optionally greater than about 94%, 96%, 98% or 99% correctly folded $C_H3$ domain polypeptides.

7. An isolated population of immunoglobulins comprising the plurality of immunoglobulin $C_H3$ region domain polypeptides, or functional fragments thereof, of claim 5, wherein said population of claim 5 comprises a plurality of polypeptides according to claim 1.

8. An isolated population of binding polypeptides comprising the plurality of immunoglobulin $C_H3$ region domain polypeptides, or functional fragments thereof, of claim 5, wherein said population of claim 5 comprises a plurality of polypeptides according to claim 1, fused to a target binding domain.

9. A method of producing a dimeric binding polypeptide comprising combining a first polypeptide comprising an immunoglobulin $C_H3$ region domain polypeptide, or functional fragment thereof, having a Pro to Gly substitution at $C_H3$ domain position 38 (P38G) and a target binding domain with a second polypeptide comprising an immunoglobulin $C_H3$ region domain polypeptide, or functional fragment thereof, and a target binding domain, wherein said immunoglobulin $C_H3$ region domain having a P38G substitution exhibits enhanced stability compared to an unsubstituted $C_H3$ domain.

10. The method of claim 9:

    wherein said immunoglobulin $C_H3$ domain polypeptide having a P38G substitution, or functional fragment thereof, further comprises two or more Pro to Gly substitutions; or
    wherein said immunoglobulin $C_H3$ region domain polypeptide further comprises an enhanced folding rate compared to an unsubstituted $C_H3$ domain, optionally, wherein said enhanced folding rate comprises an increase selected from about 3-fold, 6-fold, about 12-fold and about 18-fold or more.; or
    wherein said immunoglobulin $C_H3$ domain polypeptide having a P38G substitution further comprises a dimeric complex having a second immunoglobulin $C_H3$ region domain polypeptide, optionally wherein said second immunoglobulin $C_H3$ region domain polypeptide comprises an immunoglobulin $C_H3$ region domain polypeptide, or functional fragment thereof, having Pro to Gly substitution at $C_H3$ domain position 38 and exhibiting an enhanced folding rate compared to an unsubstituted $C_H3$ domain, optionally, wherein said immunoglobulin $C_H3$ domain polypeptide having a P38G substitution comprises an immunoglobulin.

11. An effective amount of a binding polypeptide comprising an immunoglobulin $C_H3$ region domain polypeptide, or functional fragment thereof, for use in the treatment of a pathological condition **characterized by** aberrant cell growth, said immunoglobulin $C_H3$ region domain polypeptide, or functional fragment thereof, having a Pro to Gly substitution at $C_H3$ domain position 38 fused to a target binding domain selective for a target molecule expressed on the surface of cells mediating the pathological condition, wherein said immunoglobulin $C_H3$ region domain having a P38G substitution exhibits enhanced stability compared to an unsubstituted $C_H3$ domain.

12. The effective amount of a binding polypeptide of claim 11, wherein said immunoglobulin $C_H3$ domain polypeptide having a P38G substitution, or functional fragment thereof, further comprises two or more Pro to Gly substitutions.

13. The effective amount of a binding polypeptide of claim 11, wherein said immunoglobulin $C_H3$ region domain polypeptide further comprises an enhanced folding rate compared to an unsubstituted $C_H3$ domain, optionally wherein said enhanced folding rate comprises an increase selected from about 3-fold, 6-fold, about 12-fold and about 18-fold or more.

14. The effective amount of a binding polypeptide of claim 11, wherein said immunoglobulin $C_H3$ domain polypeptide having a P38G substitution further comprises a dimeric complex having a second immunoglobulin CH3 region domain polypeptide; optionally wherein said second immunoglobulin $C_H3$ region domain polypeptide comprises an immunoglobulin $C_H3$ region domain polypeptide, or functional fragment thereof, having Pro to Gly substitution at $C_H3$ domain position 38 and exhibiting an enhanced folding rate compared to an unsubstituted $C_H3$ domain; optionally wherein said immunoglobulin $C_H3$ domain polypeptide having a P38G substitution comprises an immunoglobulin.

15. The effective amount of a binding polypeptide of claim 11, wherein said pathological condition: further comprises cancer; or is selected from the group consisting of prostate, breast, lung and skin cancer; or further comprises an infectious disease; or further comprises an autoimmune disease.

**Patentansprüche**

1. Isoliertes Polypeptid, umfassend ein Immunoglobulin-$C_H$3-Region-Domäne-Polypeptid oder ein funktionales Fragment davon, mit mindestens einer Pro zu Gly-Substitution und Aufzeigen einer erhöhten Stabilität im Vergleich zu einer unsubstituierten $C_H$3-Domäne, wobei die mindestens eine Pro zu Gly-Substitution den $C_H$3-Rest Pro38 umfaßt, wobei das isolierte Polypeptid weiter eine erhöhte Faltungsrate im Vergleich zu einer unsubstituierten $C_H$3-Domäne umfaßt, wobei gegebenenfalls die erhöhte Faltungsrate eine Erhöhung von etwa 3-fach oder mehr im Vergleich zu einer Wildtyp-$C_H$3-Domäne umfaßt.

2. Isoliertes Polypeptid nach Anspruch 1, wobei die erhöhte Faltungsrate eine Erhöhung ausgewählt von etwa 6-fach, etwa 12-fach und etwa 18-fach oder mehr, umfaßt.

3. Isoliertes Immunoglobulin, umfassend das Immunoglobulin-$C_H$3-Region-Domäne-Polypeptid oder ein funktionales Fragment davon nach Anspruch 1.

4. Isoliertes Bindungspolypeptid, umfassend das Immunoglobulin-$C_H$3-Region-Domäne-Polypeptid oder ein funktionales Fragment davon nach Anspruch 1, fusioniert an eine Zielbindungsdomäne.

5. Isolierte Population von Polypeptiden, worin die Population eine Mehrzahl von Polypeptiden nach Anspruch 1 oder Anspruch 2 umfaßt.

6. Isolierte Population von Polypeptiden nach Anspruch 5, wobei die Population eine Mehrzahl von Polypeptiden nach Anspruch 1 umfaßt, und worin die Mehrzahl von Immunoglobulin-$C_H$3-Region-Domäne-Polypeptiden oder funktionale Fragmente davon mehr als etwa 92% korrekt gefalteter $C_H$3-Domäne-Polypeptiden und gegebenenfalls mehr als etwa 94%, 96%, 98% oder 99% korrekt gefalteter $C_H$3-Domäne-Polypeptiden umfaßt.

7. Isolierte Population von Immunoglobulinen, umfassend die Mehrzahl von Immunglobulin-$C_H$3-Region-Domäne-Polypeptiden oder funktionale Fragmente davon nach Anspruch 5, worin die Population nach Anspruch 5 eine Mehrzahl von Polypeptiden gemäß Anspruch 1 umfaßt.

8. Isolierte Population von Bindungspolypeptiden, umfassend die Mehrzahl von Immunglobulin-$C_H$3-Region-Domäne-Polypeptiden oder funktionale Fragmente davon nach Anspruch 5, wobei die Population nach Anspruch 5 eine Mehrzahl von Polypeptiden gemäß Anspruch 1, fusioniert an eine zielbindende Domäne, umfaßt.

9. Verfahren zur Herstellung eines dimeren Bindungspolypeptid, umfassend das Kombinieren eines ersten Polypeptids, umfassend ein Immunglobulin-$C_H$3-Region-Domäne-Polypeptid oder ein funktionales Fragment davon, mit einer Pro zu Gly-Substitution bei der $C_H$3-Domänen-Position 38 (P38G) und einer zielbindenden Domäne mit einem zweiten Polypeptid, umfassend ein Immunoglobulin-$C_H$3-Region-Domäne-Polypeptid oder ein funktionales Fragment davon und eine zielbindende Domäne, wobei die Immunglobulin-$C_H$3-Region-Domäne mit einer P38G-Substitution eine erhöhte Stabilität im Vergleich zu einer unsubstituierten $C_H$3-Domäne aufweist.

10. Verfahren nach Anspruch 9,
wobei das Immunglobulin-$C_H$3-Domäne-Polypeptid mit einer P38G-Substitution oder einem funktionalen Fragment davon, weiter zwei oder mehr Pro zu Gly-Substitutionen umfaßt; oder
wobei das Immunglobulin $C_H$3-Region-Domäne-Polypeptid weiter eine erhöhte Faltungsrate im Vergleich zu einer unsubstituierten $C_H$3-Domäne umfaßt, wobei gegebenenfalls die erhöhte Faltungsrate eine Erhöhung, ausgewählt aus etwa 3-fach, 6-fach, etwa 12-fach und etwa 18-fach oder mehr, umfaßt; oder
wobei das Immunglobulin-$C_H$3-Domäne-Polypeptid mit einer P38G-Substitution weiter einen dimeren Komplex mit einem zweiten Immunglobulin-$C_H$3-Region-Domäne-Polypeptid umfaßt, wobei gegebenenfalls das zweite Immunglobulin-$C_H$3-Region-Domäne-Polypeptid ein Immunoglobulin-$C_H$3-Region-Domäne-Polypeptid oder funktionales Fragment davon mit einer Pro zu Gly-Substitution bei $C_H$3-Domänen Position 38 und das Aufzeigen einer erhöhten Faltungsrate im Vergleich zu einer unsubstituierten $C_H$3-Domäne umfaßt, wobei gegebenenfalls das Immunglobulin-$C_H$3-Domäne-Polypeptid mit einer P38G-Substitution ein Immunglobulin umfaßt.

11. Wirksame Menge eines Bindungspolypeptids, umfassend ein Immunoglobulin-$C_H$3-Region-Domäne-Polypeptid oder ein funktionales Fragment davon, zur Verwendung in der Behandlung eines pathologischen Zustands, **gekennzeichnet durch** ein aberrantes Zellwachstum, wobei das Immunglobulin-$C_H$3-Region-Domäne-Polypeptid oder ein funktionales Fragment davon eine Pro zu Gly-Substitution bei $C_H$3-Domäne in Position 38, fusioniert an eine zielbindende Domäne, welche selektiv für ein Targetmolekül ist, welches auf der Oberfläche von Zellen, welche den pathologischen Zustand vermitteln, exprimiert ist, aufweist, wobei die Immunglobulin-$C_H$3-Region-Domäne mit einer P38G-Substitution eine erhöhte Stabilität in Vergleich zu einer unsubstituierten $C_H$3-Domäne aufweist.

12. Wirksame Menge eines Bindungspolypeptids nach Anspruch 11, wobei das Immunglobulin-$C_H$3-Domäne-Polypeptid mit einer P38G-Substitution oder ein funktionales Fragment davon weiter zwei oder mehr Pro zu Gly-Substitutionen umfaßt.

13. Wirksame Menge eines Bindungspolypeptids nach Anspruch 11, wobei das Immunglobulin-$C_H$3-Region-Domäne-Polypeptid weiter eine erhöhte Faltungsrate im Vergleich zu einer unsubstituierten $C_H$3-Domäne umfaßt, wobei gegebenenfalls die erhöhte Faltungsrate eine Erhöhung, ausgewählt aus etwa 3-fach, 6-fach, etwa 12-fach und etwa 16-fach oder mehr, umfaßt.

14. Wirksame Menge eines Bindungspolypeptids nach Anspruch 11, wobei das Immunglobulin-$C_H$3-Domäne-Polypeptid mit einer P38G-Substitution weiter einen dimeren Komplex mit einem zweiten Immunglobulin-$C_H$3-Region-Domäne-Polypeptid-Domäne umfaßt; wobei gegebenenfalls das zweite Immunglobulin-$C_H$3-Region-Domäne-Polypeptid ein Immunglobulin-$C_H$3-Region-Domäne-Polypeptid oder ein funktionales Fragment davon mit einer Pro zu Gly-Substitution bei $C_H$3-Domäne in Position 38 und mit einer erhöhten Faltungsrate im Vergleich zu einer unsubstituierten $C_H$3-Domäne umfaßt; wobei gegebenenfalls das Immunglobulin-$C_H$3-Domäne-Polypeptid mit einer P38G-Substitution ein Immunglobulin umfaßt.

15. Die wirksame Menge eines Bindungspolypeptids nach Anspruch 11, wobei der pathologische Zustand weiter Krebs umfaßt; oder ausgewählt ist aus der Gruppe, bestehend aus Prostata-, Brust-, Lungen- und Hautkrebs; oder weiter eine infektiöse Krankheit umfaßt; oder weiter eine Autoimmunkrankheit umfaßt.

## Revendications

1. Polypeptide isolé comprenant un polypeptide de domaine de région $C_H$3 d'immunoglobuline ou un fragment fonctionnel de celui-ci, ayant au moins une substitution Pro en Gly et présentant une stabilité accrue par comparaison à un domaine $C_H$3 sans substitution, où ladite au moins une substitution Pro en Gly comprend le résidu Pro38 de $C_H$3, où ledit peptide isolé comprend en outre, un taux de repliement amélioré par comparaison à un domaine $C_H$3 sans substitution, le cas échéant où ledit taux de repliement amélioré comprend une augmentation d'environ 3 fois ou plus par comparaison à un domaine $C_H$3 de type sauvage.

2. Peptide isolé selon la revendication 1, où ledit taux de repliement amélioré comprend une augmentation d'environ 6 fois, d'environ 12 fois, d'environ 18 fois ou plus.

3. Immunoglobuline isolée comprenant le polypeptide de domaine de région $C_H$3 d'immunoglobuline, ou un fragment fonctionnel de celui-ci, selon la revendication 1.

4. Polypeptide de liaison isolé, comprenant le polypeptide de domaine de région $C_H$3 d'immunoglobuline, ou un fragment fonctionnel de celui-ci, selon la revendication 1, fusionné à un domaine de liaison cible.

5. Population isolée de polypeptides, où ladite population comprend une série de polypeptides selon la revendication 1 ou la revendication 2.

6. Population isolée de polypeptides selon la revendication 5, où ladite population comprend une série de polypeptides selon la revendication 1, et où ladite série de polypeptides de domaine de région $C_H$3 d'immunoglobuline, ou de fragments fonctionnels de ceux-ci, comprend plus d'environ 92% de polypeptides $C_H$3 correctement repliés, et le cas échéant plus d'environ 94%, 96%, 98% ou 99% de polypeptides de domaine $C_H$3 correctement repliés.

7. Population isolée d'immunoglobulines comprenant la série de polypeptides de domaine de région $C_H$3 d'immunoglobuline, ou de fragments fonctionnels de ceux-ci, selon la revendication 5, où ladite population de la revendication 5 comprend une série de polypeptides selon la revendication 1.

8. Population isolée de polypeptides de liaison comprenant la série de polypeptides de domaine de région $C_H$3 d'immunoglobuline, ou de fragments fonctionnels de ceux-ci, selon la revendication 5, où ladite population de la revendication 5 comprend une série de polypeptides selon la revendication 1, fusionnés à un domaine de liaison cible.

9. Procédé de production d'un polypeptide de liaison dimère, comprenant la combinaison d'un premier polypeptide comprenant un polypeptide de domaine de région $C_H3$ d'immunoglobuline, ou un fragment fonctionnel de celui-ci, ayant une substitution Pro en Gly en la position 38 (P38G) du domaine $C_H3$ et un domaine de liaison cible, avec un deuxième polypeptide comprenant un polypeptide de domaine de région $C_H3$ d'immunoglobuline, ou un fragment fonctionnel de celui-ci, et un domaine de liaison cible, où ladite région $C_H3$ d'immunoglobuline ayant une substitution P38G présente une stabilité accrue par comparaison à un domaine $C_H3$ sans substitution.

10. Procédé selon la revendication 9,
où ledit polypeptide de domaine de région $C_H3$ d'immunoglobuline ayant une substitution P38G, ou un fragment fonctionnel de celui-ci, comprend en outre, deux substitutions Pro en Gly ou plus, ou
où ledit polypeptide de domaine de région $C_H3$ d'immunoglobuline comprend un taux de repliement amélioré par comparaison au domaine $C_H3$ sans substitution, le cas échéant où ledit taux de repliement amélioré comprend une augmentation d'environ 3 fois, d'environ 6 fois, d'environ 12 fois, d'environ 18 fois ou plus, ou
où ledit polypeptide de domaine de région $C_H3$ d'immunoglobuline ayant une substitution P38G comprend en outre, un complexe dimère ayant un deuxième polypeptide de domaine de région $C_H3$ d'immunoglobuline, le cas échéant où le deuxième polypeptide de domaine de région $C_H3$ d'immunoglobuline comprend un polypeptide de domaine de région $C_H3$ d'immunoglobuline, ou un fragment fonctionnel de celui-ci, ayant une substitution Pro en Gly en la position 38 du domaine $C_H3$ et présentant un taux de repliement amélioré par comparaison à un domaine $C_H3$ sans substitution, le cas échéant où ledit polypeptide de domaine de région $C_H3$ d'immunoglobuline ayant une substitution P38G comprend une immunoglobuline.

11. Quantité efficace d'un polypeptide de liaison comprenant un polypeptide de domaine de région $C_H3$ d'immunoglobuline, ou un fragment fonctionnel de celui-ci, à utiliser dans le traitement d'un état pathologique **caractérisé par** une croissance cellulaire aberrante, ledit polypeptide de domaine de région $C_H3$ d'immunoglobuline, ou le fragment fonctionnel de celui-ci, ayant une substitution Pro en Gly en position 38 du domaine $C_H3$, est fusionné à un domaine de liaison cible sélectif d'une molécule cible exprimée à la surface des cellules intervenant dans l'état pathologique, où ledit domaine de région $C_H3$ d'immunoglobuline ayant une substitution P38G présente une stabilité accrue par comparaison au domaine $C_H3$ sans substitution.

12. Quantité efficace d'un polypeptide de liaison selon la revendication 11, où ledit polypeptide de domaine de région $C_H3$ d'immunoglobuline ayant une substitution P38G, ou un fragment fonctionnel de celui-ci, comprend en outre, deux substitutions Pro en Gly.

13. Quantité efficace d'un polypeptide de liaison selon la revendication 11, où ledit polypeptide de domaine de région $C_H3$ d'immunoglobuline comprend en outre, un taux de repliement amélioré par comparaison à un domaine $C_H3$ sans substitution, le cas échéant
où ledit taux de repliement amélioré comprend une augmentation d'environ 3 fois, 6 fois, d'environ 12 fois, d'environ 18 fois ou plus

14. Quantité efficace d'un polypeptide de liaison selon la revendication 11, où ledit polypeptide de domaine $C_H3$ d'immunoglobuline ayant une substitution P38G, comprend en outre, un complexe dimère ayant un deuxième polypeptide de domaine de région $C_H3$ d'immunoglobuline, le cas échéant où le deuxième polypeptide de domaine de région $C_H3$ d'immunoglobuline comprend un polypeptide de domaine de région $C_H3$ d'immunoglobuline, ou un fragment fonctionnel de celui-ci, ayant une substitution Pro en Gly en la position 38 du domaine $C_H3$ et présentant un taux de repliement amélioré par comparaison à un domaine $C_H3$ sans substitution ; le cas échéant où ledit polypeptide de domaine de région $C_H3$ d'immunoglobuline ayant une substitution P38G comprend une immunoglobuline.

15. Quantité efficace d'un polypeptide de liaison selon la revendication 11, où ledit état pathologique comprend le cancer, ou est choisi parmi le groupe consistant en les cancers de la prostate, du sein, de poumon et de la peau, ou comprend une maladie infectieuse, ou comprend une maladie auto-immune.

MGSSGQPREP QVYTLPPSRD ELTKNQVSLT CLVKGFYPSD IAVEWESNGQ PENNYKTTPP

VLDSDGSFFL YSKLTVDKSR WQQGNVFSCS VMHEALHNHY TQKSLSLSPG KHHHHHH

**FIGURE 1**

**Fig. 2**

```
              10        20        30        40        50        60
               |         |         |         |         |         |
IGHG2_HUMAN  ASTKGPSVFPLAPCSRSTSESTAALGCLVKDYFPEPVTVSWNSGALTSGVHTFPAVLQSS
IGHG4_HUMAN  ASTKGPSVFPLAPCSRSTSESTAALGCLVKDYFPEPVTVSWNSGALTSGVHTFPAVLQSS
IGHG1_HUMAN  ASTKGPSVFPLAPSSKSTSGGTAALGCLVKDYFPEPVTVSWNSGALTSGVHTFPAVLQSS
IGHG3_HUMAN  --------------------QMQGVNCTVSSELKTP------LGDTT---HTCPRCPEPK
                           .:.*  *..  :   *        *   *    **.*   :..

Prim.cons.   ASTKGPSVFPLAPCSRSTSESTAALGCLVKDYFPEPVTVSWNSGALTSGVHTFPAVLQSS


              70        80        90       100       110       120
               |         |         |         |         |         |
IGHG2_HUMAN  GLYSLSSVVTVPSSNFGTQTYTCNVDHKPSNTKVDKTVERKCC---VECPPCPAPPVAG--
IGHG4_HUMAN  GLYSLSSVVTVPSSSLGTKTYTCNVDHKPSNTKVDKRVESKYG---PPCPSCPAPEFLGG.
IGHG1_HUMAN  GLYSLSSVVTVPSSSLGTQTYICNVNHKPSNTKVDKKVEPKSCDKTHTCPPCPAPELLGG
IGHG3_HUMAN  ---SCD---TPPPCPRCPEPKSC--DTPPPCPRCP---EPKSCDTPPPCPRCPAPELLGG
                *  .   * *..   .:.  *  :  *. .:     * *    **.****  .*

Prim.cons.   GLYSLSSVVTVPSSSLGTQTYTCNVDHKPSNTKVDK3VEPKSCD22PPCPPCPAPELLGG


             130       140       150       160       170       180
               |         |         |         |         |         |
IGHG2_HUMAN  PSVFLFPPKPKDTLMISRTPEVTCVVVDVSHEDPEVQFNWYVDGVEVHNAKTKPREEQFN
IGHG4_HUMAN  PSVFLFPPKPKDTLMISRTPEVTCVVVDVSQEDPEVQFNWYVDGVEVHNAKTKPREEQFN
IGHG1_HUMAN  PSVFLFPPKPKDTLMISRTPEVTCVVVDVSHEDPEVKFNWYVDGVEVHNAKTKPREEQYN
IGHG3_HUMAN  PSVFLFPPKPKDTLMISRTPEVTCVVVDVSHEDPEVQFKWYVDGVQVHNAKTKPREQQFN
             ****************************:****:*:******:**********:*:*

Prim.cons.   PSVFLFPPKPKDTLMISRTPEVTCVVVDVSHEDPEVQFNWYVDGVEVHNAKTKPREEQFN


             190       200       210       220       230       240
               |         |         |         |         |         |
IGHG2_HUMAN  STFRVVSVLTVVHQDWLNGKEYKCKVSNKGLPAPIEKTISKTKGQPREPQVYTLPPSREE
IGHG4_HUMAN  STYRVVSVLTVLHQDWLNGKEYKCKVSNKGLPSSIEKTISKAKGQPREPQVYTLPPSQEE
IGHG1_HUMAN  STYRVVSVLTVLHQDWLNGKEYKCKVSNKALPAPIEKTISKAKGQPREPQVYTLPPSRDE
IGHG3_HUMAN  STFRVVSVLTVLHQNWLDGKEYKCKVSNKALPAPIEKTISKTKGQPREPQVYTLPPSREE
             **:********:**:**:***********.**:.*******:***************::*
```

Fig. 2 (cont'd.)

```
Prim.cons.    ST2RVVSVLTVLHQDWLNGKEYKCKVSNK2LPAPIEKTISK2KGQPREPQVYTLPPSREE

                      250       260       270       280       290       300
                       |         |         |         |         |         |
IGHG2_HUMAN   MTKNQVSLTCLVKGFYPSDIAVEWESNGQPENNYKTTPPMLDSDGSFFLYSKLTVDKSRW
IGHG4_HUMAN   MTKNQVSLTCLVKGFYPSDIAVEWESNGQPENNYKTTPPVLDSDGSFFLYSRLTVDKSRW
IGHG1_HUMAN   LTKNQVSLTCLVKGFYPSDIAVEWESNGQPENNYKTTPPVLDSDGSFFLYSKLTVDKSRW
IGHG3_HUMAN   MTKNQVSLTCLVKGFYPSDIAVEWESSGQPENNYNTTPPMLDSDGSFFLYSKLTVDKSRW
              :**********************************.*******:****:**********:********
Prim.cons.    MTKNQVSLTCLVKGFYPSDIAVEWESNGQPENNYKTTPP2LDSDGSFFLYSKLTVDKSRW

                      310       320       330
                       |         |         |
IGHG2_HUMAN   QQGNVFSCSVMHEALHNHYTQKSLSLSPGK
IGHG4_HUMAN   QEGNVFSCSVMHEALHNHYTQKSLSLSLGK
IGHG1_HUMAN   QQGNVFSCSVMHEALHNHYTQKSLSLSPGK
IGHG3_HUMAN   QQGNIFSCSVMHEALHNRFTQKSLSLSPGK
              *:**:**************::*******.**
Prim.cons.    QQGNVFSCSVMHEALHNHYTQKSLSLSPGK
```

**Fig. 3**

```
                    10        20        30       40        50        60
                     |         |         |        |         |         |
IGHG1_HUMAN   ASTKGPSVFPLAPSSK--STSGGTAALGCLVKDYFP-EPVTVSWNSGALT--SGVHTFPA
IGHE_HUMAN    ASTQSPSVFPLTRCCKNIPSNATSVTLGCLATGYFP-EPVMVTWDTGSLN--GTTMTLPA
IGHA1_HUMAN   ASPTSPKVFPLSLCST-QPD--GNVVIACLVQGFFPQEPLSVTWSESGQG--VTARNFPP
IGHA2_HUMAN   ASPTSPKVFPLSLDST-PQD--GNVVVACLVQGFFPQEPLSVTWSESGQN--VTARNFPP
MUC_HUMAN     GSASAPTLFPLVSCEN-SPSDTSSVAVGCLAQDFLP-DSITPSWKYKNNSDISSTRGFP-
IGHD_HUMAN    APTKAPDVFPIISGCR-HPKDNSPVVLACLITGYHP-TSVTVTWYMGTQS--QPQRTFP-
              ...  .*  :**:         ..:.**  .: *  .: .:*           :*
Prim.cons.    AST2SP2VFPLS2CS2N5P2D4G2VVL2CLVQG2FPQEPVTVTWSEGGQ2DI2T2RTFP2


                    70        80        90       100       110       120
                     |         |         |        |         |         |
IGHG1_HUMAN   -VLQSSGLYSLSSVVTVPSSSLGTQTYICNVNHKPSN---------------------TKVDKKV
IGHE_HUMAN    TTLTLSGHYATISLLTVSG-AWAKQMFTCRVAHTPSSTDWVDNKTFSVCSRDFTPPTVKI
IGHA1_HUMAN   SQDASGDLYTTSSQLTLPATQCLAG--KSVTCHVKHYT-------------NPSQDVT
IGHA2_HUMAN   SQDASGDLYTTSSQLTLPATQCPDG--KSVTCHVKHYT-------------NPSQDVT
MUC_HUMAN     -SVLRGGKYAATSQVLLPSKDVMQGTDEHVVCKVQHP--------------NGNKEKN
IGHD_HUMAN    EIQRRDSYYMTSSQLSTPLQQWRQGEYKCVVQHTASK--------------SKKEIFR
              ..  *   *  :    .                      .  :
Prim.cons.    SQ2ASGGLY2TSSQLTLP2TQ26QGTYKCVVCHV22YTDWVDNKTFSVCSRDFNPSQDKT


                    130       140       150      160       170       180
                     |         |         |        |         |         |
IGHG1_HUMAN   EPKSCDKTHTCP---------------PCPAPELLG----------------------
IGHE_HUMAN    LQSSCDGGGHFP--------------PTIQLLCLVS--GYTPGTINITWLEDGQVMDVDL
IGHA1_HUMAN   VPCPVPSTPPTPS------------PSTPP---------TPS----------------
IGHA2_HUMAN   VPCPVP-------------------P-------------P------------------
MUC_HUMAN     VPLPVIAELPPKVSVFVPPRDGFFGNPRSKSKLICQATGFSPRQIQVSWLREGKQVGSGV
IGHD_HUMAN    WPESPKAQASS-------------VPTAQPQAEGSLAKATTAPATTRNTGRGGEEKKKE
                                           *
Prim.cons.    VPC2V2AT5P5P2SVFVPPRDGFFG2PT5QP4LLGS22G3TP431333WL33G3333333


                  190       200       210      220       230       240
                   |         |         |        |         |         |
IGHG1_HUMAN   ----------------------------------------------------------
IGHE_HUMAN    ST---ASTTQEGELASTQSELTLSQK--HWLSDRTYTCQVTYQGHTFEDSTKKCADSNPR
IGHA1_HUMAN   -------------PSCCHPRLSL------------------HR----------------
IGHA2_HUMAN   -------------PPCCHPRLSL------------------HR----------------
MUC_HUMAN     TTDQVQAEAKESGPTTYKVTSTLTIKESDWLSQSMFTCRVDHRGLTFQQNASSMCVPDQD
IGHD_HUMAN    KEKEEQEERETKTPECPSHTQPLG----------------------------------

Prim.cons.    3T222Q3E33E33P5CCHP2L2L32KES2WLS2222TC2V2HRG2TF2222222222222
```

Fig. 3 (cont'd.)

```
                  250       260       270       280       290       300
                   |        .|        |         |         |         |
IGHG1_HUMAN   GPSVFLFPPKPKDTLMISRTPEVTCVVVDVSHEDPEVKFNWYVDGVEVHNAKTK-PREEQ
IGHE_HUMAN    GVSAYLSRPSPFD-LFIRKSPTITCLVVDLAPSKGTVNLTWSRASGKPVNHSTR-KEEKQ
IGHA1_HUMAN   ----------PALEDLLLGSEANLTCTLTGLRDASG-VTFTWTPSSGKSAVQGP---PERD
IGHA2_HUMAN   ----------PALEDLLLGSEANLTCTLTGLRDASG-ATFTWTPSSGKSAVQGP---PERD
MUC_HUMAN     TAIRVFAIPPSFASIFLTKSTKLTCLVTDLTTYDS-VTISWTRQNGEAVKTHTN-ISESH
IGHD_HUMAN    ----VYLLTPAVQDLWLRDKATFTCFVVGSDLKDA--HLTWEVAGKVPTGGVEEGLLERH
                               :  :    . .**  :..    .    :.*  .          *  .
Prim.cons.    G3S3VL44PPA22DL2L222A2LTC2V22LRDADG2VTFTWT32SGK222QGT4G4PER3


                  310       320       330      ·340       350       360
                   |         |        .  |        ·|        |         |
IGHG1_HUMAN   YNSTYRVVSVLTVLHQDWLNGKEYKCKVSNKALPAPIEKTIS-KAKGQPREPQVYTLPPS
IGHE_HUMAN    RNGTLTVTSTLPVGTRDWIEGETYQCRVTHPHLPRALMRSTT-KTSGPRAAPEVYAFATP
IGHA1_HUMAN   LCGCYSVSSVLPGCAEPWNHGKTFTCTAAYPESKTPLTATLS--KSGNTFRPEVHLLPPP
IGHA2_HUMAN   LCGCYSVSSVLPGCAQPWNHGETFTCTAAHPELKTPLTANIT--KSGNTFRPEVHLLPPP
MUC_HUMAN     PNATFSAVGEASICEDDWNSGERFTCTVTHTDLPSPLKQTISRPKGVALHRPDVYLLPPA
IGHD_HUMAN    SNGSQSQHSRLTLPRSLWNAGTSVTCTLNHPSLPPQRLMALREPAAQAPVKLSLNLLASS
                              .      *     *       *                 .:  :....
Prim.cons.    LNGTYSV2SVLP2CAQDWNHGETFTCTV2HPELPTPLTATIS22KSG22FRPEVYLLPPP


                  370       380       390       400       410       420
                   |         |         |·        |         |         |
IGHG1_HUMAN   RDELTK-NQVSLTCLVKGFYPSDIAVEWESNGQ--PENNYKTTPPVLDS---DGSFFLYS
IGHE_HUMAN    EWPGSR-DKRTLACLIQNFMPEDISVQWLHNEVQLPDARHSTTQPRKTK---GSGFFVFS
IGHA1_HUMAN   SEELALNELVTLTCLARGFSPKDVLVRWLQGSQELPREKYLTWASRQEPSQGTTTPAVTS
IGHA2_HUMAN   SEELALNELVTLTCLARGFSPKDVLVRWLQGSQELPREKYLTWASRQEPSQGTTTPAVTS
MUC_HUMAN     REQLNLRESATITCLVTGFSPADVFVQWMQRGQPLSPEKYVTSAPMPEP-QAPGRYFAHS
IGHD_HUMAN    DPPEAA---SWLLCEVSGFSPPNILLMWLEDQREVNTSGFAPARPPPQP--GSTTFWAWS
                          :  *   .*  *  ::  :  *       .    . .         :     *
Prim.cons.    2EELALNELVTLTCLVRGFSPKD2LV2WLQ22QELPREKYLT2APR2EPSQGTTTPFVTS


                  430       440       450.      460       470
                   |         |.        |         |         |    ·
IGHG1_HUMAN   KLTVDKSRWQQGNVFSCSVMHEALHN-HYTQKSLSLSPGK----------------------
IGHE_HUMAN    RLEVTRAEWEQKDEFICRAVHEAASPSQTVQRAVSVNPGK------------------
IGHA1_HUMAN   ILRVAAEDWKKGDTFSCMVGHEALPL-AFTQKTIDRLAGKPTHVNVSVVMAEVDGTCY
IGHA2_HUMAN   ILRVAAEDWKKGDTFSCMVGHEALPL-AFTQKTIDRLAGKPTHVNVSVVMAEVDGTCY
MUC_HUMAN     ILTVSEEEWNTGETYTCVVAHEALPN-RVTERTVDKSTGKPTLYNVSLVMSDTAGTCY
IGHD_HUMAN    VLRVPAPPSPQPATYTCVVSHEDSRTLLNASRSLEVSYVTDHGPM-------------
                * *.       : * . **      ..:::.      .
Prim.cons.    ILRVAAE2WKQGDTFSC2VGHEALP22AFTQ2T3D2S2GKPTHVNVSVVMAEVDGTCY
```

## CANCEROUS PATHOLOGICAL CONDITIONS

### HEMATOPORETIC NEOPLASMS
Lymphoid Neoplasms
Myeloid Neoplasms
Histiocytoses
Precursor B lymphoblastic leukemia/lymphoma (ALL)
Precursor T lymphoblastic leukemia/lymphoma (ALL)
Chronic lymphocytic leukemia/small lymphocytic lymphoma (SLL)
Lymphoplasmacytic lymphoma
Mantle cell lymphoma
Follicular lymphoma
Marginal zone lymphoma
Hairy cell leukemia
Plasmacytoma/plasma cell myeloma
Diffuse large B-cell lymphoma
Burkitt lymphoma
T-cell chronic lymphocytic leukemia
Large granular lymphocytic leukemia
Mycosis fungoids and sezary syndrome
Peripheral T-cell lymphoma, unspecified
Angioimmunoblastic T-cell lymphoma

Angiocentric lymphoma (NK/T-cell lymphoma)
Intestinal T-cell lymphoma
Adult T-cell leukemia/lymphoma
Anaplastic large cell lymphoma
*Hodgkin Diseases (HD)*
Acute myclogenous leukemia (AML)
Myclodysplastic syndromes
Chronic Myclofroliferative Disorders
Chronic Myclogenous Leukemia (CML)
Polycythemia Vera
Essential Thrombocytosis
Myelofibrosis with Myeloid Metaplasia
Hemangioma
Lymphangioma
Glomangioma
Kaposi Sarcoma
Hemanioendothelioma
Angiosarcoma
Hemangiopericytoma

### HEAD & NECK
Basal Cell Carcinoma
Squamous Cell Carcinoma
Ceruminoma
Osteoma
Nonchromaffin Paraganglioma
Acoustic Neurinoma
Adenoid Cystic Carcinoma
Mucoepidermoid Carcinoma
Malignant Mixed Tumors
Adenocarcinoma
Lymphoma
Fibrosarcoma
Osteosarcoma

Chondrosarcoma
Melanoma
Olfactory Neuroblastoma
Isolated Plasmocytoma
Inverted Papillomas
Undifferentiated Carcinoma
Mucoepidermoid Carcinoma
Acinic Cell Carcinoma
Malignant Mixed Tumor
Other Carcinomas
Amenoblastoma
Odontoma

### THYMUS
Malignant Thymoma
Type I (Invasive thymoma)
Type II (Thymic carcinoma)

Squamous cell carcinoma
Lymph epithelioma

**FIGURE 4**

## THE LUNG
Squamous Cell Carcinoma
Adenocarcinoma
Bronchial derived
Acinar; papillary; solid
Bronchioalveolar
Small Cell Carcinoma

Oat Cell
Intermediate Cell
Large Cell Carcinoma
Undifferentiated; giant cell; clear cell
Malignant Mesothelioma
Sarcomotoid Type
Epithelial Type

## THE GASTROINTESTINAL TRACT
Squamous Cell Carcinoma
Adenocarcinoma
Carcinoid
Malignant Melanoma
Adenocarcinoma
Gastric Carcinoma
Gastric Lymphoma

Gastric Stromal Cell Tumors
Lymphoma
Kaposi's Sarcoma
Intestinal Stromal Cell Tumors
Carcinids
Malignant Mesethelioma
Non-mucin producing adenocarcinoma

## THE LIVER AND THE BILIARY TRACT
Hepatocellular Carcinoma
Cholangiocarcinoma
Hepatoblastoma
Angiosarcoma
Fibrolameller Carcinoma

Carcinoma of the Gallbladder
Adenocarcinoma
Squamous Cell Carcinoma
Papillary, poorly differentiated

## THE PANCREAS
Adenocarcinoma
Cystadenocarcinoma
Insulinoma

Gastrinoma
Glucagonamoa

## THE KIDNEY
Renal Cell Carcinoma

Nephroblastoma (Wilm's Tumor)

## THE LOWER URINARY TRACT
Urothelial Tumors
Squamous Cell Carcinoma
Mixed Carcinoma

Adenocarcinoma
Small Cell Carcinoma
Sarcoma

## THE MALE GENITAL TRACT
Squamous Cell CarcinomaSarcinoma
Speretocytic Sarcinoma
Embyonal Carcinoma
Choriocarcinoma
Teratoma
Leydig Cell Tumor

Sertoli Cell Tumor
Lymphoma
Adenocarcinoma
Undifferentiated Prostatic Carcinoma
Ductal Transitional Carcinoma

**FIGURE 4 CONT.**

## THE FEMALE GENITAL TRACT

Squamous Cell Carcinoma
Basal Cell Carcinoma
Melanoma
Fibrosarcoma
Intaepithelial Carcinoma
Adenocarcinoma Embryonal Rhabdomysarcoma
Large Cell Carcinoma
Neuroendocrine or Oat Cell Carcinoma
Adenocarcinoma
Adenosquamous Carcinoma
Undifferentiated Carcinoma
Carcinoma
Adenoacanthoma
Sarcoma

Carcinosarcoma
Leiomyosarcoma
Endometrial Stromal Sarcoma
Serous Cystadenocarcinoma
Mucinous Cystadenocarcinoma
Endometrioid Tumors
Adenosarcoma
Celioblastoma (Brenner Tumor)
Clear Cell Carcinoma
Unclassified Carcinoma
Granulosa-Theca Cell Tumor
Sertoli-Leydig Cell Tumor
Disgerminoma
Teratoma

## THE BREAST

Phyllodes Tumor
Sarcoma
Paget's Disease

Carcinoma
Insitu Carcinoma
Invasive Carcinoma

## THE ENDOCRINE SYSTEM

Adenoma
Carcinoma
Meningnoma
Cramiopharlingioma
Papillary Carcinoma
Follicular Carcinoma
Medullary Carcinoma
Anoplastic Carcinoma

Adenoma
Carcinoma
Pheochromocytoma
Neuroblastome
Paraganglioma
Pineal
Pineoblastoma
Pineocytoma

## THE SKIN

Melanoma
Squamous cell carcinoma
Basal cell carcinoma
Merkel cell carcinoma

Extramamary Paget's Disease
Paget's Disease of the nipple
Kaposi's Sarcoma
Cutaneous T-cell lymphoma

## BONES, JOINTS, AND SOFT TISSUE TUMORS

Multiple Myeloma
Malignant Lymphoma
Chondrosacrcooma
Mesenchyrnal Chondrosarcoma
Osteosarcoma
Ewing Tumor (Ewing Sarcoma)
Malignant Giant Cell Tumor
Adamantinoma
Malignant Fibrous Histiocytoma
Desmoplastc Fibroma

Fibrosarcoma
Chordoma
Hemangioendothelioma
Memangispericytoma
Liposarcoma
Malignant Fibrous Histiocytoma
Rhabdomysarcoms
Leiomyosarcoma
Angiosarcoma

**FIGURE 4 CONT.**

## NERVOUS SYSTEM
Schwannoma
Neurofibroma
Malignant Periferal Nerve Sheath Tumor
Astrocytoma
Fibrillary Astrocytoma
Glioblastoma Multiforme
Brain Stem Glioma
Pilocytic Astrocytoma
Pleomorphic Xanthorstrocytoma
Oligodendroglioma

Ependymoma
Gangliocytoma
Cerebral Neuroblastoma
Central Neurocytoma
Dysembryoplastic Neuroepithelial Tumor
Medulloblastoma
Malignant Meningioma
Primary Brain Lymphoma
Primary Brain Germ Cell Tumor

## THE EYE
Carcinoma
Squamous Cell Carcinoma
Mucoepidermoid Carcinoma

Melanoma
Retinoblastoma
Glioma
Meningioma

## THE HEART
Myxoma
Fibroma
Lipoma
Papillary Fibroelastoma

Rhasdoyoma
Angiosarcoma
Other Sarcoma

## HISTIOCYTOSES
Langerhans Cell Histiocytosis

## FIGURE 4 CONT.

A

B

C

**FIGURE 5**

**FIGURE 6**

**A**

**B**

**FIGURE 7**

**FIGURE 8**

**A**

**B**

**FIGURE 9**

**FIGURE 10**

A

B

**FIGURE 11**

**FIGURE 12**

**A**

**WT**

**B**

**P38G**

**FIGURE 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6054297 A
- US 5886152 A
- US 5877293 A
- US 6660843 B
- US 20030176352 A
- WO 9640912 A
- WO 9605309 A
- WO 9706816 A
- WO 9718833 A
- US 20070020284 A
- US 6235883 B
- US 7060808 B
- US 20030091561 A
- US 20030194403 A
- WO 0024782 A
- US 20030236193 A
- US 20070072801 A
- US 6936439 B
- US 6294170 B
- US 5605690 A
- US 5143854 A, Pirrung
- WO 9015070 A
- WO 9210092 A, Fodor
- US 6136269 A, Winkler
- WO 8910977 A, Southern
- WO 9841531 A, Blanchard
- US 6521437 B, Evans
- US 5420109 A
- US 5849690 A
- US 5686567 A
- US 5990273 A
- WO 0100656 A
- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- US 4522811 A
- US 5374548 A
- US 5399331 A
- US 5416016 A, Low

**Non-patent literature cited in the description**

- **DEMAREST, S. J. et al.** *J. Mol. Biol.,* 02 January 2004, vol. 335 (1), 41-48
- **CLARKE et al.** *Structure Fold. Des.,* 1999, vol. 7, 1145-53
- **ISACKE ; HORTON.** The Adhesion Molecule Facts-Book. Academic Press, 2000
- **FITZGERALD et al.** The Cytokine FactsBook. Academic Press, 2001
- **MARSH et al.** The HLA FactsBook. Academic Press, 1999
- **HARLOW ; LANE.** Antibodies: A Laboratory Manual. Cold Spring Harbor Laboratory Press, 1989
- **HAMMERLING et al.** Monoclonal Antibodies and T-Cell Hybridomas. Elsevier, 1981, 563-681
- **HARLOW et al.** Using Antibodies: A Laboratory Manual. Cold Spring Harbor Laboratory Press, 1999
- Antibody Engineering: A Practical Guide. Oxford University Press, 1995
- Antibody Engineering: A Practical Guide
- Molec. Biology and Biotechnology: A Comprehensive Desk Reference. VCH Publisher, Inc,
- **HUSTON et al.** *Cell Biophysics,* 1993, vol. 22, 189-224
- **PLÜCKTHUN ; SKERRA.** *Meth. Enzymol.,* 1989, vol. 178, 497-515
- **DAY, E.D.** Advanced Immunochemistry. Wiley-Liss, Inc, 1990
- **WARD et al.** *Nature,* 1989, vol. 341, 544-546
- **BIRD et al.** *Science,* 1988, vol. 242, 423-26
- **HUSTON et al.** *Proc. Natl. Acad Sci. USA,* 1988, vol. 85, 5879-83
- **HOLLIGER et al.** *Proc. Natl. Acad Sci. USA,* 1993, vol. 90, 6444-48
- **POLJAK et al.** *Structure,* 1994, vol. 2, 1121-23
- **KABAT et al.** *J. Biol. Chem.,* 1977, vol. 252, 6609-6616

- **KABAT.** *Adv. Prot. Chem.,* 1978, vol. 32, 1-75
- **CHOTHIA ; LESK.** *J. Mol. Biol.,* 1987, vol. 196, 901-917
- **AL-LAZIKANI et al.** *J. Mol. Biol.,* 1997, vol. 273, 927-948
- **MOREA et al.** *Methods,* 2000, vol. 20, 267-279
- **HOLLIGER et al.** *Proc. Natl. Acad. Sci. USA,* 1993, vol. 90, 6444-6448
- **CARTER ; MERCHANT.** *Curr. Opin. Biotech.,* 1997, vol. 8, 449-454
- **HOLLIGER ; WINTER.** *Cur. Op. in Biotechnology,* 1993, vol. 4, 446-449
- **PACK et al.** *Biotechnol.,* 1993, vol. 11, 1271
- **SAMBROOK et al.** Molecular Cloning: A Laboratory Manual. Cold Spring Harbor Laboratory, 2001
- **ANSUBEL et al.** Current Protocols in Molecular Biology. John Wiley and Sons, 1999
- Antibody Engineering: Methods and Protocols, in Methods in Molecular Biology. Humana Press, 2004
- Molecular Biology and Biotechnology: A Comprehensive Desk Reference. VCH Publishers, Inc, 1995
- **B.K.C. LO.** *Antibody Engineering: Methods and Protocols, in Methods in Molecular Biology*
- **R.A. MYERS.** *Molecular Biology and Biotechnology: A Comprehensive Desk Reference*
- Oligonucleotide Synthesis: A Practical Approach. IRL Press, 1984
- **WEILER et al.** *Anal. Biochem.,* 1996, vol. 243, 218
- **MASKOS et al.** *Nucleic Acids Res.,* 1992, vol. 20, 1679
- **ATKINSON et al.** Solid-Phase Synthesis of Oligodeoxyribonucleotides by the Phosphitetriester Methods, in Oligonucleotide Synthesis. 1984, vol. 35
- Nucleic Acids in Chemistry and Biology. Oxford University Press, 1996
- **FODOR et al.** *Science,* 1991, vol. 251, 767-777
- **JÖNSSON ; MALMQUIST.** *Advances in Biosnsors,* 1992, vol. 2, 291-336
- **WU et al.** *Proc. Natl. Acad. Sci. USA,* 1998, vol. 95, 6037-6042
- **V. V. RANADE.** *J. Clin. Pharmacol.,* 1989, vol. 29, 685
- **UMEZAWA et al.** *Biochem. Biophys. Res. Commun.,* 1988, vol. 153, 1038
- **P. G. BLOEMAN et al.** *FEBS Lett.,* 1995, vol. 357, 140
- **M. OWAIS et al.** *Antimicrob. Agents Chemother,* 1995, vol. 39, 180
- **BRISCOE et al.** *Am. J. Physiol.,* 1995, vol. 1233, 134
- **ISENMAN et al.** *Biochemistry,* 1979, vol. 18, 3327-36
- **RAMACHANDRAN, G. N. ; MITRA, A. K.** *J. Mol. Biol.,* 1976, vol. 107, 85-92
- **ODEFEY et al.** *J. Mol. Biol.,* 1995, vol. 245, 69-78
- **NEET, K. E. ; TIMM, D. E.** *Protein Science,* 1994, vol. 3, 2167-74
- **ASHIKARI et al.** *Biochem (Tokyo),* 1985, vol. 97, 517-28
- **THIES et al.** *J. Mol. Biol.,* 2002, vol. 319, 1267-77
- **SAPHIRE et al.** *J. Mol. Biol.,* 2002, vol. 319, 9-18
- **MILLA, M. E. ; SAUER, R. T.** *Biochemistry,* 1994, vol. 33, 1125-33
- **GLOSS, L. M. ; MATTHEWS, C. R.** *Biochemistry,* 1998, vol. 37, 15990-15999
- **THIES et al.** *J. Mol. Biol.,* 1999, vol. 293, 67-79
- **BRANDTS et al.** *Biochemistry,* 1975, vol. 14, 4953-63
- **QI et al.** *Nature Struct. Biol.,* 1998, vol. 5, 882-84